# EUROPEAN PATENT APPLICATION

(11) **EP 3 766 543 A1**
(43) Date of publication of application: **20.01.2021**
(21) Application number: 19305964.9
(22) Date of filing: 19.07.2019
(51) Int. Cl.: A61P 31/14, C07D 233/48, C07D 403/12, C07D 405/12, C07D 405/14, C07D 413/12, A61K 31/41

(54) **N-ARYL DERIVATIVES FOR TREATING A RNA VIRUS INFECTION**

(71) Applicant: ABIVAX, 75008 Paris (FR); CENTRE NATIONAL DE LA RECHERCHE SCIENTIFIQUE, 75794 Paris Cedex 16 (FR); UNIVERSITE DE MONTPELLIER, 34090 Montpellier (FR); Institut Curie, 75248 Paris Cedex 05 (FR)
(72) Inventor: The designation of the inventor has not yet been filed
(74) Representative: Nony

(57) **Abstract**

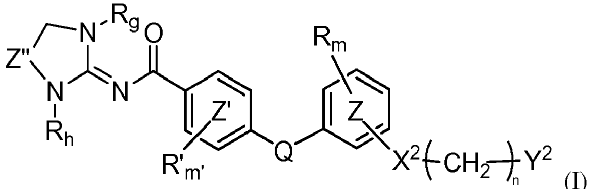

wherein:
X² represents a -CO-NRk- group, a -NR'k-CO- group, a -O- group, a -CO- group, a -SO₂-group, a -CS-NH- group, a -CH₂-NH-, a 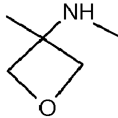 group, or a divalent 5-membered heteroaromatic ring comprising 1, 2, 3 or 4 heteroatoms; **Y²** represents a hydrogen atom, a halogen atom, a hydroxyl group, a morpholinyl group, optionally substituted by a (C₁-C₄)alkyl group, a piperidinyl group, a (C₁-C₄)alkenyl group, a -PO(ORₐ)(OR_{b}) group, Or a -CR¹R²R³ group, or any of its pharmaceutically acceptable salt.

The present invention further relates to new compounds, to pharmaceutical compositions containing them and to synthesis process for manufacturing them.

## Description

### INFECTION

The present invention relates to new compounds useful for preventing and/or treating a RNA virus infection, and most preferably a RNA virus infection caused by RNA viruses belonging to group V of the Baltimore classification, more particularly Respiratory Syncytial virus (RSV) infection.

The present invention further relates to the use of said compounds, in particular useful for preventing and/or treating a RNA virus infection, and most preferably a RNA virus infection caused by a RNA virus belonging to group V of the Baltimore classification, more particularly Respiratory Syncytial virus (RSV) infection.

It further relates to the pharmaceutical compositions containing said new compounds and to the chemical synthesis processes for obtaining them.

### BACKGROUND

Viruses are one of the major causes of diseases around the world. Viruses are generally defined as small, non-living, infectious agents that replicate only within living cells, as they do not possess a completely autonomous replication mechanism. Although diverse in shape and size, they typically consist of a virus particle (known as a "virion"), made from a protein coat which comprises at least one nucleic acid molecule and optionally, depending on the type of virus, one or more proteins or nucleoproteins.

Because viruses do not possess a completely autonomous replication mechanism, they must necessarily rely on the machinery and metabolism of the infected cell or host, in order to replicate and produce multiple copies of themselves.

Even though their replication cycle varies greatly between species, it is generally recognized that the life cycle of viruses includes six basic steps: attachment, penetration, uncoating, replication, assembly and release.

Depending on the nature of the targeted virus, therapeutic molecules have been designed which may interfere with one or more of those mechanisms.

Among those, the replication step involves not only the multiplication of the viral genome, but also the synthesis of viral messenger RNA, of viral protein, and the modulation of the transcription or translation machinery of the host. However, it is also clear that the type of genome (single-stranded, double-stranded, RNA, DNA...) characterizes dramatically this replication step. For instance, most DNA viruses assemble in the nucleus while most RNA viruses develop solely in the cytoplasm. Also, there is increasing evidence that single-stranded RNA viruses such as Influenza use the host RNA splicing and maturation machinery.

Accordingly, and considering the implications of a given type of genome in the replication step, the Baltimore classification of viruses was developed. This classification clusters viruses into families (or *"groups"*) depending on their type of genome. The present virus classification, as in 2018, comprises seven different groups:
- Group I: double-stranded DNA viruses (dsDNA);
- Group II: single-stranded DNA viruses (ssDNA);
- Group III: double-stranded RNA viruses (dsRNA);
- Group IV: (+)strand or sense RNA viruses ((+)ssRNA);
- Group V: (-)strand or antisense RNA viruses ((-)ssRNA);
- Group VI: single-stranded RNA viruses having DNA intermediates (ssRNA-RT);
- Group VII: double-stranded DNA viruses having RNA intermediates (dsDNA-RT).

According to that classification, viruses belonging to the Group VI are not, *stricto sensu*, RNA viruses. For the same reasons, viruses belonging to the Group VII are not, *stricto sensu*, DNA viruses. One well-studied example of a virus family belonging to the Group VI is the family *Retroviridae* (retrovirus) which includes HIV. One well-studied example of a virus family belonging to the Group VII is the family *Hepadnaviridae* which includes the Hepatitis B virus (HBV).

As a representative of viruses pertaining to group V one may cite the *Filoviridae* virus family encompassing the Ebola virus, the *Paramyxoviridae* family encompassing the Respiratory Syncytial virus (RSV), the *Rhabdoviridae* family, the *Orthomyxoviridae* family encompassing the Influenzavirus A, Influenzavirus B and Influenzavirus C.

Groups within the virus families particularly focused in the framework of the present invention are the ones encompassing RNA viruses, especially single-stranded RNA viruses, and more specifically RNA viruses belonging to group V of the Baltimore classification.

There are few cures for diseases caused by RNA virus infections, in particular single-stranded RNA viruses, and more specifically RNA virus infections from viruses belonging to V of the Baltimore classification. Treatment is focused on relieving the symptoms. Therefore, there is still a need to identify new antiviral drugs to treat RNA virus infections, such as RNA virus infection from group V, more particularly Respiratory Syncytial virus (RSV) infection, in particular small chemical molecules.

### DEFINITIONS

As used herein, the term "patient" refers to either an animal, such as a valuable animal for breeding, company or preservation purposes, or preferably a human or a human child, which is afflicted with, or has the potential to be afflicted with, one or more diseases and conditions described herein.

In particular, as used in the present application, the term "patient" refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of, or preventing the disease resulting from RNA virus infection, and more particularly RNA virus infection from group V, or one or more symptoms of such disease.

As used herein, an "effective amount" refers to an amount of a compound of the present invention which is effective in preventing, reducing, eliminating, treating or controlling the symptoms of the herein-described diseases and conditions, *i.e.* RNA virus infection, and more particularly RNA virus infection from group V. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "effective amount" includes "prophylaxis-effective amount" as well as "treatment-effective amount".

The term "preventing", as used herein, means reducing the risk of onset or slowing the occurrence of a given phenomenon, namely in the present invention, a disease resulting from a RNA virus infection, and more particularly a RNA virus infection from group V, and even more particularly Respiratory Syncytial virus (RSV) infection.

As used herein, « *preventing* » also encompasses « *reducing the likelihood of occurrence* » or « *reducing the likelihood of reoccurrence* ».

The term "prophylaxis-effective amount" refers to a concentration of compound of this invention that is effective in inhibiting, preventing, decreasing the likelihood of the disease by RNA viruses, and more particularly by a RNA virus from group V of the Baltimore classification, and even more particularly by Respiratory Syncytial virus (RSV) or preventing the RNA virus infection and in particular a RNA virus infection from group V or preventing the delayed onset of the disease by the RNA virus, and more particularly by a RNA virus from group V, and even more particularly by Respiratory Syncytial virus (RSV), when administered before infection, *i.e.* before, during and/or slightly after the exposure period to the RNA virus, and in particular to the RNA virus from group V, and even more particularly to Respiratory Syncytial virus (RSV).

Likewise, the term "treatment-effective amount" refers to a concentration of compound that is effective in treating the RNA virus infection, e.g. leads to a reduction in RNA viral infection, following examination when administered after infection has occurred.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, a *"viral infection or related condition"* refers to an infection of condition related to a virus, more particularly said virus having a RNA genome, and especially a RNA virus belonging to group V according to the Baltimore classification. Viruses may be further classified in distinct families, orders and genus.

For reference, the content of the *"Baltimore classification"* which is reported herein further references to the virus taxonomy as set forth in the database of the 2017 International Committee of Taxonomy of Viruses (ICTV) as released online on March 12, 2018 at http://ictvonline.org/virusTaxonomy.asp. This taxonomy is incorporated herein in its entirety.

Viruses of the *Mononegavirales* order are also particularly considered by the invention. The order *Mononegavirales* includes viruses belonging to Group V of the Baltimore classification. As of 2018, this order includes mainly the following virus families: *Bornaviridae*, *Mymonaviridae*, *Filoviridae*, *Nyamiviridae*, *Paramyxoviridae*, *Pneumoviridae*, *Rhabdoviridae*, and *Sunviridae.*

Human respiratory syncytial virus (HRSV) is a syncytial virus that causes respiratory tract infections. It is a major cause of lower respiratory tract infections and hospital visits during infancy and childhood. HRSV virus may in particular be considered by the invention and pertain to the Group V of RNA viruses. More particularly, RSV virus is a (-)ssRNA virus belonging to group V of the Baltimore classification. It is a pneumovirus which is part of the *Paramyxoviridae* family, which belongs to the *Mononegavirales* order. Among other viruses of the *Mononegavirales* order, those which are particularly considered by the invention include: measles virus, mumps virus, Nipah virus, rabies virus, and human parainfluenza virus (which includes HPIV-1, HPIV-2, HPIV-3 and HPIV-4). Of note, the *Paramyxovirinae* subfamily was conventionally merged into the *Paramyxoviridae* family, by reference to the taxonomy of the *Mononegavirales* order updated in 2016.

The virus genus which are particularly considered within the *Paramyxoviridae* family include: *Aquaparamyxovirus, Avulavirus, Ferlavirus, Henipavirus, Morbillivirus, Respirovirus* and *Rubulavirus* genus. Viruses of the *Orthomyxoviridae* family are also particularly considered by the invention. The *Orthomyxoviridae* family belongs to an "Unassigned" order according to the 2017 Virus Taxonomy. The virus genus which are particularly considered within the *Orthomyxoviridae* family include: *Alphainfluenzavirus, Betainfluenzavirus, Deltainfluenzavirus, Gammainfluenzavirus, Isavirus, Quaranjavirus,* and *Thogotovirus.*

Influenzavirus A, Influenzavirus B, Influenzavirus C may in particular be considered by the invention and pertain to the Group V RNA viruses and the *Orthomyxoviridae* family, which can be defined as a negative-sense single-stranded RNA or (-)ss RNA viruses. Isavirus and Thogotovirus also belong to the *Orthomyxoviridae* order.

### DETAILED DESCRIPTION OF THE INVENTION

The inventors have surprisingly found that aryl-N-aryl compounds are endowed with a broad-spectrum activity against RNA viruses, and more particularly single-stranded RNA viruses belonging to Group V of the Baltimore classification. Groups V include respectively (+)ssRNA viruses and (-)ssRNA viruses; which also refer to positive-sense single-stranded RNA viruses and negative-sense single-stranded RNA viruses.

For reference, the content of the « *Baltimore classification* » is considered in light of the Classification and Nomenclature of viruses as set forth in the 10th report on Virus Taxonomy dated 2017.

According to a **first aspect**, the present invention relates to a compound of formula (I) 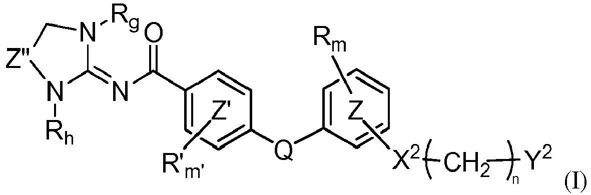 wherein: 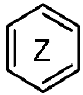
ring and 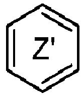 ring independently mean a phenylene or a pyridylene group,
Z" represents a -CH₂- group or a -CO- group,
R_{g} and Rₕ independently represent a hydrogen atom, a (C₁-C₄)alkyl group, a (C₃-C₆)cycloalkyl group or a -COCH₃ group,
Q is NH or O,
X² represents
   a -CO-NRₖ- group, wherein Rₖ represents a hydrogen atom or a methyl group,
   a -NR'ₖ-CO- group, wherein R'ₖ represents a hydrogen atom or a methyl group,
   a -O- group,
   a -CO- group,
   a -SO₂-group,
   a -CS-NH- group,
   a -CH₂-NH-,
   a 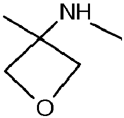 group,
      or
   a divalent 5-membered heteroaromatic ring comprising 1, 2, 3 or 4 heteroatoms, such as a triazole, an imidazole, a tetrazole or an oxadiazole,
n is 0, 1, 2 or 3,
m and m' are independently 0, 1 or 2,
Y² represents
   a hydrogen atom,
   a halogen atom,
   a hydroxyl group,
   a morpholinyl group, optionally substituted by a (C₁-C₄)alkyl group,
   a piperidinyl group, optionally interrupted by a SO₂ group,
   a (C₁-C₄)alkenyl group,
   a -PO(ORₐ)(OR_{b}) group,
   or
   a -CR¹R²R³ group, wherein R¹, R² and R³ independently represent a hydrogen atom, a fluorine atom or a (C₁-C₄)alkyl group, being understood that no more than one of R¹, R² and R³ is a hydrogen atom, or R¹ and R² form together with the carbon atom bearing them a (C₃-C₈)cycloalkyl group, said (C₃-C₈)cycloalkyl group being optionally substituted by one or two (C₁-C₄)alkyl group, halogen atom, trifluoromethyl group, cyano group or (C₁-C₄)alkoxy group and said (C₃-C₈)cycloalkyl group being optionally interrupted on said R¹ and/or R² by one or two oxygen atom(s) or by a -SO₂-group,
R and R' independently represent
   a (C₁-C₄)alkyl group, optionally substituted by a hydroxyl group, and optionally interrupted by a -SO₂- group or a -SO- group,
   a (C₁-C₄)alkenyl group,
   a (C₃-C₆)cycloalkyl group,
   a tetrahydrofuranyl group,
   a tetrahydropyranyl group,
   a trifluoromethyl group,
   a furanyl group,
   a halogen atom,
   or
   a (C₁-C₅)alkoxy group,
Rₐ and R_{b} independently represent a hydrogen atom or a (C₁-C₄)alkyl group,
or any of its pharmaceutically acceptable salt.

In other words, 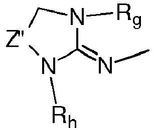 represents a group A 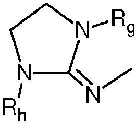 (A) or a group (B) 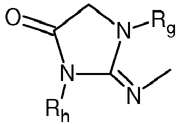 (B).

According to a **second aspect**, the present invention relates to a compound of formula (I) as defined above or any of its pharmaceutically acceptable salts, and any of compounds (1) to (111) as defined herein after or any of its pharmaceutically acceptable salts, for use as a medicament.

According to a **third aspect**, the present invention relates to compounds of formula (I) as defined above or any of its pharmaceutically acceptable salts, and any of compounds (1) to (111) as defined herein after or any of its pharmaceutically acceptable salts for use in the treatment and/or prevention of a RNA virus infection caused by a RNA virus belonging to group V of the Baltimore classification, and in particular a RSV viral infection or a virus-related condition.

The above-mentioned compounds (I) are particularly suitable for treating or preventing a virus infection or related condition, in particular a RNA virus infection caused by a RNA virus belonging to group V of the Baltimore classification or related condition, and most preferably a RSV viral infection or a virus-related condition.

According to a particular embodiment, the present invention relates to a compound of formula (I) as defined above, wherein 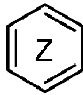 ring and 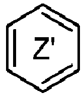 ring both represent a phenylene group or 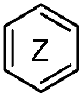 ring represents a pyridylene group and 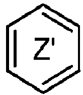 ring represents a phenylene group, or any of its pharmaceutically acceptable salt.

In another embodiment, the present invention relates to a compound of formula (I) as defined above, wherein Q is a NH group or any of its pharmaceutically acceptable salt.

In another embodiment, the present invention relates to a compound of formula (I) as defined above, wherein R_{g} and Rₕ represent a hydrogen atom or any of its pharmaceutically acceptable salt.

In another embodiment, the present invention relates to a compound of formula (I) as defined above, wherein
X² represents
a -CO-NH- group,
a -NH-CO- group,
a -O- group,
a -CO- group,
or
a divalent 5-membered heteroaromatic ring comprising at least 2 nitrogen atoms, such as a triazole, an imidazole or a tetrazole,
or any of its pharmaceutically acceptable salt.

In another embodiment, the present invention relates to a compound of formula (I) as defined above, wherein
Y² represents
a hydrogen atom,
a halogen atom,
a morpholinyl group, optionally substituted by a (C₁-C₄)alkyl group,
a (C₁-C₄)alkenyl group,
a -PO(ORₐ)(OR_{b}) group,
or
a -CR¹R²R³ group, wherein R¹, R² and R³ independently represent a hydrogen atom, a fluorine atom or a (C₁-C₄)alkyl group, being understood that no more than one of R¹, R² and R³ is a hydrogen atom, or R¹ and R² form together with the carbon atom bearing them a (C₃-C₈)cycloalkyl group, said (C₃-C₈)cycloalkyl group being optionally substituted by one or two (C₁-C₄)alkyl group, halogen atom, trifluoromethyl group or cyano group and said (C₃-C₈)cycloalkyl group being optionally interrupted on said R¹ and/or R² by one or two oxygen atom(s) or by a -SO₂- group,
with Rₐ and R_{b} being as defined above,
or any of its pharmaceutically acceptable salt.

Any combination of the above-defined embodiments for R, R', Z", R_{g}, Rₕ, Q, m, m', 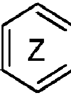 ring, 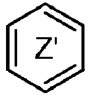 ring, X², n and Y² with each other does form part of the instant invention.

According to a preferred embodiment of the present invention, the compound of formula (I) is chosen from:
- (1) 3-cyclopropyl-N-[(2Z)-imidazolidin-2-ylidene]-4-[(3-{ [1-(propan-2-yl)cyclopropyl]carbamoyl}phenyl)amino]benzamide
- (2) 3-cyclopentyl-N-[(2E)-imidazolidin-2-ylidene]-4-{[3-(2-methylpropanamido)phenyl]amino}benzamide
- (3) 4-{[2-chloro-3-(morpholine-4-carbonyl)phenyl]amino}-3-cyclopentyl-N-[(2E)-imidazolidin-2-ylidene]benzamide
- (4) 4-{[2-chloro-3-(morpholine-4-carbonyl)phenyl]amino}-3-cyclopropyl-N-[(2E)-imidazolidin-2-ylidene]benzamide
- (5) 3-tert-butyl-N-[(2E)-imidazolidin-2-ylidene]-4-{[3-(4-methylpentanamido)phenyl]amino}benzamide
- (6) 3-tert-butyl-N-[(2E)-imidazolidin-2-ylidene]-4-({3-[(4-methylpentan-2-yl)carbamoyl]phenyl}amino)benzamide
- (7) 2-cyclopropyl-3-[(2-cyclopropyl-4-{[(2E)-imidazolidin-2-ylidene]carbamoyl}phenyl)amino]-N-(3-methylbutyl)benzamide
- (8) 3-tert-butyl-N-[(2E)-imidazolidin-2-ylidene]-4-({3-[(3-methylbutyl)carbamoyl]phenyl}amino)benzamide
- (9) 3-tert-butyl-4-{[2-cyclopropyl-3-(morpholine-4-carbonyl)phenyl]amino}-N-[(2E)-imidazolidin-2-ylidene]benzamide
- (10) 4-({2-chloro-3-[(3-methylbutyl)carbamoyl]phenyl}amino)-3-cyclopropyl-N-[(2E)-imidazolidin-2-ylidene]benzamide
- (11) 4-({2-chloro-3-[(propan-2-yl)carbamoyl]phenyl}amino)-3-cyclopropyl-N-[(2E)-imidazolidin-2-ylidene]benzamide
- (12) 4-({2-chloro-3-[2-(1,4-dioxan-2-yl)ethoxy]phenyl}amino)-3-cyclopropyl-N-[(2E)-imidazolidin-2-ylidene]benzamide
- (13) 4-[(3-cyclopropaneamidophenyl)amino]-3-cyclopropyl-N-[(2E)-imidazolidin-2-ylidene]benzamide
- (14) 3-cyclopropyl-N-[(2Z)-imidazolidin-2-ylidene]-4-({3-[(1-methylcyclopropyl)carbamoyl]phenyl}amino)benzamide
- (15) 4-{[2-chloro-3-(morpholine-4-carbonyl)phenyl]amino}-N-[(2E)-imidazolidin-2-ylidene]-3-(oxolan-3-yl)benzamide
- (16) 4-{[2-chloro-3-(morpholine-4-carbonyl)phenyl]amino}-N-[(2E)-imidazolidin-2-ylidene]-3-(trifluoromethyl)benzamide
- (17) 3-tert-butyl-N-[(2E)-1-methylimidazolidin-2-ylidene]-4-({3-[(propan-2-yl)carbamoyl]phenyl}amino)benzamide
- (18) 3-cyclobutyl-N-[(2Z)-imidazolidin-2-ylidene]-4-({3-[(1-methylcyclopropyl)carbamoyl]phenyl}amino)benzamide
- (19) 3-tert-butyl-N-[(2E)-imidazolidin-2-ylidene]-4-({3-[(propan-2-yl)carbamoyl]phenyl}amino)benzamide
- (20) 3-tert-butyl-4-{[3-(2,2-dimethylpropanamido)phenyl]amino}-N-[(2Z)-imidazolidin-2-ylidene]benzamide
- (21) 3-cyclopropyl-N-[(2Z)-imidazolidin-2-ylidene]-4-{[3-(1-methylcyclopropaneamido)phenyl]amino}benzamide
- (22) 4-{[3-(3-cyclohexylpropoxy)phenyl]amino}-3-cyclopropyl-N-[(2E)-imidazolidin-2-ylidene]benzamide
- (23) 4-({2-chloro-3-[(1,4-dioxan-2-yl)methoxy]phenyl}amino)-3-cyclopropyl-N-[(2E)-imidazolidin-2-ylidene]benzamide
- (24) 3-tert-butyl-4-({3-[(1-methylcyclopropyl)carbamoyl]phenyl}amino)-N-[(2E)-1-methylimidazolidin-2-ylidene]benzamide
- (25) 4-{[2-chloro-3-(cyclopropylcarbamoyl)phenyl]amino}-3-cyclopropyl-N-[(2Z)-imidazolidin-2-ylidene]benzamide
- (26) 3-cyclopropyl-N-[(2E)-imidazolidin-2-ylidene]-4-{[3-(2-methylpropanamido)phenyl]amino}benzamide
- (27) 3-cyclopropyl-N-[(2Z)-imidazolidin-2-ylidene]-4-({2-methoxy-3-[(3-methylbutyl)carbamoyl]phenyl}amino)benzamide
- (28) 4-({2-chloro-3-[(oxolan-3-yl)carbamoyl]phenyl}amino)-3-cyclopropyl-N-[(2Z)-imidazolidin-2-ylidene]benzamide
- (29) N-[(2E)-imidazolidin-2-ylidene]-4-({3-[(3-methylbutyl)carbamoyl]phenyl}amino)-3-(prop-1-en-2-yl)benzamide
- (30) 3-(2-hydroxypropan-2-yl)-N-[(2E)-imidazolidin-2-ylidene]-4-{ [3-(morpholine-4-carbonyl)-2-(trifluoromethyl)phenyl]amino}benzamide
- (31) 3-cyclopropyl-4-({3-[(1-ethylcyclopropyl)carbamoyl]phenyl}amino)-N-[(2Z)-imidazolidin-2-ylidene]benzamide
- (32) 3-cyclopropyl-4-({3-fluoro-5-[(propan-2-yl)carbamoyl]phenyl}amino)-N-[(2Z)-imidazolidin-2-ylidene]benzamide
- (33) 3-tert-butyl-N-[(2E)-imidazolidin-2-ylidene]-4-[(3-{ [2-(oxan-3-yl)ethyl]carbamoyl}phenyl)amino]benzamide
- (34) 3-cyclopropyl-N-[(2Z)-imidazolidin-2-ylidene]-4-({3-[(propan-2-yl)carbamothioyl]phenyl}amino)benzamide
- (35) 3-tert-butyl-N-[(2E)-imidazolidin-2-ylidene]-4-[(3-{ [2-(oxan-2-yl)ethyl]carbamoyl}phenyl)amino]benzamide
- (36) 3-cyclopropyl-N-[(2E)-1-methylimidazolidin-2-ylidene]-4-({3-[(propan-2-yl)carbamoyl]phenyl}amino)benzamide
- (37) 3-tert-butyl-N-[(2E)-imidazolidin-2-ylidene]-4-{[3-(2-methylpropanamido)phenyl]amino}benzamide
- (38) 3-tert-butyl-N-[(2E)-imidazolidin-2-ylidene]-4-({3-[methyl(propan-2-yl)carbamoyl]phenyl}amino)benzamide
- (39) 3-tert-butyl-N-[(2E)-imidazolidin-2-ylidene]-4-[(3-{ [2-(oxan-4-yl)ethyl]carbamoyl}phenyl)amino]benzamide
- (40) 2-[(2-cyclopropyl-4-{[(2E)-imidazolidin-2-ylidene]carbamoyl}phenyl)amino]-3-methyl-N-(3-methylbutyl)pyridine-4-carboxamide
- (41) 4-{[3-(3-cyclohexylpropoxy)phenyl]amino}-3-cyclopropyl-N-[(2E)-4-oxoimidazolidin-2-ylidene]benzamide
- (42) 4-{[2-cyclopropyl-3-(morpholine-4-carbonyl)phenyl]amino}-N-[(2Z)-imidazolidin-2-ylidene]-3-(trifluoromethyl)benzamide
- (43) 3-cyclopropyl-N-[(2E)-1-cyclopropylimidazolidin-2-ylidene] -4-({3-[(propan-2-yl)carbamoyl]phenyl}amino)benzamide
- (44) 3-(2-hydroxypropan-2-yl)-N-[(2E)-imidazolidin-2-ylidene]-4-({3-[(3-methylbutyl)carbamoyl]phenyl}amino)benzamide
- (45) 4-{[2-chloro-3-(morpholine-4-carbonyl)phenyl]amino}-3-(2-hydroxypropan-2-yl)-N-[(2E)-imidazolidin-2-ylidene]benzamide
- (46) N-[(2E)-1-acetylimidazolidin-2-ylidene]-4-{[3-(3-cyclohexylpropoxy)phenyl]amino}-3-cyclopropylbenzamide
- (47) 3-cyclopropyl-4-({3-fluoro-5-[(1-methylcyclopropyl)carbamoyl]phenyl}amino)-N-[(2Z)-imidazolidin-2-ylidene]benzamide
- (48) 3-tert-butyl-N-[(2E)-imidazolidin-2-ylidene]-4-[(3-{[2-(oxolan-3-yl)ethyl]carbamoyl}phenyl)amino]benzamide
- (49) 3-tert-butyl-N-[(2E)-4-oxoimidazolidin-2-ylidene]-4-({3-[(propan-2-yl)carbamoyl]phenyl}amino)benzamide
- (50) 4-({2-chloro-3-[(oxan-4-yl)carbamoyl]phenyl}amino)-3-cyclopropyl-N-[(2E)-imidazolidin-2-ylidene]benzamide
- (51) 3-cyclopentyl-4-({3-[(1,4-dioxan-2-yl)methoxy]phenyl}amino)-N-[(2E)-imidazolidin-2-ylidene]benzamide
- (52) 3-tert-butyl-4-({3-[(1,4-dioxan-2-yl)methoxy]phenyl}amino)-N-[(2E)-imidazolidin-2-ylidene]benzamide
- (53) 3-cyclopropyl-N-[(2Z)-imidazolidin-2-ylidene]-4-[(3-{[1-(trifluoromethyl)cyclopropyl]carbamoyl}phenyl)amino]benzamide
- (54) 3-tert-butyl-N-[(2E)-imidazolidin-2-ylidene]-4-({2-[(propan-2-yl)carbamoyl]phenyl}amino)benzamide
- (55) 2-[(2-tert-butyl-4-{ [(2E)-imidazolidin-2-ylidene]carbamoyl}phenyl)amino]-N-(3-methylbutyl)pyridine-4-carboxamide
- (56) 3-tert-butyl-N-[(2E)-imidazolidin-2-ylidene]-4-({2-[(2-methylpropyl)carbamoyl]phenyl}amino)benzamide
- (57) 4-{[3-(5-chloro-1H-imidazol-2-yl)-2-methylphenyl]amino}-3-cyclopropyl-N-[(2Z)-imidazolidin-2-ylidene]benzamide
- (58) 3-cyclopropyl-N-[(2E)-imidazolidin-2-ylidene]-4-[(3-{ [2-(oxan-4-yl)ethyl]carbamoyl}phenyl)amino]benzamide
- (59) N-[(2E)-imidazolidin-2-ylidene]-3-(oxolan-3-yl)-4-({3-[(propan-2-yl)carbamoyl]phenyl}amino)benzamide
- (60) 3-cyclopropyl-N-[(2Z)-imidazolidin-2-ylidene]-4-({3-[1-(3-methylbut-2-en-1-yl)-1H-1,2,3,4-tetrazol-5-yl]phenyl}amino)benzamide
- (61) 3-cyclopropyl-N-[(2E)-1-methyl-5-oxoimidazolidin-2-ylidene]-4-({3-[(propan-2-yl)carbamoyl]phenyl}amino)benzamide
- (62) 3-cyclopropyl-N-[(2E)-imidazolidin-2-ylidene]-4-({2-[(3-methylbutyl)carbamoyl]phenyl}amino)benzamide
- (63) N-[(2E)-imidazolidin-2-ylidene]-4-{[3-(2-methylpropanamido)phenyl]amino}-3-(oxolan-3-yl)benzamide
- (64) 4-({3-[(1-cyanocyclopropyl)carbamoyl]-2-methylphenyl}amino)-3-cyclopropyl-N-[(2Z)-imidazolidin-2-ylidene]benzamide
- (65) 3-cyclopropyl-N-[(2Z)-imidazolidin-2-ylidene]-4-({3-[(propan-2-yl)carbamoyl]-5-(trifluoromethyl)phenyl}amino)benzamide
- (66) 3-cyclopropyl-4-({4-fluoro-3-[(propan-2-yl)carbamoyl]phenyl}amino)-N-[(2Z)-imidazolidin-2-ylidene]benzamide
- (67) 3-(2-hydroxypropan-2-yl)-N-[(2E)-imidazolidin-2-ylidene]-4-({3-[3-(oxan-4-yl)propoxy]phenyl} amino)benzamide
- (68) N-{3-[(2-cyclopropyl-4-{[(2E)-imidazolidin-2-ylidene]carbamoyl}phenyl)amino]-2-methylphenyl}-3-methyloxetane-3-carboxamide
- (69) N-[(2Z)-imidazolidin-2-ylidene]-3-(oxan-4-yl)-4-({3-[(propan-2-yl)carbamoyl]phenyl}amino)benzamide
- (70) 3-cyclopropyl-N-[(2E)-imidazolidin-2-ylidene]-4-({2-[(propan-2-yl)carbamoyl]phenyl}amino)benzamide
- (71) 3-tert-butyl-N-[(2Z)-1,3-dimethylimidazolidin-2-ylidene]-4-({3-[(propan-2-yl)carbamoyl]phenyl}amino)benzamide
- (72) 3-chloro-N-[(2E)-imidazolidin-2-ylidene]-4-{ [3-(2-methylpropanamido)phenyl]amino}benzamide
- (73) 3-cyclopropyl-4-{[3-fluoro-5-(morpholine-4-carbonyl)phenyl]amino}-N-[(2Z)-imidazolidin-2-ylidene]benzamide
- (74) 2-[(2-tert-butyl-4-{[(2E)-imidazolidin-2-ylidene]carbamoyl}phenyl)amino]-N-[2-(oxan-4-yl)ethyl]pyridine-4-carboxamide
- (75) 3-cyclopropyl-4-{ [3-(N,2-dimethylpropanamido)phenyl]amino}-N-[(2E)-imidazolidin-2-ylidene]benzamide
- (76) 3-cyclopropyl-N-[(2Z)-imidazolidin-2-ylidene]-4-[(3-{[(propan-2-yl)carbamoyl]methyl}phenyl)amino]benzamide
- (77) 3-cyclopropyl-4-({2-fluoro-5-[(propan-2-yl)carbamoyl]phenyl}amino)-N-[(2Z)-imidazolidin-2-ylidene]benzamide
- (78) 4-{[2-cyclopropyl-3-(morpholine-4-carbonyl)phenyl]amino}-3-(2-hydroxypropan-2-yl)-N-[(2E)-imidazolidin-2-ylidene]benzamide
- (79) 3-(furan-3-yl)-N-[(2E)-imidazolidin-2-ylidene]-4-[(3-{ [2-(oxan-4-yl)ethyl]carbamoyl}phenyl)amino]benzamide
- (80) 3-cyclopropyl-N-[(2E)-imidazolidin-2-ylidene]-4-({2-[(2-methylpropyl)carbamoyl]phenyl}amino)benzamide
- (81) 3-cyclopropyl-N-[(2E)-1-methyl-4-oxoimidazolidin-2-ylidene]-4-({3-[(propan-2-yl)carbamoyl]phenyl}amino)benzamide
- (82) 3-cyclopropyl-N-[(2Z)-imidazolidin-2-ylidene]-4-[(3-{3-[(propan-2-yl)amino]oxetan-3-yl}phenyl)amino]benzamide
- (83) 3-cyclopropyl-N-[(2Z)-imidazolidin-2-ylidene]-4-({3-[(2-methylpropanamido)methyl]phenyl}amino)benzamide
- (84) 3-(2-hydroxypropan-2-yl)-N-[(2E)-imidazolidin-2-ylidene]-4-({3-[3-(oxan-4-yl)propanesulfonyl]phenyl}amino)benzamide
- (85) 3-(2-hydroxypropan-2-yl)-N-[(2E)-imidazolidin-2-ylidene]-4-({3-[2-(oxan-4-yl)ethoxy]phenyl} amino)benzamide
- (86) 3-cyclopropyl-N-[(2Z)-imidazolidin-2-ylidene]-4-({3-[(1-methylcyclopropyl)carbamoyl]-5-(trifluoromethyl)phenyl}amino)benzamide
- (87) 3-cyclopropyl-N-[(2E)-imidazolidin-2-ylidene]-4-{2-[(3-methylbutyl)carbamoyl]phenoxy}benzamide
- (88) 4-({3-[2-(1,4-dioxan-2-yl)ethoxy]phenyl}amino)-3-(2-hydroxypropan-2-yl)-N-[(2Z)-imidazolidin-2-ylidene]benzamide
- (89) 3-[(2-cyclopropyl-4-{[(2Z)-imidazolidin-2-ylidene]carbamoyl}phenyl)amino]-2-methylphenyl diethyl phosphate
- (90) 3-cyclopropyl-N-[(2Z)-imidazolidin-2-ylidene]-4-({3-[2-(3-methylbut-2-en-1-yl)-2H-1,2,3,4-tetrazol-5-yl]phenyl}amino)benzamide
- (91) 3-cyclopropyl-5-fluoro-N-[(2Z)-imidazolidin-2-ylidene]-4-({3-[(propan-2-yl)carbamoyl]phenyl}amino)benzamide
- (92) 3-[(2-cyclopropyl-4-{[(2Z)-imidazolidin-2-ylidene]carbamoyl}phenyl)amino]-2-(furan-3-yl)-N-(propan-2-yl)benzamide
- (93) 3-tert-butyl-N-[(2E)-imidazolidin-2-ylidene]-4-[2-(4-methylpentanamido)phenoxy]benzamide
- (94) 4-({2-chloro-3-[(1,1-dioxo-1λ⁶-thian-4-yl)carbamoyl]phenyl}amino)-3-cyclopropyl-N-[(2E)-imidazolidin-2-ylidene]benzamide
- (95) 3-cyclopentyl-N-[(2Z)-imidazolidin-2-ylidene]-4-({3-[(oxolan-3-yl)carbamoyl]phenyl}amino)benzamide
- (96) 3-cyclopentyl-N-[(2Z)-imidazolidin-2-ylidene]-4-({3-[(oxan-4-yl)carbamoyl]phenyl}amino)benzamide
- (97) 4-{[2-chloro-3-(morpholine-4-carbonyl)phenyl]amino}-N-[(2E)-imidazolidin-2-ylidene]-3-(oxolan-2-yl)benzamide
- (98) 3-cyclopropyl-4-({2-fluoro-3-[(propan-2-yl)carbamoyl]phenyl}amino)-N-[(2Z)-imidazolidin-2-ylidene]benzamide
- (99) 4-({2-chloro-3-[(1-methylcyclopropyl)carbamoyl]phenyl}amino)-3-cyclopropyl-N-[(2Z)-imidazolidin-2-ylidene]benzamide
- (100) 3-cyclopropyl-N-[(2E)-imidazolidin-2-ylidene]-4-[2-(4-methylpentanamido)phenoxy]benzamide
- (101) 4-({2-chloro-3-[(1-methylcyclopropyl)carbamoyl]phenyl}amino)-N-[(2E)-imidazolidin-2-ylidene]-3-methanesulfinylbenzamide
- (102) 2-chloro-3-[(4-{[(2Z)-imidazolidin-2-ylidene]carbamoyl}-2-methanesulfonylphenyl)amino]-N-(1-methylcyclopropyl)benzamide
- (103) 4-{[2-chloro-3-(1,1-dioxo-1λ⁶-thiomorpholine-4-carbonyl)phenyl]amino}-3-cyclopropyl-N-[(2E)-imidazolidin-2-ylidene]benzamide
- (104) 4-({2-chloro-3-[3-(propan-2-yl)morpholine-4-carbonyl]phenyl}amino)-3-cyclopropyl-N-[(2Z)-imidazolidin-2-ylidene]benzamide
- (105) 4-({2-chloro-3-[2-(propan-2-yl)morpholine-4-carbonyl]phenyl}amino)-3-cyclopropyl-N-[(2Z)-imidazolidin-2-ylidene]benzamide
- (106) 3-cyclopropyl-N-[(2Z)-imidazolidin-2-ylidene]-5-methyl-4-({3-[(propan-2-yl)carbamoyl]phenyl}amino)benzamide
- (107) 3-cyclopropyl-N-[(2Z)-imidazolidin-2-ylidene]-4-[(3-{[1-(2-methylpropyl)cyclopropyl]carbamoyl}phenyl)amino]benzamide
- (108) N-{3-[(2-cyclopropyl-4-{[(2E)-imidazolidin-2-ylidene]carbamoyl}phenyl)amino]phenyl}oxolane-2-carboxamide
- (109) 4-[(2-chloro-3-{[(oxolan-2-yl)methyl]carbamoyl}phenyl)amino]-3-cyclopropyl-N-[(2E)-imidazolidin-2-ylidene]benzamide
- (110) N-[(2E)-1-acetylimidazolidin-2-ylidene]-3-cyclopropyl-4-{ [3-(2-methylpropanamido)phenyl]amino}benzamide
- (111) 4-({3-[(1-tert-butylcyclopropyl)carbamoyl]phenyl}amino)-3-cyclopropyl-N-[(2Z)-imidazolidin-2-ylidene]benzamide
and their pharmaceutically acceptable salts.

The present invention therefore extends to compounds (1) to (111) and their pharmaceutically acceptable salts, such as hydrobromide, tartrate, citrate, trifluoroacetate, ascorbate, hydrochloride, tosylate, triflate, maleate, mesylate, formate, acetate and fumarate.

According to another aspect, a subject-matter of the present invention relates to compounds (1) to (111) or any of its pharmaceutically acceptable salts, for use as a medicament.

According to another aspect, a subject-matter of the present invention relates to a compound of formula (I) as defined above or any of its pharmaceutically acceptable salts, and any of compounds (1) to (111) or any of its pharmaceutically acceptable salts, for use as an agent for preventing, inhibiting or treating a RNA virus infection caused by a RNA virus belonging to group V of the Baltimore classification, and in particular a RSV viral infection or a virus-related condition.

The compounds of the invention may exist in the form of free bases or of addition salts with pharmaceutically acceptable acids.

« Pharmaceutically acceptable salt thereof » refers to salts which are formed from acid addition salts formed with inorganic acids (*e.g*. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and polygalacturonic acid.

Suitable physiologically acceptable acid addition salts of compounds of formula (I) include hydrobromide, tartrate, citrate, trifluoroacetate, ascorbate, hydrochloride, tosylate, triflate, maleate, mesylate, formate, acetate and fumarate.

The compounds of formula (I) and any of compounds (1) to (111) or any of their pharmaceutically acceptable salts may form solvates or hydrates and the invention includes all such solvates and hydrates.

The compounds of formula (I) may be present as well under tautomer forms and are part of the invention.The terms "hydrates" and "solvates" simply mean that the compounds (I) according to the invention can be in the form of a hydrate or solvate, i.e. combined or associated with one or more water or solvent molecules. This is only a chemical characteristic of such compounds, which can be applied for all organic compounds of this type.

In the context of the present invention, the term:
- "halogen" is understood to mean chlorine, fluorine, bromine, or iodine, and in particular denotes chlorine, fluorine or bromine,
- "(C₁-Cₓ)alkyl", as used herein, respectively refers to a C₁-Cₓ normal, secondary or tertiary saturated hydrocarbon, for example (C₁-C₆)alkyl. Examples are, but are not limited to, methyl, ethyl, 1-propyl, 2-propyl, butyl, pentyl,
- an "alkenylene" means a divalent (C₁-Cₓ)alkyl group comprising a double bond, and more particularly a ethenylene group, also known as vinylene or 1,2-ethenediyl,
- "(C₃-C₆)cycloalkyl", as used herein, refers to a cyclic saturated hydrocarbon. Examples are, but are not limited to, cyclopropyl, cyclobutyl, cyclopentyl, cyclohexyl,
- "(C₁-Cₓ)alkoxy", as used herein, refers to a O-(C₁-Cₓ)alkyl moiety, wherein alkyl is as defined above, for example (C₁-C₆)alkoxy. Examples are, but are not limited to, methoxy, ethoxy, 1-propoxy, 2-propoxy, butoxy, pentoxy,
- a "divalent 5-membered heteroaromatic ring comprising 1, 2, 3 or 4 heteroatoms" as used herein, means a divalent ring consisting of an aromatic ring comprising 5 bonds and 1, 2, 3 or 4 heteroatoms selected from nitrogen and oxygen atoms. In one embodiment, it comprises at least 1 heteroatom, and preferably at least one nitrogen atom. In another embodiment, it comprises at least 2 heteroatoms, with for example at least one nitrogen atom. According to a further embodiment, it comprises 2, 3 or 4 nitrogen atoms, preferably 3 nitrogen atoms. According to an even further embodiment, it comprises one nitrogen atom and one oxygen atom or two nitrogen atoms and one oxygen atom. Examples are, but not limited to, divalent tetrazole, divalent triazole, such as 1,2,3- or 1,2,4- triazoles, oxadiazoles, such as 1,2,4- oxadiazole or 1,2,3-oxadiazole and divalent diazoles such as divalent diazole and divalent imidazole. According to a preferred embodiment, such divalent 5-membered heteroaromatic ring comprising 2 or 3 heteroatoms is a divalent triazole.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

The compounds of general formula (I) when Q is NH can be prepared according to scheme 1 below.

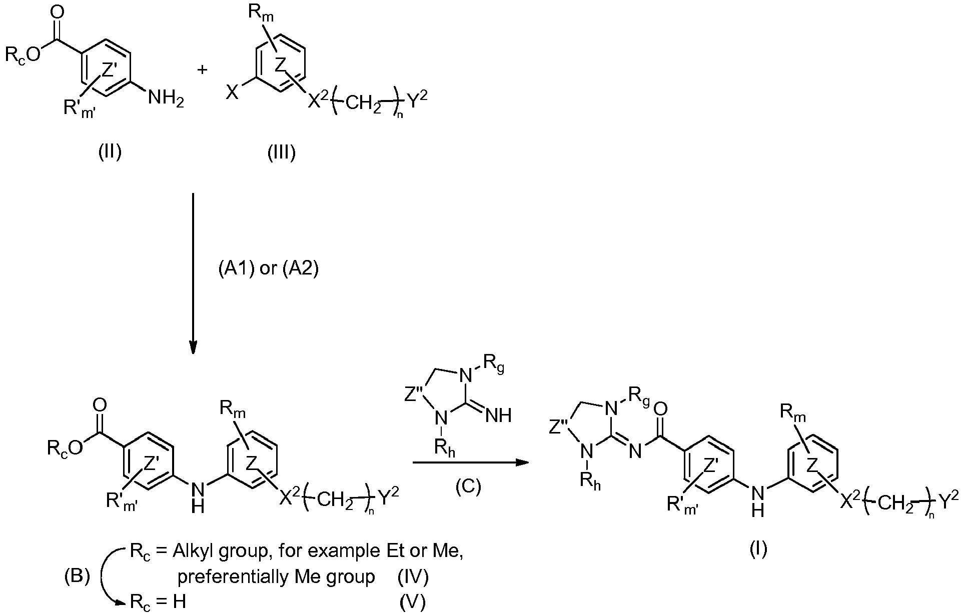

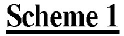

The synthesis is based on a coupling reaction of a halogeno aromatic compound of formula (III) with an aniline derivative (II), wherein R, R', R_{c}, R_{g}, Rₕ, m, m', 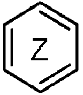 ring, 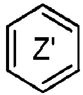 ring, X², n, Y² are as defined above and X is a chlorine atom, an iodine atom or a bromine atom, following procedure (A1) or (A2), followed by at least a further step reacting the carboxylic acid derivative of the compound obtained after said coupling step with a compound of formula 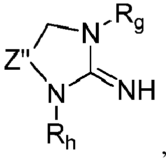 wherein Z", R_{g} and Rₕ are as defined above.

In the case where R_{g} = Rₕ = H and Z'' is -CO-, the procedure may consist firstly in reacting compound (V) with guanidine and secondly in a cyclisation step to afford final cyclised derivative (I) where R_{g} = Rₕ = H and Z'' is -CO-.

According to one embodiment, procedure (A1) may advantageously be used when the group 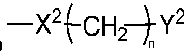 is in meta or para position on the 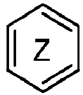 ring, with respect to the -NH- group.

According to procedure (A1), the compound of formula (III) may be placed in a protic solvent such as *tert*-butanol. The compound of formula (II) may then be added, for example in a molar ratio ranging from 1 to 1.5 with respect to the compound of formula (III) in presence of an inorganic base, such as Cs₂CO₃ or K₂CO₃, for example in a molar ratio ranging from 1 to 5 still with respect to the compound of formula (III), in the presence of a diphosphine, such as Xantphos (4,5-Bis(diphenylphosphino)-9,9-dimethylxanthene) or X-Phos (2-Dicyclohexylphosphino-2',4',6'-triisopropylbiphenyl) or rac-BINAP in particular in an amount ranging from 2 mol% to 15 mol% relative to the total amount of compound of formula (III), and in the presence of an organometallic catalyst, such as Pd(OAc)₂ or Pd₂dba₃, or BrettPhos Pd G3 in an amount ranging from 2 mol% to 25 mol% relative to the total amount of compound of formula (III). The reaction mixture can then be heated at a temperature ranging from 80 to 130°C, for example at 90°C, and stirred for a time ranging from 15 to 25 hours, for example during 20 hours, under inert gas and for example argon. The reaction mixture can be concentrated under reduced pressure and the residue can be diluted with an organic solvent such as ethyl acetate. The organic phase can be washed with water, decanted, dried over magnesium sulphate, filtered and then concentrated under reduced pressure to give a compound of formula (IV).

According to one embodiment, procedure (A2) may advantageously be used when the group 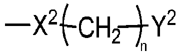 is in ortho position on the 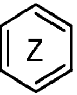 ring, with respect to the -NH- group.

According to procedure (A2), the compound of formula (II) may be placed in a polar aprotic solvent such as dimethylsulfoxide. The compound of formula (III) may then be added, for example in a molar ratio ranging from 1 to 1.5 with respect to the compound of formula (II) in presence of an inorganic base, such as Cs₂CO₃ or K₂CO₃, for example in a molar ratio ranging from 1 to 5 still with respect to the compound of formula (II), in the presence of a ligand, such as L-proline in particular in an amount ranging from 2 mol% to 25 mol% relative to the total amount of compound of formula (II), and in the presence of an organometallic catalyst, such as CuI, in an amount ranging from 2 mol% to 25 mol% relative to the total amount of compound of formula (II). The reaction mixture can then be heated at a temperature ranging from 80 to 130°C, for example at 90°C, and stirred for a time ranging from 15 to 25 hours, for example during 20 hours, under inert gas and for example argon. The reaction mixture can be diluted with an organic solvent such as ethyl acetate. The organic phase can be washed with water, decanted, dried over magnesium sulphate, filtered and then concentrated under reduced pressure to give a compound of formula (IV).

The starting compounds of formula (II), (III) are available or can be prepared according to methods known to the person skilled in the art.

Accordingly, the present invention further relates to the synthesis process for manufacturing new compounds of formula (I) as defined above, when Q is NH, comprising at least
(i) a step of coupling a compound of formula (II) 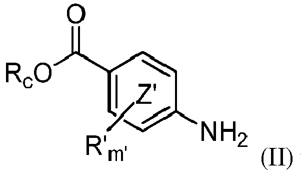 with a compound of formula (III) 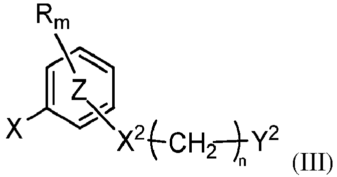 wherein R, R', m, m', n, 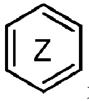 ring, 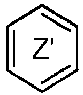 ring, X², Y² are as defined above, R_{c} is an alkyl group, such as a ethyl or methyl group and X is a chlorine atom, an iodine atom or a bromine atom, in presence of an inorganic base and a ligand and in the presence of an organometallic catalyst, to obtain a compound of formula (IV) 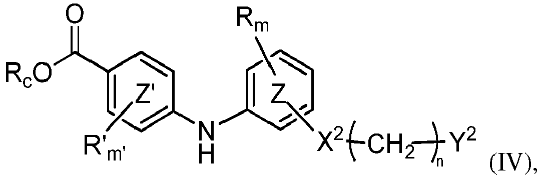 wherein R_{c} means an alkyl group, such as an ethyl group or a methyl group, preferentially a methyl group and R, R', m, m',n, 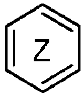 ring, 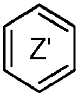 ring, X², Y² are as defined above, followed by a hydrolysis leading to a compound of formula 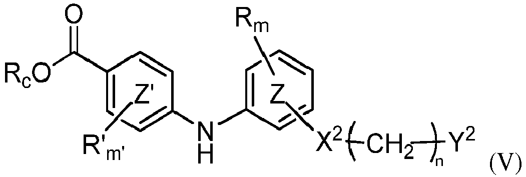 wherein R_{c} is a hydrogen atom and R, R', m, m', n, 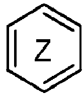 ring, 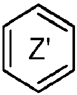 ring, X², Y² are as defined above, and
(ii) a step of reacting a compound of formula (V) with a compound of formula 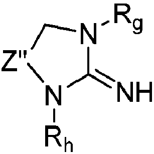 wherein Z", R_{g} and Rₕ are as defined above in presence of an organic base such as N,N-diisopropylethylamine and in presence of a coupling agent such as 1,1'-carbonyldiimidazole, to obtain a compound for formula (I) wherein Q is NH, as defined above.

The compounds of general formula (I) when Q is O can be prepared according to scheme 1' below.

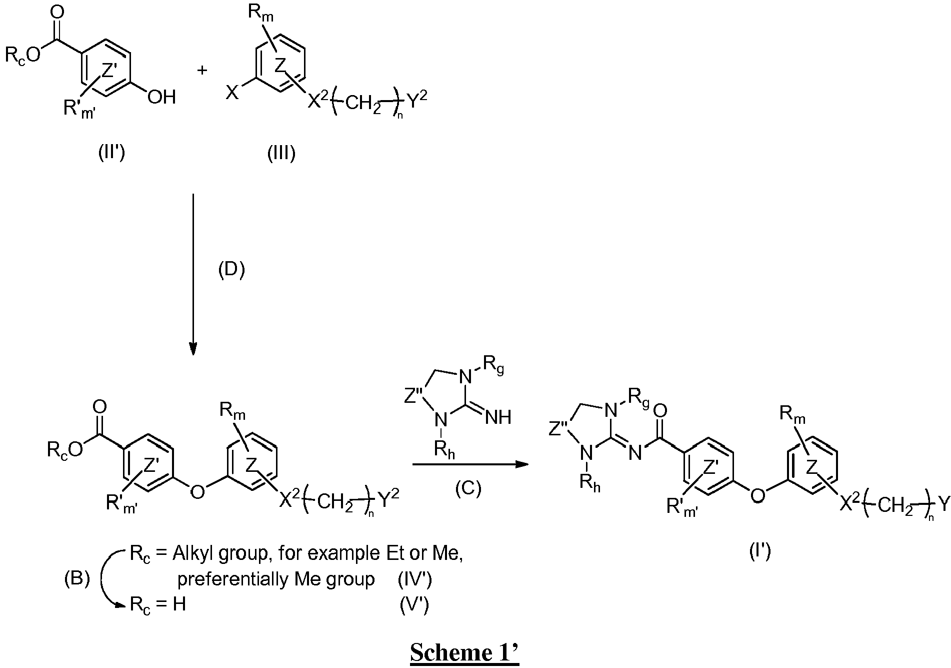

The synthesis is based on a coupling reaction starting from a halogeno aromatic compound of formula (III) with a phenol derivative of formula (II'), wherein R, R', R_{c}, R_{g}, Rₕ, Z", m, m', 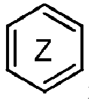 ring, 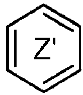 ring, X², n, Y² are as defined above and X is a fluorine atom.

According to procedure (D), the fluoroaryl derivative (III) may be placed in a polar solvent such as *N*,*N*-dimethylformamide. Phenol derivative (II') may then be added in a molar ratio ranging from 1 to 2 with respect to the fluoroaryl derivative (III) in presence of an inorganic base, such as Cs₂CO₃ or K₂CO₃, in particular in a molar ratio ranging from 1 to 5. The reaction mixture can then be heated at a temperature ranging from 50 to 150°C, for example at 70°C and stirred for a time ranging from 5 to 90 hours, for example during 16 hours, under inert gas and for example argon. The reaction mixture can be concentrated under reduced pressure and the residue can be partitioned between an organic solvent, such as dichloromethane, and water. The organic phase can be washed with water, decanted, dried over magnesium sulphate, filtered, concentrated under reduced pressure and purified to give a compound of formula (IV').

Accordingly, the present invention further relates to the synthesis process for manufacturing new compounds of formula (I) as defined above, wherein Q is O, comprising
- (i) at least a step of coupling a compound of formula (II') 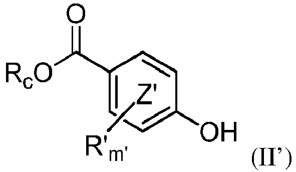 with a compound of formula (III) 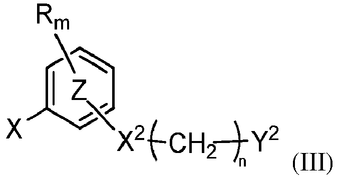 wherein R, R', R_{c}, Rₕ, R_{g}, m, m', n, 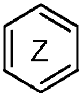 ring, 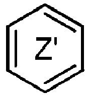 ring, X², Y² are as defined above and X is a fluorine atom, in presence of an inorganic base, to obtain a compound of formula (IV') 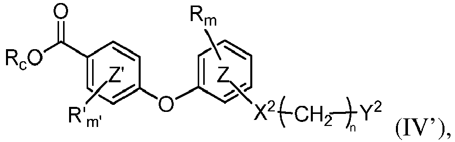 wherein Rc means an alkyl group, such as an ethyl group or a methyl group, preferentially a methyl group and R, R', m, m', n, 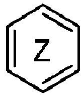 ring, 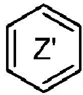 ring, X², Y² are as defined in anyone of claims 1 to 7, followed by a hydrolysis leading to a compound of formula 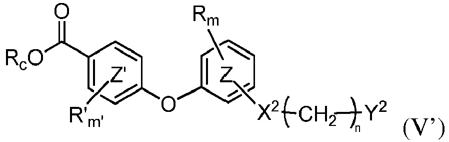 wherein Rc is a hydrogen atom and R, R', m, m', n, 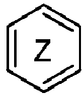 ring, 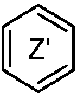 ring, X², Y² are as defined in anyone of claims 1 to 7, and
- (ii) a step of reacting a compound of formula (V') with a compound or formula 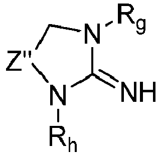 wherein Rg and Rh are as defined in claim 1, wherein Q is O, as defined above.

The starting compounds of formula (II), (II'), (III) are available or can be prepared according to methods known to the person skilled in the art.

More particularly, compounds of formula (II), when used to prepare compounds of formula (I), can be prepared according to scheme 2 below.

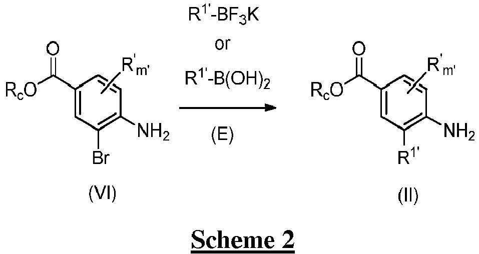

According to procedure (E), the compound of formula (VI) and an organometallic catalyst such as Pd(dppf)Cl₂.CH₂Cl₂ or Pd(OAc)₂ in an amount ranging from 2 mol% to 20 mol% relative to the amount of the compound of formula (VI) may be placed in a solvent such as 1,4-dioxane or a solvent mixture such as toluene and water. A boronic acid R^{1'}-B(OH)₂ or an organotrifluoroborate derivative R^{1'}-BF₃K may then be added in a molar ratio ranging from 1 to 5 with respect to the compound of formula (VI), in presence of an inorganic base, such as K₂CO₃ or K₃PO₄, in particular in a molar ratio ranging from 2 to 5, and in presence of a ligand, such as RuPhos, in particular in a molar ratio ranging from 2 to 5. The reaction mixture can then be heated at a temperature ranging from 50 to 150°C, for example at 110°C, and stirred for a time ranging from 2 to 70 hours, for example during 3 hours, under inert gas and for example argon. The reaction mixture can be concentrated under reduced pressure and purified to give a compound of formula (II).

More particularly, compounds of formula (II'), when used to prepare compounds of formula (I), can be prepared with a procedure similar to procedure (E) described above.

The chemical structures and spectroscopic data of some compounds of formula (I) of the invention are illustrated respectively in the following Table I and Table II.

**Table I**

| | |
|---|---|
| 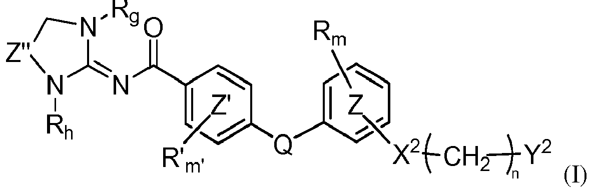 | |

| No | Structure |
|---|---|
| 1 | 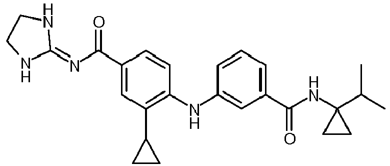 |
| 2 | 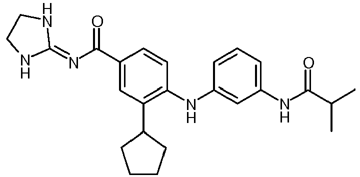 |
| 3 | 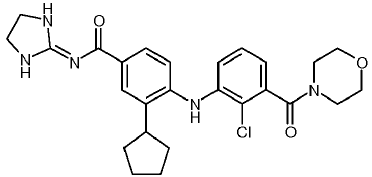 |
| 4 | 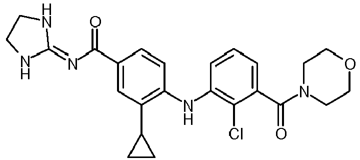 |
| 5 | 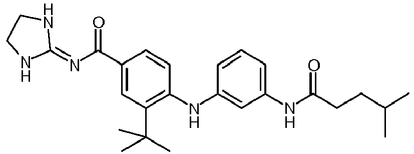 |
| 6 | 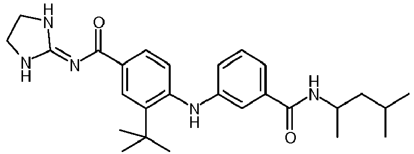 |
| 7 | 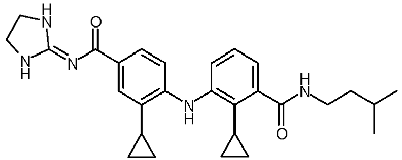 |
| 8 | 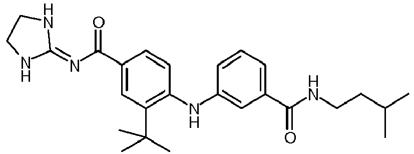 |
| 9 | 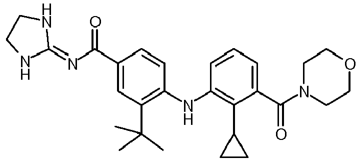 |
| 10 | 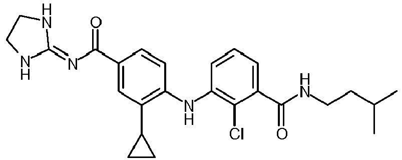 |
| 11 | 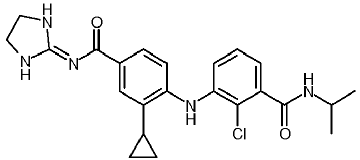 |
| 12 | 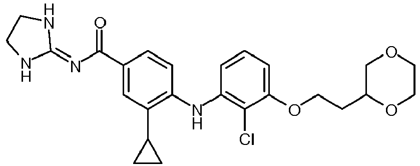 |
| 13 | 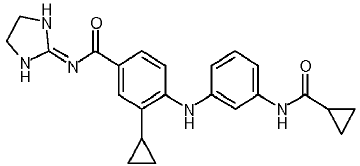 |
| 14 | 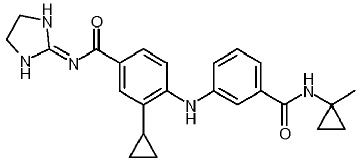 |
| 15 | 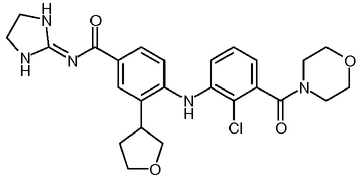 |
| 16 | 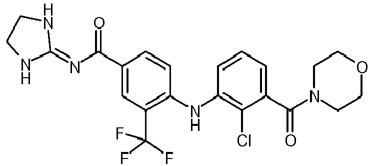 |
| 17 | 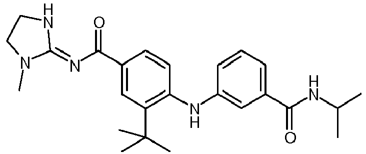 |
| 18 | 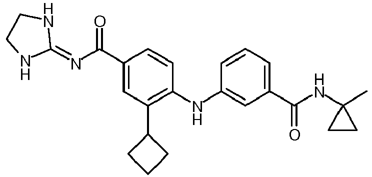 |
| 19 | 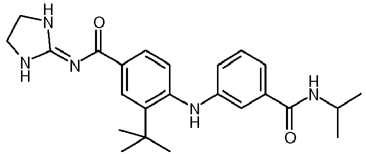 |
| 20 | 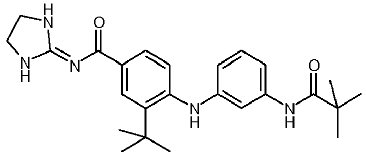 |
| 21 | 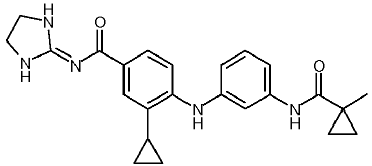 |
| 22 | 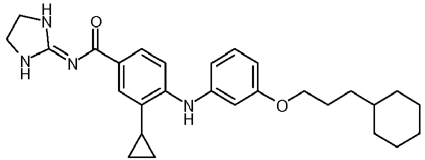 |
| 23 | 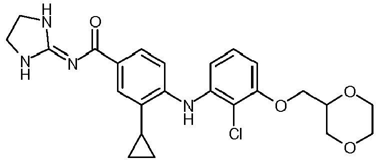 |
| 24 | 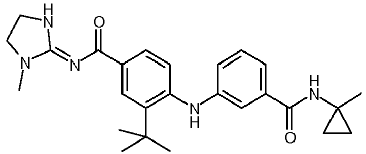 |
| 25 | 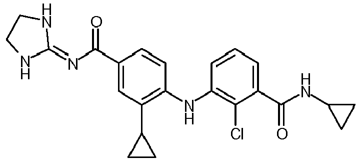 |
| 26 | 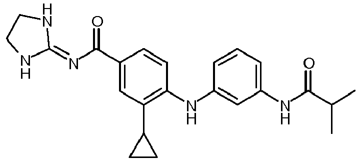 |
| 27 | 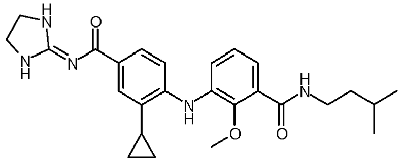 |
| 28 | 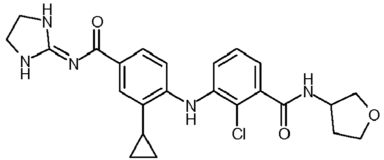 |
| 29 | 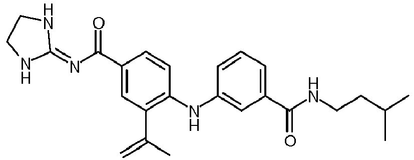 |
| 30 | 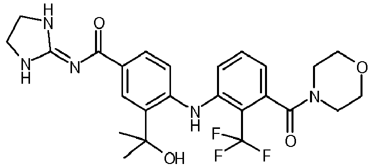 |
| 31 | 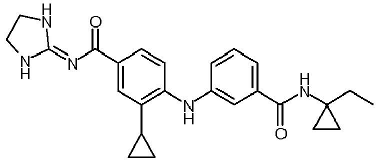 |
| 32 | 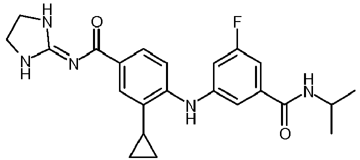 |
| 33 | 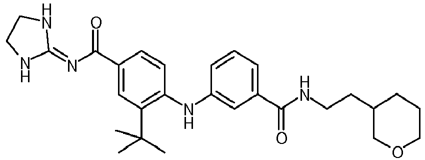 |
| 34 | 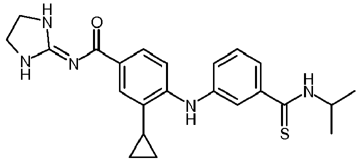 |
| 35 | 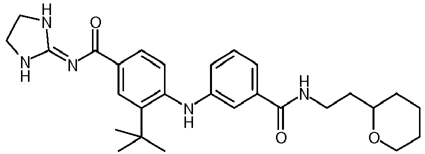 |
| 36 | 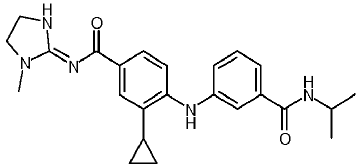 |
| 37 | 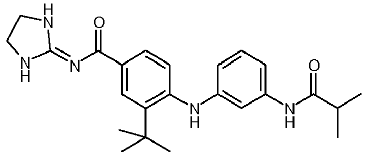 |
| 38 | 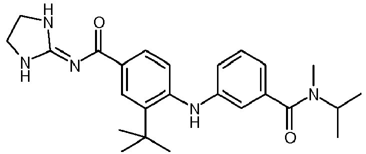 |
| 39 | 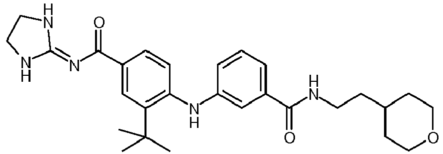 |
| 40 | 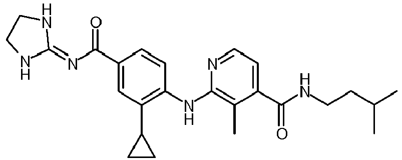 |
| 41 | 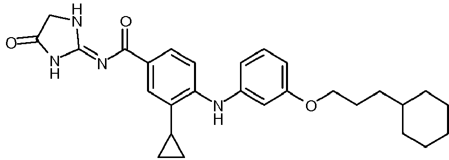 |
| 42 | 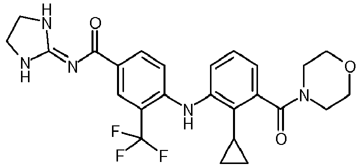 |
| 43 | 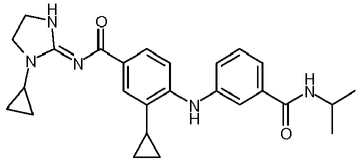 |
| 44 | 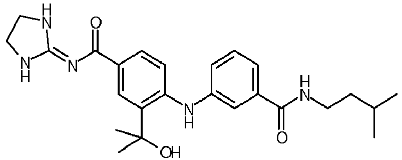 |
| 45 | 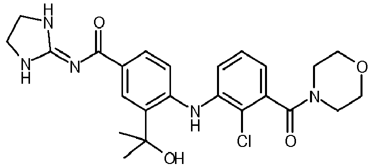 |
| 46 | 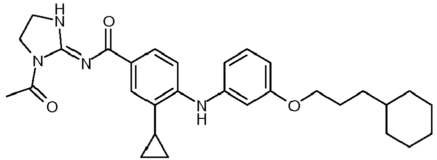 |
| 47 | 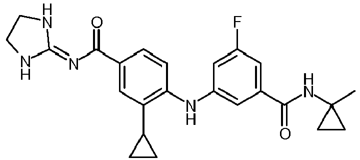 |
| 48 | 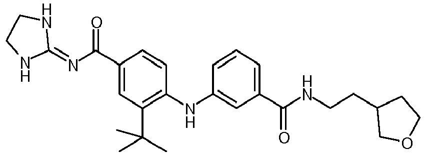 |
| 49 | 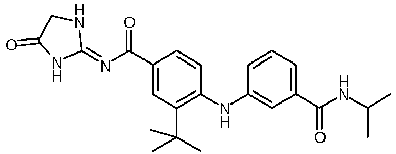 |
| 50 | 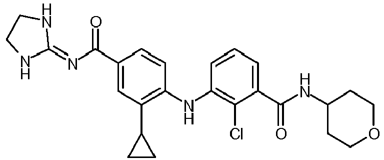 |
| 51 | 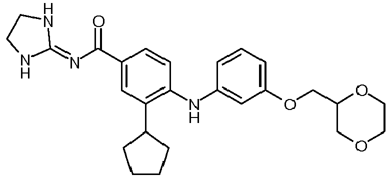 |
| 52 | 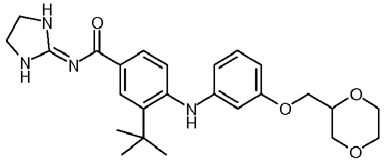 |
| 53 | 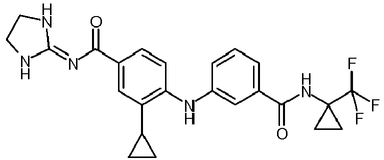 |
| 54 | 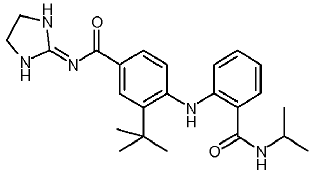 |
| 55 | 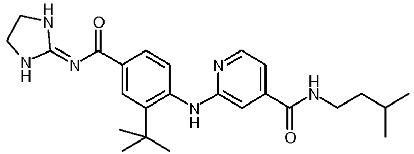 |
| 56 | 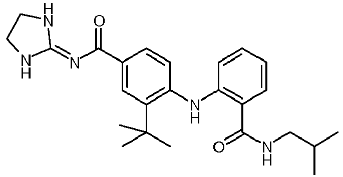 |
| 57 | 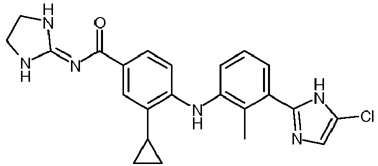 |
| 58 | 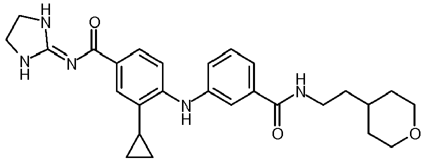 |
| 59 | 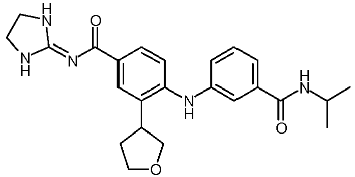 |
| 60 | 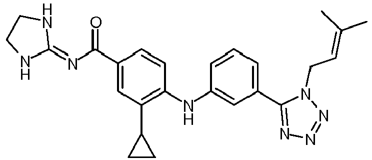 |
| 61 | 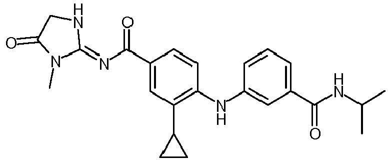 |
| 62 | 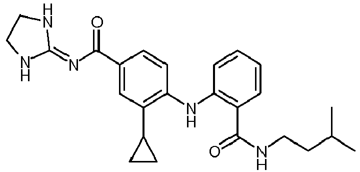 |
| 63 | 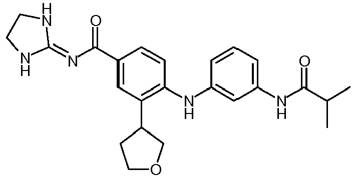 |
| 64 | 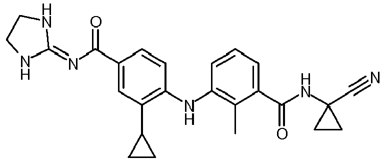 |
| 65 | 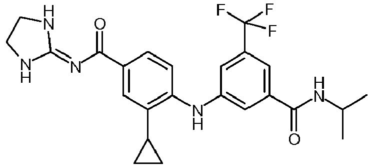 |
| 66 | 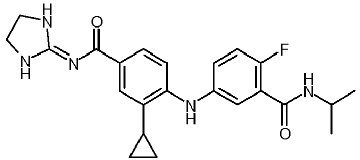 |
| 67 | 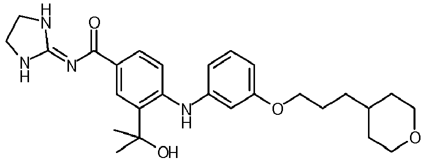 |
| 68 | 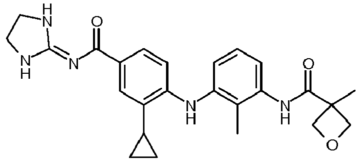 |
| 69 | 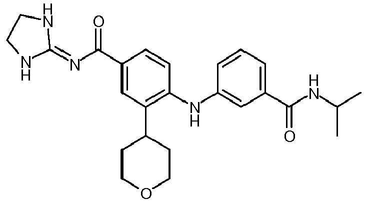 |
| 70 | 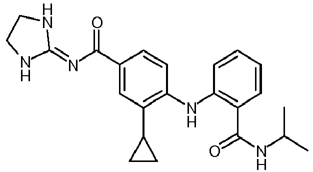 |
| 71 | 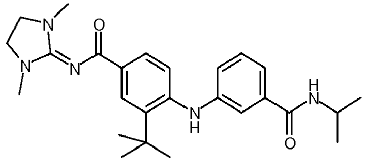 |
| 72 | 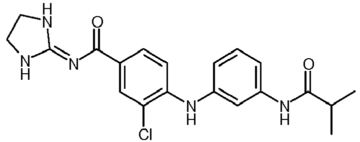 |
| 73 | 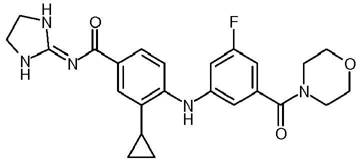 |
| 74 | 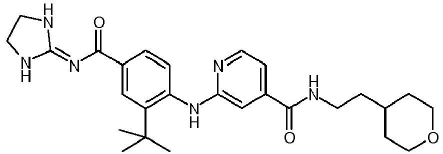 |
| 75 | 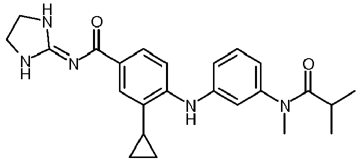 |
| 76 | 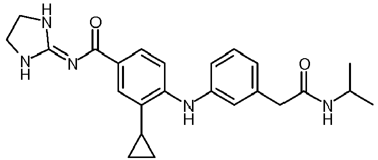 |
| 77 | 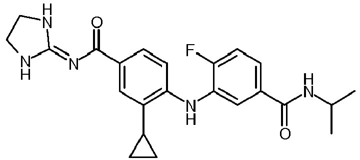 |
| 78 | 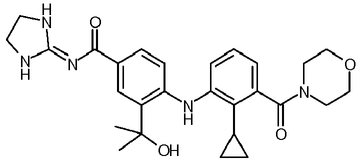 |
| 79 | 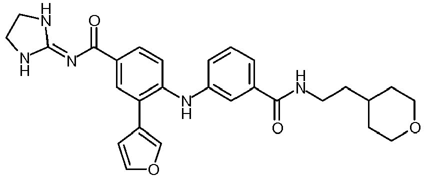 |
| 80 | 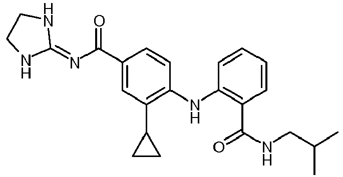 |
| 81 | 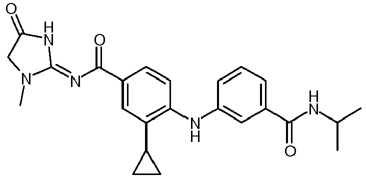 |
| 82 | 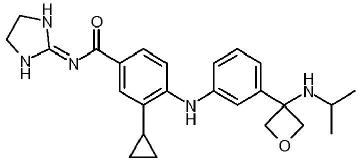 |
| 83 | 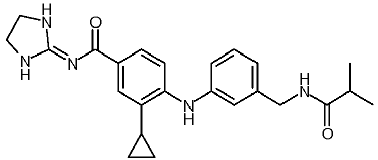 |
| 84 | 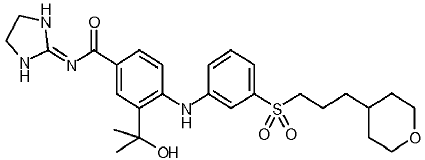 |
| 85 | 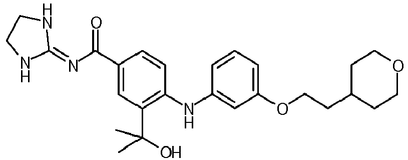 |
| 86 | 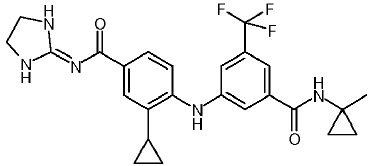 |
| 87 | 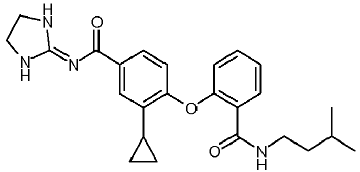 |
| 88 | 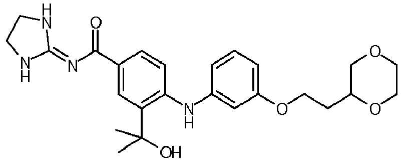 |
| 89 | 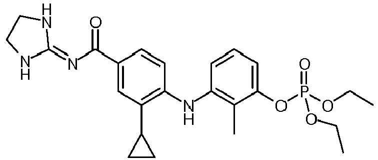 |
| 90 | 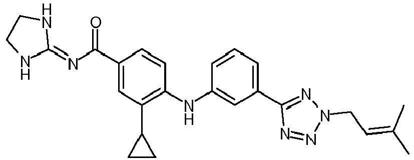 |
| 91 | 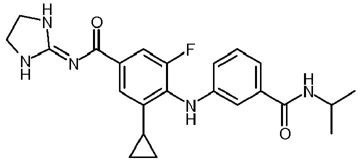 |
| 92 | 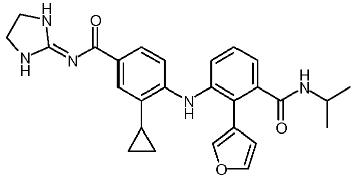 |
| 93 | 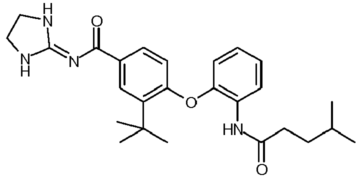 |
| 94 | 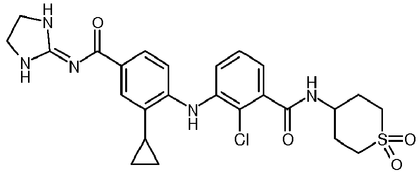 |
| 95 | 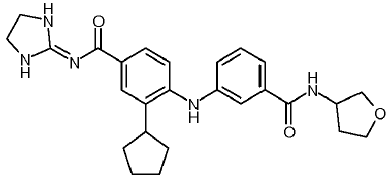 |
| 96 | 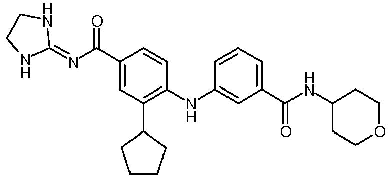 |
| 97 | 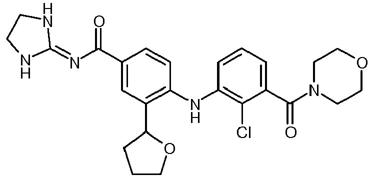 |
| 98 | 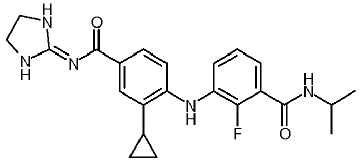 |
| 99 | 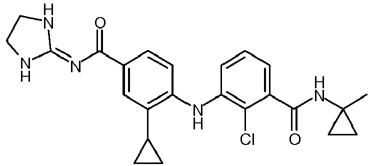 |
| 100 | 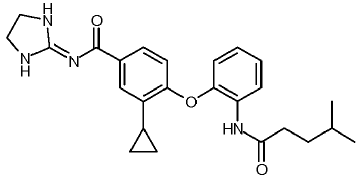 |
| 101 | 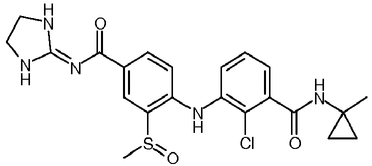 |
| 102 | 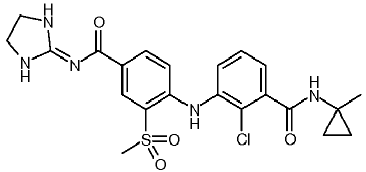 |
| 103 | 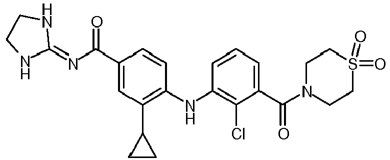 |
| 104 | 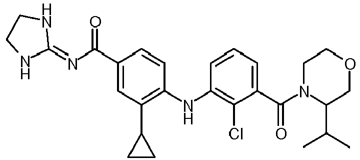 |
| 105 | 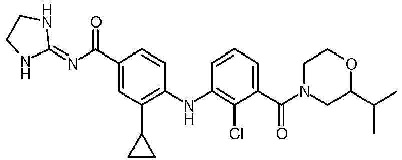 |
| 106 | 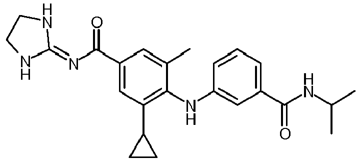 |
| 107 | 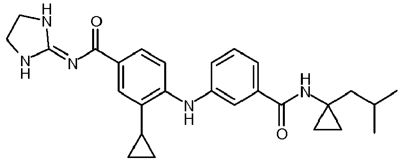 |
| 108 | 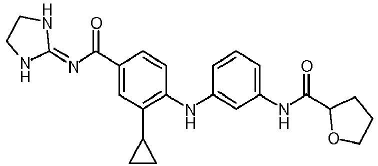 |
| 109 | 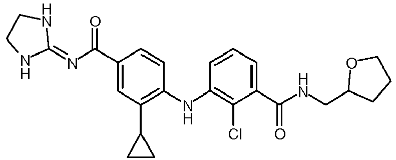 |
| 110 | 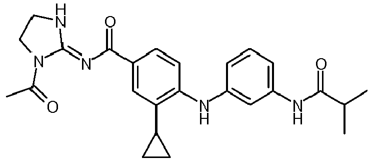 |
| 111 | 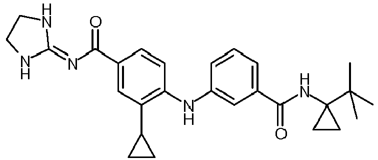 |

**Table II**

| **Ex** | **Characterizations** |
|---|---|
| **1** | ¹H NMR (400 MHz, *d₆*-DMSO) δ 8.38 (s, 1H), 8.09 (s, 2H), 7.79 (dd, J = 1.9, 8.4 Hz, 1H), 7.76 (s, 1H), 7.72 (d, J = 1.9 Hz, 1H), 7.53 - 7.49 (m, 1H), 7.31 - 7.26 (m, 2H), 7.21 - 7.15 (m, 1H), 7.11 (d, J = 8.5 Hz, 1H), 3.52 (s, 4H), 1.98 - 1.90 (m, 1H), 1.71 - 1.62 (m, 1H), 0.98 - 0.92 (m, 2H), 0.90 (d, J = 6.8 Hz, 6H), 0.70 - 0.65 (m, 4H), 0.61 - 0.55 (m, 2H). |
| | [M+H]⁺ = 446.2 |
| **2** | ¹H NMR (400 MHz, *d₆*-DMSO) δ 9.64 (s, 1H), 8.12 (s, 2H), 8.03 (d, J = 1.9 Hz, 1H), 7.80 (dd, J = 8.4, 1.9 Hz, 1H), 7.51 (s, 1H), 7.33 (s, 1H), 7.16 - 7.05 (m, 2H), 7.02 (d, J = 8.4 Hz, 1H), 6.63 (d, J = 7.9 Hz, 1H), 3.53 (s, 4H), 3.29 (s, 1H), 2.61 - 2.52 (m, 1H), 2.00 (d, J = 8.0 Hz, 2H), 1.84 - 1.71 (m, 2H), 1.64 (dd, J = 7.2, 4.7 Hz, 2H), 1.55 - 1.41 (m, 2H), 1.07 (d, J = 6.8 Hz, 6H). |
| | [M+H]⁺ = 434.0 |
| **3** | ¹H NMR (400 MHz, *d₆*-DMSO) δ 8.14 (s, 2H), 8.08 (s, 1H), 7.85 (d, J = 8.5 Hz, 1H), 7.29 (s, 1H), 7.20 (t, J = 7.8 Hz, 1H), 6.96 (d, J = 8.3 Hz, 1H), 6.78 (s, 2H), 3.66 (s, 4H), 3.56 (t, J = 4.7 Hz, 2H), 3.53 (s, 4H), 3.23 (d, J = 8.3 Hz, 1H), 3.19 (d, J = 5.1 Hz, 2H), 1.99 (s, 2H), 1.76 (s, 2H), 1.60 (d, J = 4.8 Hz, 2H), 1.52 (s, 2H). |
| | [M+H]⁺ = 496.0 |
| **4** | ¹H NMR (400 MHz, *d₆*-DMSO) δ 8.15 (s, 2H), 7.83 (d, J = 8.7 Hz, 1H), 7.77 (s, 1H), 7.44 (s, 1H), 7.31 (s, 1H), 7.13 (s, 1H), 7.03 (s, 1H), 6.92 (s, 1H), 3.67 (s, 4H), 3.61 - 3.49 (m, 6H), 3.20 (d, J = 5.5 Hz, 2H), 1.90 (d, J = 10.4 Hz, 1H), 0.95 (d, J = 8.3 Hz, 2H), 0.61 (s, 2H). |
| | [M+H]⁺ = 468.0 |
| **5** | ¹H NMR (400 MHz, *d₆*-DMSO) δ 9.61 (s, 1H), 8.18 (s, 1H), 8.13 (s, 2H), 7.86 (d, J = 8.1 Hz, 1H), 7.11 (d, J = 8.2 Hz, 1H), 7.01 (dd, J = 14.6, 6.9 Hz, 4H), 6.45 (d, J = 7.7 Hz, 1H), 3.54 (s, 4H), 2.26 - 2.20 (m, 2H), 1.52 (dd, J = 13.2, 6.4 Hz, 1H), 1.47 - 1.42 (m, 2H), 1.39 (s, 9H), 0.87 (d, J = 6.5 Hz, 6H). |
| | [M+H]⁺ = 450.2 |
| **6** | ¹H NMR (400 MHz, *d₆*-DMSO) δ 8.21 (d, J = 1.9 Hz, 1H), 8.13 (s, 2H), 7.95 (d, J = 8.5 Hz, 1H), 7.87 (dd, J = 8.2, 1.9 Hz, 1H), 7.25 - 7.21 (m, 1H), 7.21 - 7.06 (m, 4H), 6.81 - 6.76 (m, 1H), 4.16 - 3.99 (m, 1H), 3.53 (s, 4H), 1.63 - 1.46 (m, 2H), 1.39 (s, 9H), 1.22 (ddd, J = 13.5, 8.3, 5.4 Hz, 1H), 1.09 (d, J = 6.6 Hz, 3H), 0.87 (dd, J = 6.5, 1.9 Hz, 6H). |
| | [M+H]⁺ = 464.3 |
| **7** | ¹H NMR (400 MHz, *d₆*-DMSO) δ 8.14 (t, J = 5.5 Hz, 1H), 8.08 (s, 2H), 7.82 (d, J = 8.5 Hz, 2H), 7.26 - 7.15 (m, 3H), 7.03 (d, J = 8.2 Hz, 1H), 6.84 (d, J = 6.2 Hz, 1H), 3.51 (s, 4H), 3.25 (q, J = 6.5 Hz, 2H), 1.96 - 1.78 (m, 2H), 1.67 (dp, J = 13.3, 6.7 Hz, 1H), 1.43 (q, J = 7.0 Hz, 2H), 1.04 - 0.95 (m, 2H), 0.92 (d, J = 6.6 Hz, 6H), 0.87 (d, J = 8.6 Hz, 2H), 0.62 (q, J = 5.4 Hz, 2H), 0.45 (q, J = 5.4 Hz, 2H). |
| | ¹³C NMR (151 MHz, *d₆*-DMSO) δ 175.7, 169.6, 165.7, 145.6, 143.1, 141.8, 130.6, 129.6, 129.0, 128.7, 128.2, 127.1, 120.5, 119.0, 114.2, 41.7, 38.4, 37.7, 25.8, 22.9, 11.9, 11.0, 7.27, 6.5 |
| | [M+H]⁺ = 474.2 |
| **8** | ¹H NMR (400 MHz, *d₆*-DMSO) δ 8.21 (d, J = 8.0 Hz, 2H), 8.14 (s, 2H), 7.87 (d, J = 8.3 Hz, 1H), 7.23 - 7.09 (m, 5H), 6.83 (s, 1H), 3.54 (s, 4H), 3.23 (q, J = 6.5 Hz, 2H), 1.60 (dt, J = 13.6, 6.8 Hz, 1H), 1.40 (s, 11H), 0.89 (d, J = 6.6 Hz, 6H). |
| | [M+H]⁺ = 450.2 |
| **9** | ¹H NMR (400 MHz, *d₆*-DMSO) δ 8.21 (d, J = 1.9 Hz, 1H), 8.12 (s, 2H), 7.87 (dd, J = 8.2, 1.9 Hz, 1H), 7.09 (dt, J = 7.8, 3.7 Hz, 2H), 6.67 (dd, J = 8.2, 1.1 Hz, 1H), 6.60 (dd, J = 7.5, 1.1 Hz, 1H), 6.55 (s, 1H), 3.77 - 3.47 (m, 10H), 3.26 (dt, J = 7.1, 4.3 Hz, 2H), 1.77 (ddd, J = 14.0, 8.3, 5.8 Hz, 1H), 1.43 (s, 9H), 1.00 - 0.89 (m, 2H), 0.69 (s, 1H), 0.34 (dd, J = 8.9, 4.0 Hz, 1H). |
| | [M+H]⁺ = 490.3 |
| **10** | ¹H NMR (500 MHz, *d₆*-DMSO) δ 8.36 (t, J = 5.7 Hz, 1H), 8.10 (s, 2H), 7.87 - 7.77 (m, 2H), 7.32 (s, 1H), 7.27 - 7.20 (m, 1H), 7.10 (dd, J = 8.2, 1.4 Hz, 1H), 7.04 (d, J = 8.3 Hz, 1H), 6.88 (dd, J = 7.4, 1.5 Hz, 1H), 3.52 (s, 4H), 3.27 - 3.20 (m, 2H), 1.87 (ddd, J = 13.7, 8.3, 5.4 Hz, 1H), 1.68 (dp, J = 13.4, 6.7 Hz, 1H), 1.41 (q, J = 7.0 Hz, 2H), 0.98 - 0.88 (m, 8H), 0.63 - 0.56 (m, 2H). |
| | [M+H]⁺ = 468.1 |
| **11** | ¹H NMR (400 MHz, *d₆*-DMSO) δ 8.28 (d, J = 7.8 Hz, 1H), 8.11 (s, 2H), 7.85 (dd, J = 8.3, 1.9 Hz, 1H), 7.80 (d, J = 1.8 Hz, 1H), 7.32 (s, 1H), 7.28 - 7.20 (m, 1H), 7.10 (dd, J = 8.2, 1.5 Hz, 1H), 7.05 (d, J = 8.3 Hz, 1H), 6.89 (dd, J = 7.4, 1.5 Hz, 1H), 4.05 (d, J = 7.7 Hz, 1H), 3.52 (s, 4H), 1.94 - 1.82 (m, 1H), 1.15 (d, J = 6.6 Hz, 6H), 0.99 - 0.90 (m, 2H), 0.65 - 0.57 (m, 2H). |
| | [M+H]⁺ = 440.0 |
| **12** | ¹H NMR (400 MHz, *d₆*-DMSO) δ 8.11 (s, 2H), 7.83 (dd, J = 8.3, 1.9 Hz, 1H), 7.80 (d, J = 1.8 Hz, 1H), 7.21 (s, 1H), 7.17 (t, J = 8.3 Hz, 1H), 7.04 (d, J = 8.3 Hz, 1H), 6.81 - 6.75 (m, 1H), 6.75 - 6.69 (m, 1H), 4.19 - 4.08 (m, 2H), 3.80 (dd, J = 11.4, 2.5 Hz, 1H), 3.76 - 3.70 (m, 2H), 3.65 (d, J = 11.2 Hz, 1H), 3.56 (dd, J = 11.4, 2.4 Hz, 1H), 3.52 (s, 4H), 3.47 (td, J = 11.0, 2.6 Hz, 1H), 3.27 (dd, J = 11.4, 9.9 Hz, 1H), 1.92 - 1.82 (m, 2H), 1.79 (dd, J = 13.8, 8.2 Hz, 1H), 0.98 - 0.92 (m, 2H), 0.62 - 0.56 (m, 2H). |
| | [M+H]⁺ = 485.0 |
| **13** | ¹H NMR (400 MHz, *d₆*-DMSO) δ 10.05 (s, 1H), 8.10 (s, 2H), 7.78 (dd, J = 8.4, 2.0 Hz, 1H), 7.70 (d, J = 1.9 Hz, 1H), 7.62 (s, 1H), 7.44 (s, 1H), 7.17 - 7.10 (m, 2H), 7.08 (s, 1H), 6.75 (d, J = 8.9 Hz, 1H), 3.52 (s, 4H), 1.96 (ddd, J = 13.7, 8.3, 5.3 Hz, 1H), 1.81 - 1.70 (m, 1H), 0.99 - 0.89 (m, 2H), 0.76 (dt, J = 8.6, 2.5 Hz, 4H), 0.61 - 0.52 (m, 2H). |
| | [M+H]⁺ = 404.0 |
| **14** | ¹H NMR (400 MHz, *d₆*-DMSO) δ 8.53 (s, 1H), 8.11 (s, 2H), 7.79 (dd, J = 8.4, 1.9 Hz, 2H), 7.72 (d, J = 1.8 Hz, 1H), 7.52 (s, 1H), 7.28 (d, J = 5.0 Hz, 2H), 7.22 - 7.14 (m, 1H), 7.11 (d, J = 8.4 Hz, 1H), 3.52 (s, 4H), 1.94 (ddd, J = 13.8, 8.3, 5.4 Hz, 1H), 1.35 (s, 3H), 0.98 - 0.91 (m, 2H), 0.75 - 0.68 (m, 2H), 0.58 (td, J = 6.3, 4.3 Hz, 4H). |
| | [M+H]⁺ = 418.0 |
| **15** | ¹H NMR (400 MHz, *d₆*-DMSO) δ 8.15 (s, 2H), 8.08 (d, J = 1.9 Hz, 1H), 7.88 (dd, J = 8.3, 1.8 Hz, 1H), 7.47 (s, 1H), 7.24 - 7.17 (m, 1H), 6.98 (d, J = 7.7 Hz, 1H), 6.81 (dd, J = 7.5, 1.3 Hz, 1H), 6.74 (dd, J = 8.2, 1.4 Hz, 1H), 3.95 (td, J = 8.7, 8.3, 4.9 Hz, 2H), 3.74 (t, J = 7.6 Hz, 1H), 3.66 (s, 4H), 3.61 (dt, J = 5.3, 3.3 Hz, 2H), 3.56 (t, J = 4.9 Hz, 2H), 3.53 (s, 4H), 3.23 - 3.14 (m, 2H), 2.28 (dd, J = 11.9, 7.0 Hz, 1H), 1.93 - 1.83 (m, 1H). |
| | [M+H]⁺ = 497.9 |
| **16** | ¹H NMR (400 MHz, *d₆*-DMSO) δ 8.35 (d, J = 1.9 Hz, 1H), 8.17 (s, 2H), 8.10 (d, J = 8.6 Hz, 1H), 7.56 (s, 1H), 7.43 - 7.37 (m, 1H), 7.30 (dd, J = 8.1, 1.6 Hz, 1H), 7.13 (dd, J = 7.5, 1.6 Hz, 1H), 6.88 (d, J = 8.6 Hz, 1H), 3.66 (d, J = 2.7 Hz, 4H), 3.57 - 3.54 (m, 2H), 3.53 (s, 4H), 3.18 (d, J = 3.3 Hz, 2H). |
| | [M+H]⁺ = 495.9 |
| **17** | ¹H NMR (500 MHz, *d₆*-DMSO) δ 8.64 (s, 1 H), 8.28 (d, J = 2.2 Hz, 1 H), 8.04 (d, J = 8.0 Hz, 1 H), 7.94 (dd, J = 8.2, 1.9 Hz, 1 H), 7.23 (t, J = 1.9 Hz, 1 H), 7.20 - 7.17 (m, 1 H), 7.17 - 7.15 (m, 1 H), 7.14 - 7.12 (m, 1 H), 7.11 (d, J = 8.0 Hz, 1 H), 6.85 - 6.76 (m, 1 H), 4.13 - 3.97 (m, 1 H), 3.62-3.51 (m, 2 H), 3.49 - 3.41 (m, 2 H), 2.95 (s, 3 H), 1.39 (s, 9 H), 1.13 (d, J = 6.6 Hz, 6 H). |
| | [M+H]⁺ = 436.5 |
| **18** | ¹H NMR (400 MHz, *d₆*-DMSO) δ 8.52 (s, 1H), 8.12 (s, 2H), 8.02 (d, J = 1.8 Hz, 1H), 7.82 (dd, J = 8.4, 1.9 Hz, 1H), 7.48 (s, 1H), 7.43 - 7.37 (m, 1H), 7.22 (s, 2H), 7.13 - 7.02 (m, 2H), 3.70 (q, J = 8.5 Hz, 1H), 3.53 (s, 4H), 2.39 - 2.28 (m, 2H), 2.05 - 1.86 (m, 3H), 1.82 - 1.74 (m, 1H), 1.35 (s, 3H), 0.75 - 0.67 (m, 2H), 0.62 - 0.54 (m, 2H). |
| | [M+H]⁺ = 432.0 |
| **19** | ¹H NMR (400 MHz, *d₆*-DMSO) δ 8.20 (d, J = 1.7 Hz, 1H), 8.13 (s, 2H), 8.03 (d, J = 7.7 Hz, 1H), 7.87 (dd, J = 8.2, 1.8 Hz, 1H), 7.24 (s, 1H), 7.16 (t, J = 6.0 Hz, 3H), 7.10 (d, J = 8.2 Hz, 1H), 6.80 (s, 1H), 4.11 -4.00 (m, 1H), 3.54 (s, 4H), 1.39 (s, 9H), 1.14 (d, J = 6.6 Hz, 6H). |
| | [M+H]⁺ = 422.1 |
| **20** | ¹H NMR (400 MHz, *d₆*-DMSO) δ 8.96 (s, 1H), 8.25 - 8.04 (m, 3H), 7.87 (dd, J = 8.2, 1.9 Hz, 1H), 7.17 - 6.96 (m, 4H), 6.92 (s, 1H), 6.47 (dt, J = 7.2, 2.0 Hz, 1H), 3.53 (s, 4H), 1.40 (s, 9H), 1.18 (s, 9H). |
| | [M+H]⁺ = 436.3 |
| **21** | ¹H NMR (400 MHz, *d₆*-DMSO) δ 8.11 (s, 2H), 7.80 (s, 2H), 7.22 (s, 1H), 7.17 (t, J = 8.3 Hz, 1H), 7.03 (d, J = 8.3 Hz, 1H), 6.77 (d, J = 8.2 Hz, 1H), 6.73 (d, J = 8.2 Hz, 1H), 4.06 (qd, J = 10.5, 4.8 Hz, 2H), 3.96 - 3.83 (m, 2H), 3.83 - 3.75 (m, 1H), 3.72 - 3.60 (m, 2H), 3.49 (q, J = 11.7, 10.6 Hz, 6H), 1.87 (ddd, J = 13.8, 8.4, 5.5 Hz, 1H), 0.99 - 0.90 (m, 2H), 0.59 (q, J = 5.6 Hz, 2H). |
| | [M+H]⁺ = 418.3 |
| **22** | ¹H NMR (400 MHz, *d₆*-DMSO) δ 8.08 (s, 2H), 7.78 (dd, J = 8.4, 1.9 Hz, 1H), 7.70 (d, J = 1.9 Hz, 1H), 7.58 (s, 1H), 7.17 - 7.07 (m, 2H), 6.65 (dd, J = 8.0, 1.5 Hz, 1H), 6.61 (t, J = 2.1 Hz, 1H), 6.42 (dd, J = 8.0, 2.1 Hz, 1H), 3.89 (t, J = 6.5 Hz, 2H), 3.51 (s, 4H), 1.94 (ddd, J = 13.7, 8.4, 5.4 Hz, 1H), 1.67 (dd, J = 18.2, 8.9 Hz, 7H), 1.31 - 1.12 (m, 6H), 0.96 - 0.81 (m, 4H), 0.61 - 0.52 (m, 2H). |
| | [M+H]⁺ = 461.2 |
| **23** | ¹H NMR (400 MHz, *d₆*-DMSO) δ 8.11 (s, 2H), 7.80 (s, 2H), 7.22 (s, 1H), 7.17 (t, J = 8.3 Hz, 1H), 7.03 (d, J = 8.3 Hz, 1H), 6.77 (d, J = 8.2 Hz, 1H), 6.73 (d, J = 8.2 Hz, 1H), 4.06 (qd, J = 10.5, 4.8 Hz, 2H), 3.96 - 3.83 (m, 2H), 3.83 - 3.75 (m, 1H), 3.72 - 3.60 (m, 2H), 3.49 (q, J = 11.7, 10.6 Hz, 6H), 1.87 (ddd, J = 13.8, 8.4, 5.5 Hz, 1H), 0.99 - 0.90 (m, 2H), 0.59 (q, J = 5.6 Hz, 2H). |
| | [M+H]⁺ = 471.0 |
| **24** | ¹H NMR (400 MHz, *d₆*-DMSO) δ 8.66 (s, 1H), 8.48 (s, 1H), 8.31 (d, J = 1.9 Hz, 1H), 7.96 (dd, J = 8.1, 1.9 Hz, 1H), 7.25 - 7.05 (m, 5H), 6.83 (dd, J = 7.7, 2.3 Hz, 1H), 3.68 - 3.53 (m, 2H), 3.52 - 3.42 (m, 2H), 2.97 (s, 3H), 1.41 (s, 9H), 1.35 (s, 3H), 0.75 - 0.68 (m, 2H), 0.61 - 0.56 (m, 2H). |
| | [M+H]⁺ = 448.2 |
| **25** | ¹H NMR (400 MHz, *d₆*-DMSO) δ 8.45 (d, J = 4.5 Hz, 1H), 8.11 (s, 2H), 7.85 (dd, J = 8.3, 1.9 Hz, 1H), 7.79 (d, J = 1.8 Hz, 1H), 7.33 (s, 1H), 7.23 (s, 1H), 7.10 (d, J = 1.5 Hz, 1H), 7.04 (d, J = 8.3 Hz, 1H), 6.92 - 6.85 (m, 1H), 3.52 (s, 4H), 2.87 - 2.79 (m, 1H), 1.87 (s, 1H), 0.98 - 0.91 (m, 2H), 0.70 (td, J = 7.1, 4.8 Hz, 2H), 0.60 (dd, J = 5.5, 1.8 Hz, 2H), 0.56 - 0.50 (m, 2H). |
| | ¹³C NMR (151 MHz, *d₆*-DMSO) δ 175.5, 168.1, 165.8, 144.0, 140.9, 138.9, 132.9, 132.6, 127.9, 127.8, 120.3, 119.5, 118.8, 118.1, 41.7, 23.1, 11.7, 7.3, 6.1 |
| | [M+H]⁺ = 438.0 |
| **26** | ¹H NMR (400 MHz, *d₆*-DMSO) δ 9.68 (s, 1H), 8.08 (s, 2H), 7.79 (dd, J = 8.4, 1.9 Hz, 1H), 7.71 (d, J = 1.9 Hz, 1H), 7.60 (s, 1H), 7.45 (d, J = 1.8 Hz, 1H), 7.19 - 7.05 (m, 3H), 6.75 (d, J = 8.3 Hz, 1H), 3.51 (s, 4H), 2.64 - 2.52 (m, 1H), 1.95 (tt, J = 8.4, 5.4 Hz, 1H), 1.08 (d, J = 6.8 Hz, 6H), 0.99 - 0.89 (m, 2H), 0.61 - 0.52 (m, 2H). |
| | ¹³C NMR (151 MHz, *d₆*-DMSO) δ 175.6, 146.1, 144.0, 140.6, 131.1, 129.5, 127.8, 127.5, 116.0, 114.2, 112.2, 109.9, 41.9, 35.3, 19.9, 11.6,7.6 |
| | [M+H]⁺ = 406.1 |
| **27** | ¹H NMR (400 MHz, *d₆*-DMSO) δ 8.22 (t, J = 5.7 Hz, 1H), 8.10 (s, 2H), 7.83 (s, 2H), 7.26 (s, 2H), 7.14 - 7.01 (m, 3H), 3.74 (s, 3H), 3.52 (s, 4H), 3.30 - 3.23 (m, 2H), 1.89 (ddd, J = 13.7, 8.2, 5.3 Hz, 1H), 1.66 (dp, J = 13.4, 6.7 Hz, 1H), 1.43 (q, J = 7.0 Hz, 2H), 1.03 - 0.95 (m, 2H), 0.92 (d, J = 6.6 Hz, 6H), 0.65 - 0.56 (m, 2H). |
| | [M+H]⁺ = 464.2 |
| **28** | ¹H NMR (400 MHz, *d₆*-DMSO) δ 8.67 (d, J = 6.7 Hz, 1H), 8.11 (s, 2H), 7.84 (s, 1H), 7.80 (s, 1H), 7.34 (s, 1H), 7.29 - 7.20 (m, 1H), 7.13 - 7.08 (m, 1H), 7.05 (d, J = 8.4 Hz, 1H), 6.91 (dd, J = 7.4, 1.5 Hz, 1H), 4.42 (s, 1H), 3.87 (dd, J = 8.9, 6.2 Hz, 1H), 3.80 (t, J = 7.6 Hz, 1H), 3.71 (d, J = 5.5 Hz, 1H), 3.59 (dd, J = 8.9, 4.1 Hz, 1H), 3.52 (s, 4H), 2.15 (dd, J = 12.8, 7.6 Hz, 1H), 1.87 (s, 2H), 0.95 (dd, J = 8.3, 2.0 Hz, 2H), 0.61 (d, J = 3.6 Hz, 2H). |
| | [M+H]⁺ = 468.0 |
| **29** | ¹H NMR (400 MHz, *d₆*-DMSO) δ 8.27 (t, J = 5.7 Hz, 1H), 8.11 (s, 2H), 7.91 (d, J = 1.9 Hz, 1H), 7.88 (dd, J = 8.4, 2.0 Hz, 1H), 7.57 (s, 1H), 7.48 (s, 1H), 7.27 (d, J = 5.1 Hz, 2H), 7.16 (d, J = 8.5 Hz, 1H), 7.14 (d, J = 3.7 Hz, 1H), 5.19 (s, 1H), 5.04 (s, 1H), 3.52 (s, 4H), 3.29 - 3.21 (m, 2H), 2.00 (s, 3H), 1.61 (dp, J = 13.0, 6.9 Hz, 1H), 1.40 (q, J = 7.0 Hz, 2H), 0.90 (d, J = 6.6 Hz, 6H). |
| | [M+H]⁺ = 434.3 |
| **30** | ¹H NMR (400 MHz, *d₆*-DMSO) δ 9.32 (s, 1H), 8.11 (s, 2H), 8.05 (d, J = 1.9 Hz, 1H), 7.89 (dd, J = 8.4, 1.9 Hz, 1H), 7.53 (t, J = 7.9 Hz, 1H), 7.45 (d, J = 8.4 Hz, 1H), 7.19 (d, J = 8.4 Hz, 1H), 6.87 (d, J = 7.3 Hz, 1H), 5.96 (s, 1H), 3.70 - 3.46 (m, 10H), 3.28 - 3.19 (m, 1H), 3.14 (dq, J = 10.1, 3.4 Hz, 1H), 1.56 (d, J = 11.9 Hz, 6H). |
| | [M+H]⁺ = 520.0 |
| **31** | ¹H NMR (400 MHz, *d₆*-DMSO) δ 8.48 (s, 1H), 8.09 (s, 2H), 7.83 - 7.73 (m, 2H), 7.71 (d, J = 1.8 Hz, 1H), 7.54 - 7.48 (m, 1H), 7.33 - 7.24 (m, 2H), 7.20 - 7.14 (m, 1H), 7.10 (d, J = 8.4 Hz, 1H), 3.51 (s, 4H), 1.99 - 1.89 (m, 1H), 1.59 (q, J = 7.5 Hz, 2H), 0.97 - 0.91 (m, 2H), 0.89 (t, J = 7.4 Hz, 3H), 0.72 - 0.65 (m, 2H), 0.63 - 0.53 (m, 4H). |
| | [M+H]⁺ = 432.2 |
| **32** | ¹H NMR (400 MHz, *d₆*-DMSO) δ 8.19 (d, J = 7.7 Hz, 1H), 8.15 - 8.07 (m, 3H), 7.83 (dd, J = 2.0, 8.4 Hz, 1H), 7.71 (d, J = 2.0 Hz, 1H), 7.33 (t, J = 1.7 Hz, 1H), 7.19 (d, J = 8.4 Hz, 1H), 7.09 - 7.01 (m, 1H), 6.84 (td, J = 2.2, 11.3 Hz, 1H), 4.12 - 3.96 (m, 1H), 3.52 (s, 4H), 2.00 - 1.89 (m, 1H), 1.15 (d, J = 6.6 Hz, 6H), 1.00 - 0.89 (m, 2H), 0.62 - 0.56 (m, 2H). |
| | [M+H]⁺ = 424.1 |
| **33** | ¹H NMR (400 MHz, *d₆*-DMSO) δ 8.26 (t, J = 5.7 Hz, 1H), 8.21 (d, J = 1.7 Hz, 1H), 8.13 (s, 2H), 7.88 (dd, J = 8.1, 1.6 Hz, 1H), 7.19 (dd, J = 14.4, 6.0 Hz, 3H), 7.11 (dd, J = 7.9, 4.2 Hz, 2H), 6.81 (d, J = 7.6 Hz, 1H), 3.75 (t, J = 11.7 Hz, 2H), 3.53 (s, 4H), 3.30 - 3.17 (m, 3H), 2.98 (t, J = 10.4 Hz, 1H), 1.83 (d, J = 12.6 Hz, 1H), 1.58 - 1.45 (m, 3H), 1.39 (s, 9H), 1.38 - 1.28 (m, 2H), 1.12 (qd, J = 11.2, 4.4 Hz, 1H). |
| | [M+H]⁺ = 492.1 |
| **34** | ¹H NMR (400 MHz, *d₆*-DMSO) δ 10.04 - 9.95 (m, 1H), 8.08 (s, 2H), 7.86 - 7.76 (m, 2H), 7.71 (d, J = 1.9 Hz, 1H), 7.42 (t, J = 1.9 Hz, 1H), 7.30 - 7.21 (m, 1H), 7.17 - 7.06 (m, 3H), 4.76 - 4.58 (m, 1H), 3.51 (s, 4H), 2.01 - 1.91 (m, 1H), 1.24 (d, J = 6.6 Hz, 6H), 1.00 - 0.91 (m, 2H), 0.64 - 0.52 (m, 2H). |
| | [M+H]⁺ = 422.1 |
| **35** | ¹H NMR (400 MHz, *d₆*-DMSO) δ 8.21 (d, J = 1.8 Hz, 2H), 8.13 (s, 2H), 7.88 (dd, J = 8.1, 1.8 Hz, 1H), 7.24 - 7.13 (m, 3H), 7.10 (d, J = 8.2 Hz, 2H), 6.82 (d, J = 9.5 Hz, 1H), 3.83 (d, J = 10.7 Hz, 1H), 3.53 (s, 4H), 3.25 (td, J = 13.1, 12.4, 5.5 Hz, 4H), 1.74 (s, 1H), 1.59 (p, J = 8.9, 7.9 Hz, 3H), 1.39 (s, 12H), 1.16 (d, J = 11.3 Hz, 1H). |
| | [M+H]⁺ = 492.3 |
| **36** | ¹H NMR (400 MHz, *d₆*-DMSO) δ 8.62 (s, 1H), 8.13 (d, J = 7.8 Hz, 1H), 7.88 (d, J = 8.5 Hz, 1H), 7.81 (s, 1H), 7.76 (s, 1H), 7.55 (s, 1H), 7.31 (q, J = 7.9 Hz, 2H), 7.20 (d, J = 7.2 Hz, 1H), 7.12 (d, J = 8.4 Hz, 1H), 4.08 (dq, J = 13.4, 6.8 Hz, 1H), 3.59 - 3.50 (m, 2H), 3.48 - 3.39 (m, 2H), 2.95 (s, 3H), 1.96 (ddd, J = 13.6, 8.3, 5.5 Hz, 1H), 1.15 (d, J = 6.6 Hz, 6H), 1.01 - 0.91 (m, 2H), 0.60 (d, J = 5.0 Hz, 2H). |
| | [M+H]⁺ = 420.1 |
| **37** | ¹H NMR (400 MHz, *d₆*-DMSO) δ 9.56 (s, 1H), 8.21 - 8.06 (m, 3H), 7.87 (dd, J = 8.2, 1.9 Hz, 1H), 7.11 (d, J = 8.2 Hz, 1H), 7.06 - 6.96 (m, 4H), 6.46 (dt, J = 7.6, 1.6 Hz, 1H), 3.54 (s, 4H), 2.56 - 2.52 (m, 1H), 1.39 (s, 9H), 1.04 (d, J = 6.8 Hz, 6H). |
| | [M+H]⁺ = 422.3 |
| **38** | ¹H NMR (400 MHz, *d₆*-DMSO) δ 8.20 (d, J = 1.9 Hz, 1H), 8.13 (s, 2H), 7.88 (dd, J = 8.1, 1.9 Hz, 1H), 7.26 - 7.10 (m, 3H), 6.89 - 6.45 (m, 3H), 4.77 - 3.79 (m, 1H), 3.53 (s, 4H), 2.73 (s, 3H), 1.39 (s, 9H), 1.10 - 1.01 (m, 6H). |
| | [M+H]⁺ = 436.1 |
| **39** | ¹H NMR (400 MHz, *d₆*-DMSO) δ 8.28 - 8.18 (m, 2H), 8.13 (s, 2H), 7.88 (dd, J = 8.2, 1.8 Hz, 1H), 7.24 - 7.15 (m, 3H), 7.11 (dd, J = 7.9, 4.8 Hz, 2H), 6.85 - 6.78 (m, 1H), 3.82 (dd, J = 11.2, 3.1 Hz, 2H), 3.54 (s, 4H), 3.30 - 3.20 (m, 4H), 1.64 - 1.41 (m, 5H), 1.39 (s, 9H), 1.15 (qd, J = 12.4, 4.3 Hz, 2H). |
| | [M+H]⁺ = 492.1 |
| **40** | ¹H NMR (400 MHz, *d₆*-DMSO) δ 8.39 (t, J = 5.6 Hz, 1H), 8.12 (s, 2H), 8.08 - 8.02 (m, 2H), 7.91 - 7.83 (m, 2H), 7.62 (s, 1H), 6.71 (d, J = 5.1 Hz, 1H), 3.52 (s, 4H), 3.26 (q, J = 6.7 Hz, 2H), 2.27 (s, 3H), 1.95 (ddd, J = 13.8, 8.4, 5.5 Hz, 1H), 1.65 (dp, J = 13.3, 6.6 Hz, 1H), 1.42 (q, J = 7.0 Hz, 2H), 1.01 - 0.95 (m, 2H), 0.92 (d, J = 6.6 Hz, 6H), 0.64 - 0.56 (m, 2H). |
| | [M+H]⁺ = 449.2 |
| **41** | ¹H NMR (400 MHz, *d₆*-DMSO) δ 11.24 (s, 1H), 9.35 (s, 1H), 7.81 (d, J = 9.6 Hz, 1H), 7.75 (s, 2H), 7.16 (t, J = 8.2 Hz, 2H), 6.78 - 6.64 (m, 2H), 6.50 (d, J = 8.5 Hz, 1H), 4.06 (s, 2H), 3.91 (t, J = 6.5 Hz, 2H), 1.93 (d, J = 13.8 Hz, 1H), 1.67 (d, J = 14.8 Hz, 7H), 1.30 - 1.16 (m, 6H), 0.98 (d, J = 8.6 Hz, 2H), 0.86 (s, 2H), 0.61 (s, 2H). |
| | [M+H]⁺ = 475.1 |
| **42** | ¹H NMR (400 MHz, *d₆*-DMSO) δ 8.33 (d, J = 1.9 Hz, 1H), 8.15 (s, 2H), 8.06 (dd, J = 8.7, 1.7 Hz, 1H), 7.40 (s, 1H), 7.36 - 7.24 (m, 2H), 6.98 (dd, J = 7.0, 1.8 Hz, 1H), 6.94 (d, J = 8.7 Hz, 1H), 3.74 - 3.49 (m, 10H), 3.29 - 3.17 (m, 2H), 1.70 (ddd, J = 14.2, 8.4, 5.7 Hz, 1H), 0.75 (ddd, J = 17.3, 8.8, 4.7 Hz, 2H), 0.61 (td, J = 9.2, 8.8, 5.3 Hz, 1H), 0.31 -0.22 (m, 1H). |
| | [M+H]⁺ = 502.2 |
| **43** | ¹H NMR (400 MHz, *d₆*-DMSO) δ 8.67 (s, 1H), 8.13 (d, J = 7.8 Hz, 1H), 7.87 (d, J = 8.4 Hz, 1H), 7.82 (s, 2H), 7.55 (s, 1H), 7.31 (q, J = 7.7 Hz, 2H), 7.20 (d, J = 7.2 Hz, 1H), 7.12 (d, J = 8.4 Hz, 1H), 4.08 (dq, J = 13.4, 6.7 Hz, 1H), 3.55 - 3.46 (m, 2H), 3.44 - 3.35 (m, 2H), 2.71 (ddt, J = 10.9, 7.1, 3.5 Hz, 1H), 1.97 (ddd, J = 13.7, 8.4, 5.0 Hz, 1H), 1.15 (d, J = 6.6 Hz, 6H), 1.01 - 0.91 (m, 2H), 0.75 (d, J = 6.7 Hz, 4H), 0.61 (q, J = 5.7 Hz, 2H). |
| | [M+H]⁺ = 446.1 |
| **44** | ¹H NMR (500 MHz, *d₆*-DMSO) δ 8.92 (s, 1H), 8.37 (t, J = 5.6 Hz, 1H), 8.08 (s, 2H), 8.03 (d, J = 1.8 Hz, 1H), 7.87 (dd, J = 8.4, 1.8 Hz, 1H), 7.51 (s, 1H), 7.37 - 7.30 (m, 2H), 7.25 - 7.20 (m, 2H), 5.91 (s, 1H), 3.51 (s, 4H), 3.29 - 3.23 (m, 2H), 1.61 (dt, J = 13.6, 6.8 Hz, 1H), 1.57 (s, 6H), 1.41 (q, J = 7.0 Hz, 2H), 0.90 (d, J = 6.6 Hz, 6H). |
| | [M+H]⁺ = 452.3 |
| **45** | ¹H NMR (400 MHz, *d₆*-DMSO) δ 9.33 (s, 1H), 8.13 (s, 2H), 8.05 (s, 1H), 7.92 (d, J = 8.2 Hz, 1H), 7.42 (d, J = 7.4 Hz, 1H), 7.29 (dd, J = 8.1, 3.9 Hz, 2H), 6.85 (d, J = 7.7 Hz, 1H), 6.04 (s, 1H), 3.67 (s, 4H), 3.59 - 3.50 (m, 6H), 3.21 (d, J = 5.1 Hz, 2H), 1.56 (d, J = 10.1 Hz, 6H). |
| | ¹³C NMR (151 MHz, *d₆*-DMSO) δ 166.3, 140.3, 137.3, 135.5, 128.8, 128.6, 127.1, 118.9, 118.2, 117.9, 116.9, 72.8, 66.6, 66.4, 47.1, 41.9, 41.7, 29.9 |
| | [M+H]⁺ = 486.2 |
| **46** | ¹H NMR (400 MHz, *d₆*-DMSO) δ 9.70 (s, 1H), 7.81 (d, J = 8.4 Hz, 1H), 7.73 (d, J = 10.6 Hz, 2H), 7.16 (dd, J = 14.6, 8.2 Hz, 2H), 6.71 (d, J = 8.1 Hz, 1H), 6.67 (s, 1H), 6.48 (d, J = 8.4 Hz, 1H), 3.91 (t, J = 6.5 Hz, 2H), 3.87 - 3.78 (m, 2H), 3.59 (t, J = 8.5 Hz, 2H), 2.72 (s, 3H), 1.96 (t, J = 5.3 Hz, 1H), 1.68 (t, J = 14.8 Hz, 7H), 1.33 - 1.09 (m, 6H), 0.98 (q, J = 5.1, 4.5 Hz, 2H), 0.88 (q, J = 10.2, 9.2 Hz, 2H), 0.58 (q, J = 5.1 Hz, 2H). |
| | ¹³C NMR (151 MHz, *d₆*-DMSO) δ 176.3, 170.0, 160.0, 158.4, 146.5, 144.8, 131.1, 130.2, 129.6, 128.1, 127.8, 115.8, 111.5, 107.6, 105.5, 68.1, 42.5, 37.2, 33.7, 33.3, 26.6, 26.5, 26.3, 25.9, 11.4, 7.7 |
| | [M+H]⁺ = 503.1 |
| **47** | ¹H NMR (400 MHz, *d₆*-DMSO) δ 8.59 (s, 1H), 8.11 (s, 2H), 8.07 (s, 1H), 7.84 (dd, J = 8.3, 2.0 Hz, 1H), 7.71 (d, J = 1.9 Hz, 1H), 7.29 (t, J = 1.7 Hz, 1H), 7.18 (d, J = 8.4 Hz, 1H), 7.03 - 6.97 (m, 1H), 6.83 (dt, J = 11.2, 2.2 Hz, 1H), 3.52 (s, 4H), 2.00 - 1.88 (m, 1H), 1.34 (s, 3H), 0.99 - 0.89 (m, 2H), 0.75 - 0.68 (m, 2H), 0.63 - 0.54 (m, 4H). |
| | [M+H]⁺ = 436.2 |
| **48** | ¹H NMR (400 MHz, *d₆*-DMSO) δ 8.29 (t, J = 5.7 Hz, 1H), 8.21 (d, J = 1.8 Hz, 1H), 8.13 (s, 2H), 7.88 (dd, J = 8.2, 1.8 Hz, 1H), 7.23 - 7.15 (m, 3H), 7.14 - 7.07 (m, 2H), 6.81 (dd, J = 7.6, 1.8 Hz, 1H), 3.81 (t, J = 7.6 Hz, 1H), 3.71 (td, J = 8.2, 4.7 Hz, 1H), 3.61 (q, J = 7.7 Hz, 1H), 3.53 (s, 4H), 3.27 - 3.17 (m, 3H), 2.13 (dq, J = 14.7, 7.4 Hz, 1H), 2.06 - 1.96 (m, 1H), 1.56 (q, J = 6.9 Hz, 2H), 1.39 (s, 10H). |
| | [M+H]⁺ = 478.3 |
| **49** | ¹H NMR (400 MHz, *d₆*-DMSO) δ 11.33 (s, 1H), 9.43 (s, 1H), 8.22 (s, 1H), 8.05 (d, J = 8.0 Hz, 1H), 7.90 (d, J = 8.2 Hz, 1H), 7.31 (s, 1H), 7.20 (s, 3H), 7.15 (d, J = 8.2 Hz, 1H), 6.89 (s, 1H), 4.17 - 3.99 (m, 3H), 1.42 (s, 9H), 1.14 (d, J = 6.6 Hz, 6H). |
| | [M+H]⁺ = 436.1 |
| **50** | ¹H NMR (400 MHz, *d₆*-DMSO) δ 8.43 (d, J = 7.8 Hz, 1H), 8.12 (s, 2H), 7.85 (dd, J = 8.3, 1.9 Hz, 1H), 7.80 (d, J = 1.8 Hz, 1H), 7.34 (s, 1H), 7.28 - 7.21 (m, 1H), 7.10 (dd, J = 8.2, 1.5 Hz, 1H), 7.05 (d, J = 8.4 Hz, 1H), 6.90 (dd, J = 7.4, 1.5 Hz, 1H), 3.97 (d, J = 7.6 Hz, 1H), 3.90 - 3.81 (m, 2H), 3.52 (s, 4H), 3.44 - 3.35 (m, 2H), 1.93 - 1.83 (m, 1H), 1.79 (d, J = 12.5 Hz, 2H), 1.58 - 1.45 (m, 2H), 1.00 - 0.90 (m, 2H), 0.64 - 0.57 (m, 2H). |
| | [M+H]⁺ = 482.0 |
| **51** | ¹H NMR (400 MHz, *d₆*-DMSO) δ 8.11 (s, 2H), 8.03 (d, J = 1.8 Hz, 1H), 7.80 (dd, J = 8.4, 1.9 Hz, 1H), 7.51 (s, 1H), 7.15 (d, J = 8.4 Hz, 1H), 7.08 (t, J = 8.1 Hz, 1H), 6.55 (d, J = 8.1 Hz, 1H), 6.49 (d, J = 2.1 Hz, 1H), 6.37 (dd, J = 8.1, 2.1 Hz, 1H), 3.93 - 3.72 (m, 5H), 3.69 - 3.58 (m, 2H), 3.52 (s, 4H), 3.50 - 3.34 (m, 2H), 3.28 (t, J = 8.6 Hz, 1H), 2.01 (s, 2H), 1.79 (d, J = 8.2 Hz, 2H), 1.64 (dd, J = 7.1, 4.7 Hz, 2H), 1.48 (s, 2H). |
| | [M+H]⁺ = 465.1 |
| **52** | ¹H NMR (400 MHz, *d₆*-DMSO) δ 8.19 (d, J = 1.9 Hz, 1H), 8.12 (s, 2H), 7.87 (dd, J = 8.2, 1.9 Hz, 1H), 7.12 (d, J = 8.2 Hz, 1H), 7.06 - 6.96 (m, 2H), 6.33 (d, J = 8.2 Hz, 1H), 6.29 - 6.22 (m, 2H), 3.85 - 3.71 (m, 5H), 3.67 - 3.57 (m, 2H), 3.53 (s, 4H), 3.49 - 3.43 (m, 1H), 3.39 - 3.36 (m, 1H), 1.38 (s, 9H). |
| | [M+H]⁺ = 453.1 |
| **53** | ¹H NMR (400 MHz, *d₆*-DMSO) δ 9.11 - 9.01 (m, 1 H), 8.13 - 8.05 (m, 2 H), 7.87 - 7.77 (m, 2 H), 7.74 - 7.70 (m, 1 H), 7.56 - 7.49 (m, 1 H), 7.36 - 7.27 (m, 2 H), 7.26 - 7.19 (m, 1 H), 7.15 -7.06 (m, 1 H), 3.51 (s, 4 H), 1.99 - 1.87 (m, 1 H), 1.34 - 1.25 (m, 2 H), 1.16 - 1.08 (m, 2 H), 1.00 - 0.89 (m, 2 H), 0.62 - 0.52 (m, 2 H). |
| | [M+H]⁺ = 472.1 |
| **54** | ¹H NMR (400 MHz, *d₆*-DMSO) δ 9.66 (s, 1H), 8.33 (d, J = 8.0 Hz, 1H), 8.19 (s, 1H), 8.12 (s, 2H), 7.87 (d, J = 6.3 Hz, 1H), 7.66 (d, J = 6.6 Hz, 1H), 7.23 (d, J = 8.3 Hz, 2H), 6.92 (d, J = 8.3 Hz, 1H), 6.75 (t, J = 7.3 Hz, 1H), 4.18 - 4.05 (m, 1H), 3.54 (s, 4H), 1.41 (s, 9H), 1.18 (d, J = 6.6 Hz, 6H). ¹³C NMR (151 MHz, *d₆*-DMSO) δ 168.6, 146.0, 142.4, 141.9, 132.2, 129.2, 128.2, 127.7, 124.1, 118.6, 117.5, 114.7, 41.8, 41.2, 34.9, 30.4, 22.7 |
| | [M+H]⁺ = 422.0 |
| **55** | ¹H NMR (400 MHz, *d₆*-DMSO) δ 8.46 (s, 1H), 8.22 (s, 1H), 8.15 (s, 2H), 8.08 (d, J = 5.2 Hz, 1H), 8.03 (s, 1H), 7.90 (d, J = 8.1 Hz, 1H), 7.17 (d, J = 7.9 Hz, 1H), 6.95 (d, J = 5.8 Hz, 1H), 6.90 (s, 1H), 3.54 (s, 4H), 3.23 (d, J = 8.1 Hz, 2H), 1.58 (d, J = 6.7 Hz, 1H), 1.37 (s, 11H), 0.89 (d, J = 6.6 Hz, 6H). |
| | [M+H]⁺ = 451.1 |
| **56** | ¹H NMR (400 MHz, *d₆*-DMSO) δ 9.64 (s, 1H), 8.57 (s, 1H), 8.17 (s, 3H), 7.84 (s, 1H), 7.67 (d, J = 7.8 Hz, 1H), 7.26 (s, 2H), 6.78 (s, 2H), 3.56 (s, 4H), 3.12 - 3.07 (m, 2H), 1.90 - 1.81 (m, 1H), 1.41 (s, 9H), 0.91 (d, J = 6.7 Hz, 6H). |
| | [M+H]⁺ = 436.0 |
| **57** | [M+H]⁺ = 435.3 |
| **58** | ¹H NMR (500 MHz, *d₆*-DMSO) δ 8.32 (t, J = 5.6 Hz, 1H), 8.08 (s, 2H), 7.78 (s, 2H), 7.72 (d, J = 1.9 Hz, 1H), 7.53 (s, 1H), 7.30 (d, J = 5.0 Hz, 2H), 7.22 - 7.15 (m, 1H), 7.11 (d, J = 8.4 Hz, 1H), 3.82 (dd, J = 11.4, 2.8 Hz, 2H), 3.51 (s, 4H), 3.30 - 3.21 (m, 4H), 1.94 (ddd, J = 13.7, 8.4, 5.4 Hz, 1H), 1.60 (d, J = 13.0 Hz, 2H), 1.45 (q, J = 6.9 Hz, 3H), 1.15 (qd, J = 12.0, 4.4 Hz, 2H), 0.98 - 0.90 (m, 2H), 0.61 - 0.54 (m, 2H). |
| | [M+H]⁺ = 476.1 |
| **59** | ¹H NMR (400 MHz, *d₆*-DMSO) δ 8.11 (d, J = 11.1 Hz, 3H), 8.05 (s, 1H), 7.84 (d, J = 9.8 Hz, 1H), 7.81 (s, 1H), 7.43 (s, 1H), 7.28 (s, 2H), 7.14 (d, J = 8.4 Hz, 1H), 7.07 (s, 1H), 4.11 - 3.99 (m, 2H), 3.96 (d, J = 4.6 Hz, 1H), 3.80 - 3.65 (m, 2H), 3.59 - 3.54 (m, 1H), 3.53 (s, 4H), 2.33 (s, 1H), 1.89 (d, J = 7.4 Hz, 1H), 1.15 (d, J = 6.6 Hz, 6H). |
| | [M+H]⁺ = 436.1 |
| **60** | [M+H]⁺ = 457.3 |
| **61** | ¹H NMR (400 MHz, *d₆*-DMSO) δ 9.45 (s, 1H), 8.16 (d, J = 7.8 Hz, 1H), 7.92 (s, 2H), 7.81 (s, 1H), 7.62 (s, 1H), 7.39 (d, J = 7.8 Hz, 1H), 7.34 (t, J = 7.8 Hz, 1H), 7.27 (d, J = 8.1 Hz, 1H), 7.15 (d, J = 8.5 Hz, 1H), 4.12 (s, 2H), 4.11 -4.04 (m, 1H), 3.06 (s, 3H), 1.98 (s, 1H), 1.16 (d, J = 6.6 Hz, 6H), 1.02 - 0.94 (m, 2H), 0.62 (q, J = 5.6 Hz, 2H). |
| | [M+H]⁺ = 434.0 |
| **62** | ¹H NMR (400 MHz, *d₆*-DMSO) δ 9.91 (s, 1H), 8.52 (s, 1H), 8.10 (s, 2H), 7.83 (d, J = 10.4 Hz, 2H), 7.63 (d, J = 7.5 Hz, 1H), 7.41 - 7.32 (m, 2H), 7.29 (d, J = 8.3 Hz, 1H), 6.93 - 6.86 (m, 1H), 3.52 (s, 4H), 3.28 (d, J = 6.7 Hz, 2H), 1.81 (s, 1H), 1.67 - 1.59 (m, 1H), 1.43 (q, J = 7.0 Hz, 2H), 1.01 (d, J = 8.3 Hz, 2H), 0.90 (d, J = 6.6 Hz, 6H), 0.57 (d, J = 3.7 Hz, 2H). |
| | [M+H]⁺ = 434.0 |
| **63** | ¹H NMR (400 MHz, *d₆*-DMSO) δ 9.66 (s, 1H), 8.13 (s, 2H), 8.03 (s, 1H), 7.84 (d, J = 9.8 Hz, 1H), 7.66 (s, 1H), 7.33 (s, 1H), 7.15 (d, J = 8.4 Hz, 1H), 7.10 (t, J = 8.1 Hz, 1H), 7.03 (d, J = 8.0 Hz, 1H), 6.62 (d, J = 7.2 Hz, 1H), 4.02 (s, 1H), 3.94 (dd, J = 8.4, 4.5 Hz, 1H), 3.76 (q, J = 7.8 Hz, 1H), 3.72 - 3.65 (m, 1H), 3.58 - 3.54 (m, 1H), 3.52 (s, 4H), 2.59 - 2.52 (m, 1H), 2.31 (dd, J = 11.8, 4.5 Hz, 1H), 1.86 (dd, J = 12.2, 7.9 Hz, 1H), 1.07 (d, J = 6.8 Hz, 6H). |
| | [M+H]⁺ = 436.0 |
| **64** | ¹H NMR (300 MHz, *d₆*-DMSO) δ 9.22 (s, 1H), 8.12 (s, 2H), 7.77 - 7.71 (m, 2H), 7.27 - 7.15 (m, 3H), 7.05 (d, *J* = 7.0 Hz, 1H), 6.56 (d, *J* = 9.0 Hz, 1H), 3.52 (s, 4H), 2.17 (s, 3H), 1.94 - 1.82 (m, 1H), 1.56 (dd, *J* = 8.2, 5.4 Hz, 2H), 1.28 (dd, *J* = 8.2, 5.4 Hz, 2H), 1.01 - 0.91 (q, *J* = 5.7 Hz, 2H), 0.59 (q, *J* = 5.7 Hz, 2H). |
| | [M+H]⁺ = 443.3 |
| **65** | ¹H NMR (400 MHz, *d₆*-DMSO) δ 8.40 (d, J = 7.8 Hz, 1H), 8.31 (s, 1H), 8.13 (s, 2H), 7.86 (dd, J = 8.3, 2.0 Hz, 1H), 7.77 - 7.66 (m, 2H), 7.58 (s, 1H), 7.32 (t, J = 1.9 Hz, 1H), 7.20 (d, J = 8.4 Hz, 1H), 4.10 (dq, J = 13.5, 6.7 Hz, 1H), 3.54 (s, 4H), 1.93 (m, 1H), 1.18 (d, J = 6.6 Hz, 6H), 1.00 - 0.88 (m, 2H), 0.66 - 0.58 (m, 2H). |
| | [M+H]⁺ = 474.2 |
| **66** | ¹H NMR (400 MHz, *d₆*-DMSO) δ 8.13 - 8.03 (m, 3H), 7.82 - 7.68 (m, 3H), 7.26 - 7.21 (m, 1H), 7.20 - 7.11 (m, 2H), 7.05 (d, J = 8.4 Hz, 1H), 4.09 - 3.99 (m, 1H), 3.51 (s, 4H), 1.96 - 1.87 (m, 1H), 1.14 (d, J = 6.6 Hz, 6H), 0.99 - 0.91 (m, 2H), 0.62 - 0.54 (m, 2H). |
| | [M+H]⁺ = 424.1 |
| **67** | ¹H NMR (400 MHz, *d₆*-DMSO) δ 8.79 (s, 1H), 8.08 (s, 2H), 8.02 (d, J = 1.9 Hz, 1H), 7.87 (dd, J = 8.5, 1.8 Hz, 1H), 7.24 (d, J = 8.4 Hz, 1H), 7.15 (t, J = 8.1 Hz, 1H), 6.63 (d, J = 8.1 Hz, 1H), 6.59 (d, J = 2.2 Hz, 1H), 6.47 (dd, J = 8.2, 2.0 Hz, 1H), 5.86 (s, 1H), 3.92 (d, J = 6.5 Hz, 2H), 3.83 (dd, J = 10.8, 3.0 Hz, 2H), 3.52 (s, 4H), 3.30 - 3.22 (m, 2H), 1.71 (dt, J = 14.5, 6.6 Hz, 2H), 1.60 (s, 1H), 1.56 (s, 7H), 1.50 (s, 1H), 1.37 - 1.31 (m, 2H), 1.20 - 1.07 (m, 2H). |
| | [M+H]⁺ = 481.1 |
| **68** | ¹H NMR (300 MHz, CDCl₃) δ 8.01 (s, 1H), 7.93 (d, *J* = 9.5 Hz, 1H), 7.67 (s, 1H), 7.53 (d, *J* = 8.4 Hz, 1H), 7.23 (d, *J* = 8.0 Hz, 1H), 7.18 (d, *J* = 7.5 Hz, 1H), 6.67 (d, *J* = 8.5 Hz, 1H), 6.31 (s, 1H), 5.00 (d, *J* = 6.0 Hz, 2H), 4.61 (d, *J* = 6.1 Hz, 2H), 3.72 (s, 4H), 2.14 (s, 3H), 1.82 - 1.73 (m, 1H), 1.71 (s, 3H), 1.04 - 0.95 (m, 2H), 0.81 - 0.74 (m, 2H). |
| | [M+H]⁺ = 448.3 |
| **69** | ¹H NMR (400 MHz, *d₆*-DMSO) δ 8.79 (s, 1H), 8.08 (s, 2H), 8.02 (d, J = 1.9 Hz, 1H), 7.87 (dd, J = 8.5, 1.8 Hz, 1H), 7.24 (d, J = 8.4 Hz, 1H), 7.15 (t, J = 8.1 Hz, 1H), 6.63 (d, J = 8.1 Hz, 1H), 6.59 (d, J = 2.2 Hz, 1H), 6.47 (dd, J = 8.2, 2.0 Hz, 1H), 5.86 (s, 1H), 3.92 (d, J = 6.5 Hz, 2H), 3.83 (dd, J = 10.8, 3.0 Hz, 2H), 3.52 (s, 4H), 3.30 - 3.22 (m, 2H), 1.71 (dt, J = 14.5, 6.6 Hz, 2H), 1.60 (s, 1H), 1.56 (s, 7H), 1.50 (s, 1H), 1.37 - 1.31 (m, 2H), 1.20 - 1.07 (m, 2H). |
| | [M+H]⁺ = 450.0 |
| **70** | ¹H NMR (400 MHz, *d₆*-DMSO) δ 9.89 (s, 1H), 8.35 (d, J = 7.7 Hz, 1H), 8.10 (s, 2H), 7.84 (d, J = 8.7 Hz, 2H), 7.64 (d, J = 7.4 Hz, 1H), 7.42 - 7.33 (m, 2H), 7.29 (d, J = 8.2 Hz, 1H), 6.93 - 6.86 (m, 1H), 4.14 - 4.04 (m, 1H), 3.52 (s, 4H), 1.84 - 1.76 (m, 1H), 1.17 (d, J = 6.6 Hz, 6H), 1.02 (dd, J = 8.3, 1.9 Hz, 2H), 0.57 (d, J = 3.8 Hz, 2H). |
| | [M+H]⁺ = 406.0 |
| **71** | ¹H NMR (400 MHz, *d₆*-DMSO) δ 8.17 (d, J = 2.0 Hz, 1H), 8.03 (d, J = 7.8 Hz, 1H), 7.84 (dd, J = 8.1, 1.9 Hz, 1H), 7.25 - 7.06 (m, 5H), 6.84 - 6.74 (m, 1H), 4.14 - 3.99 (m, 1H), 3.58 (s, 4H), 2.81 (s, 6H), 1.40 (s, 9H), 1.16 (d, J = 6.6 Hz, 6H). |
| | [M+H]⁺ = 450.3 |
| **72** | ¹H NMR (400 MHz, *d₆*-DMSO) δ 9.76 (s, 1H), 8.11 (s, 2H), 8.07 (s, 1H), 7.87 (s, 1H), 7.84 (d, J = 8.9 Hz, 1H), 7.54 (s, 1H), 7.21 (d, J = 4.4 Hz, 3H), 6.84 (s, 1H), 3.53 (s, 4H), 2.62 - 2.53 (m, 1H), 1.08 (d, J = 6.8 Hz, 6H). |
| | [M+H]⁺ = 400.0 |
| **73** | ¹H NMR (400 MHz, *d₆*-DMSO) δ 8.18 - 8.06 (m, 3H), 7.84 (dd, J = 8.3, 2.0 Hz, 1H), 7.70 (d, J = 1.9 Hz, 1H), 7.20 (d, J = 8.3 Hz, 1H), 6.83 - 6.72 (m, 2H), 6.65 - 6.53 (m, 1H), 3.70 - 3.33 (m, 12H), 2.01 - 1.86 (m, 1H), 1.02 - 0.91 (m, 2H), 0.66 - 0.55 (m, 2H). |
| | [M+H]⁺ = 452.2 |
| **74** | ¹H NMR (400 MHz, *d₆*-DMSO) δ 8.48 (t, J = 5.7 Hz, 1H), 8.22 (d, J = 1.9 Hz, 1H), 8.15 (s, 2H), 8.08 (d, J = 5.2 Hz, 1H), 8.04 (s, 1H), 7.90 (dd, J = 8.1, 1.8 Hz, 1H), 7.17 (d, J = 8.1 Hz, 1H), 6.95 (d, J = 5.3 Hz, 1H), 6.89 (s, 1H), 3.82 (dd, J = 11.1, 3.0 Hz, 2H), 3.54 (s, 4H), 3.29 - 3.20 (m, 4H), 1.59 (d, J = 13.2 Hz, 2H), 1.54 - 1.40 (m, 3H), 1.37 (s, 8H), 1.15 (qd, J = 12.4, 4.3 Hz, 2H). |
| | [M+H]⁺ = 493.1 |
| **75** | ¹H NMR (400 MHz, *d₆*-DMSO) δ 8.10 (s, 2H), 7.86 (s, 1H), 7.79 (d, J = 8.4 Hz, 1H), 7.70 (s, 1H), 7.31 (t, J = 7.9 Hz, 1H), 7.16 (d, J = 8.4 Hz, 1H), 7.05 (d, J = 8.5 Hz, 1H), 6.90 (s, 1H), 6.76 (d, J = 8.1 Hz, 1H), 3.52 (s, 4H), 3.12 (s, 3H), 2.58 (s, 1H), 2.02 - 1.90 (m, 1H), 0.94 (d, J = 6.6 Hz, 8H), 0.58 (q, J = 5.0, 4.5 Hz, 2H). |
| | [M+H]⁺ = 420.0 |
| **76** | ¹H NMR (400 MHz, *d₆*-DMSO) δ 8.08 (s, 2H), 7.89 (d, J = 7.5 Hz, 1H), 7.76 (dd, J = 1.9, 8.4 Hz, 1H), 7.70 (d, J = 1.8 Hz, 1H), 7.59 (s, 1H), 7.21 - 7.13 (m, 1H), 7.09 (d, J = 8.5 Hz, 1H), 7.04 - 6.99 (m, 1H), 6.97 - 6.90 (m, 1H), 6.76 (d, J = 7.4 Hz, 1H), 3.86 - 3.74 (m, 1H), 3.50 (s, 4H), 3.28 (s, 2H), 2.00 - 1.87 (m, 1H), 1.04 (d, J = 6.6 Hz, 6H), 0.97 - 0.90 (m, 2H), 0.60 - 0.51 (m, 2H). |
| | [M+H]⁺ = 420.2 |
| **77** | ¹H NMR (400 MHz, *d₆*-DMSO) δ 8.19 (d, J = 7.7 Hz, 1H), 8.15 - 8.07 (m, 3H), 7.83 (dd, J = 2.0, 8.4 Hz, 1H), 7.71 (d, J = 2.0 Hz, 1H), 7.33 (t, J = 1.7 Hz, 1H), 7.19 (d, J = 8.4 Hz, 1H), 7.09 - 7.01 (m, 1H), 6.84 (td, J = 2.2, 11.3 Hz, 1H), 4.12 - 3.96 (m, 1H), 3.52 (s, 4H), 2.00 - 1.89 (m, 1H), 1.15 (d, J = 6.6 Hz, 6H), 1.00 - 0.89 (m, 2H), 0.62 - 0.56 (m, 2H). |
| | [M+H]⁺ = 424.1 |
| **78** | ¹H NMR (400 MHz, *d₆*-DMSO) δ 9.11 (s, 1H), 8.09 (s, 2H), 8.05 (d, J = 1.9 Hz, 1H), 7.87 (dd, J = 8.5, 1.9 Hz, 1H), 7.30 (dd, J = 8.2, 1.1 Hz, 1H), 7.25 (d, J = 8.5 Hz, 1H), 7.17 (t, J = 7.8 Hz, 1H), 6.67 (dd, J = 7.5, 1.1 Hz, 1H), 5.82 (s, 1H), 3.76 - 3.48 (m, 10H), 3.24 (t, J = 5.5 Hz, 2H), 1.59 (d, J = 9.9 Hz, 7H), 1.00 - 0.88 (m, 2H), 0.69 - 0.61 (m, 1H), 0.36 - 0.29 (m, 1H). |
| | [M+H]⁺ = 492.3 |
| **79** | ¹H NMR (400 MHz, *d₆*-DMSO) δ 8.29 (t, J = 5.7 Hz, 1H), 8.13 (d, J = 2.3 Hz, 3H), 7.92 (dd, J = 10.4, 1.4 Hz, 2H), 7.73 (t, J = 1.7 Hz, 1H), 7.66 (s, 1H), 7.44 (s, 1H), 7.29 - 7.22 (m, 3H), 7.11 - 7.03 (m, 1H), 6.73 (d, J = 1.1 Hz, 1H), 3.82 (dd, J = 10.9, 3.5 Hz, 2H), 3.53 (s, 4H), 3.29 - 3.20 (m, 4H), 1.60 (d, J = 13.5 Hz, 2H), 1.53 (s, 1H), 1.44 (q, J = 6.8 Hz, 2H), 1.15 (qd, J = 12.4, 4.5 Hz, 2H). |
| | [M+H]⁺ = 502.2 |
| **80** | ¹H NMR (400 MHz, *d₆*-DMSO) δ 9.86 (s, 1H), 8.58 (t, J = 5.8 Hz, 1H), 8.11 (s, 2H), 7.83 (dd, J = 10.8, 2.4 Hz, 2H), 7.68 - 7.63 (m, 1H), 7.38 (d, J = 6.6 Hz, 2H), 7.30 (d, J = 8.3 Hz, 1H), 6.91 (t, J = 6.3 Hz, 1H), 3.53 (s, 4H), 3.12 - 3.06 (m, 2H), 1.90 - 1.83 (m, 1H), 1.83 - 1.77 (m, 1H), 1.05 - 0.98 (m, 2H), 0.90 (d, J = 6.7 Hz, 6H), 0.56 (d, J = 3.8 Hz, 2H). |
| | [M+H]⁺ = 420.0 |
| **81** | ¹H NMR (400 MHz, *d₆*-DMSO) δ 11.17 (s, 1H), 8.16 (d, J = 7.8 Hz, 1H), 7.91 (d, J = 8.7 Hz, 2H), 7.77 (s, 1H), 7.62 (s, 1H), 7.43 - 7.30 (m, 2H), 7.27 (d, J = 7.7 Hz, 1H), 7.14 (d, J = 8.4 Hz, 1H), 4.16 - 4.01 (m, 3H), 3.09 (s, 3H), 1.97 (ddd, J = 13.7, 8.3, 5.4 Hz, 1H), 1.16 (d, J = 6.6 Hz, 6H), 1.03 - 0.94 (m, 2H), 0.61 (q, J = 5.3 Hz, 2H). |
| | [M+H]⁺ = 434.3 |
| **82** | ¹H NMR (400 MHz, *d₆*-DMSO) δ 8.09 (s, 2H), 7.78 (dd, J = 8.4, 2.0 Hz, 1H), 7.73 (d, J = 2.0 Hz, 1H), 7.66 (s, 1H), 7.30 (t, J = 7.8 Hz, 1H), 7.23 (s, 1H), 7.11 (d, J = 8.4 Hz, 1H), 7.04 (td, J = 7.6, 1.9 Hz, 2H), 4.76 (d, J = 5.9 Hz, 2H), 4.66 (d, J = 5.9 Hz, 2H), 3.52 (s, 4H), 2.83 - 2.65 (m, 1H), 2.44 (t, J = 6.2 Hz, 1H), 1.98 (m, 1H), 1.02 - 0.93 (m, 2H), 0.86 (d, J = 6.3 Hz, 6H), 0.65 - 0.55 (m, 2H). |
| | [M+H]⁺ = 434.2 |
| **83** | ¹H NMR (400 MHz, *d₆*-DMSO) δ 8.26 - 8.17 (m, 1H), 8.08 (s, 2H), 7.76 (dd, J = 1.9, 8.5 Hz, 1H), 7.71 (d, J = 1.7 Hz, 1H), 7.62 (s, 1H), 7.19 (t, J = 7.8 Hz, 1H), 7.10 (d, J = 8.4 Hz, 1H), 6.99 - 6.93 (m, 2H), 6.78 - 6.72 (m, 1H), 4.20 (d, J = 5.8 Hz, 2H), 3.51 (s, 4H), 2.45 - 2.37 (m, 1H), 2.00 - 1.89 (m, 1H), 1.02 (d, J = 6.8 Hz, 6H), 0.98 - 0.92 (m, 2H), 0.60 - 0.52 (m, 2H). |
| | [M+H]⁺ = 420.2 |
| **84** | ¹H NMR (400 MHz, *d₆*-DMSO) δ 9.02 (s, 1H), 8.10 (s, 3H), 7.92 (d, J = 8.5 Hz, 1H), 7.50 (d, J = 12.5 Hz, 2H), 7.38 (d, J = 8.0 Hz, 1H), 7.33 (d, J = 7.0 Hz, 1H), 7.28 (d, J = 8.2 Hz, 1H), 5.93 (s, 1H), 3.78 (d, J = 11.6 Hz, 2H), 3.53 (s, 4H), 3.29 - 3.19 (m, 4H), 1.56 (s, 9H), 1.46 (d, J = 12.2 Hz, 2H), 1.24 (s, 2H), 1.06 (d, J = 10.5 Hz, 2H). |
| | [M+H]⁺ = 529.0 |
| **85** | ¹H NMR (400 MHz, *d₆*-DMSO) δ 8.79 (s, 1H), 8.08 (s, 2H), 8.02 (d, J = 1.9 Hz, 1H), 7.87 (dd, J = 8.4, 1.9 Hz, 1H), 7.25 (d, J = 8.5 Hz, 1H), 7.15 (t, J = 8.1 Hz, 1H), 6.67 - 6.58 (m, 2H), 6.48 (dd, J = 8.1, 2.1 Hz, 1H), 5.85 (s, 1H), 3.98 (t, J = 6.3 Hz, 2H), 3.83 (dd, J = 11.1, 3.0 Hz, 2H), 3.52 (s, 4H), 3.30 - 3.23 (m, 2H), 1.69 (s, 1H), 1.67 - 1.62 (m, 3H), 1.60 (s, 1H), 1.56 (s, 6H), 1.26 - 1.16 (m, 2H). |
| | [M+H]⁺ = 467.1 |
| **86** | ¹H NMR (400 MHz, *d₆*-DMSO) δ 8.81 (s, 1H), 8.29 (s, 1H), 8.13 (s, 2H), 7.86 (dd, J = 8.3, 2.0 Hz, 1H), 7.73 (d, J = 2.0 Hz, 1H), 7.68 (t, J = 1.8 Hz, 1H), 7.54 (s, 1H), 7.31 (t, J = 2.0 Hz, 1H), 7.19 (d, J = 8.3 Hz, 1H), 3.54 (s, 4H), 1.99 - 1.86 (m, 1H), 1.37 (s, 3H), 0.99 - 0.88 (m, 2H), 0.74 (q, J = 4.6, 4.1 Hz, 2H), 0.62 (m, 4H). |
| | [M+H]⁺ = 486.2 |
| **87** | ¹H NMR (400 MHz, *d₆*-DMSO) δ 8.15 (t, J = 5.5 Hz, 3H), 7.82 (d, J = 7.8 Hz, 1H), 7.68 (s, 1H), 7.65 (dd, J = 7.6, 1.7 Hz, 1H), 7.43 (t, J = 7.1 Hz, 1H), 7.21 (t, J = 7.3 Hz, 1H), 6.84 (d, J = 7.1 Hz, 1H), 6.81 (d, J = 8.5 Hz, 1H), 3.55 (br. s, 4H), 3.22 (q, J = 6.7 Hz, 2H), 2.15 (br. s, 1H), 1.52 (dp, J = 13.3, 6.7 Hz, 1H), 1.31 (q, J = 7.0 Hz, 2H), 0.97 - 0.86 (m, 2H), 0.82 (d, J = 6.6 Hz, 6H), 0.72 - 0.61 (m, 2H). |
| | [M+H]⁺ = 435.2 |
| **88** | ¹H NMR (400 MHz, *d₆*-DMSO) δ 8.81 (s, 1H), 8.10 (s, 2H), 8.02 (d, J = 1.9 Hz, 1H), 7.87 (dd, J = 8.5, 1.9 Hz, 1H), 7.25 (d, J = 8.5 Hz, 1H), 7.16 (t, J = 8.1 Hz, 1H), 6.65 (dd, J = 7.9, 1.6 Hz, 1H), 6.61 (s, 1H), 6.48 (dd, J = 8.1, 2.0 Hz, 1H), 5.86 (s, 1H), 4.00 (t, J = 6.4 Hz, 2H), 3.72 (td, J = 11.7, 2.5 Hz, 2H), 3.68 - 3.61 (m, 2H), 3.60 - 3.55 (m, 1H), 3.52 (s, 4H), 3.45 (td, J = 11.0, 2.6 Hz, 1H), 3.22 (dd, J = 11.3, 9.9 Hz, 1H), 1.75 (pt, J = 13.9, 6.4 Hz, 2H), 1.56 (s, 6H). |
| | [M+H]⁺ = 469.1 |
| **89** | ¹H NMR (300 MHz, CDCl₃) δ 8.00 (s, 1H), 7.93 (dd, *J* = 8.6, 1.8 Hz, 1H), 7.16 (d, *J* = 5.3 Hz, 2H), 7.13 - 7.07 (m, 1H), 6.86 (d, *J* = 8.5 Hz, 1H), 6.29 (s, 1H), 4.31 - 4.18 (m, 4H), 3.71 (s, 4H), 2.22 (s, 3H), 1.81 - 1.72 (m, 1H), 1.38 (td, *J* = 7.1, 0.9 Hz, 7H), 1.04 - 0.94 (m, 2H), 0.81 - 0.74 (m, 2H). |
| | [M+H]⁺ = 487.3 |
| **90** | ¹H NMR (300 MHz, CDCl₃) δ 7.96 (d, *J* = 8.4 Hz, 3H), 7.78 (d, *J* = 7.7 Hz, 1H), 7.43 (t, *J* = 7.9 Hz, 1H), 7.33 - 7.26 (m, 2H), 6.50 (s, 1H), 5.55 (t, *J* = 7.2 Hz, 1H), 5.23 (d, *J* = 7.2 Hz, 2H), 3.65 (s, 4H), 1.86 (s, 3H), 1.81 (s, 3H), 1.81 - 1.74 (m, 1H), 1.00 (q, *J* = 5.5 Hz, 2H), 0.77 (q, *J* = 5.5 Hz, 2H). |
| | [M+H]⁺ = 457.3 |
| **91** | ¹H NMR (400 MHz, *d₆*-DMSO) δ 8.15 (s, 2H), 8.05 (d, J = 7.8 Hz, 1H), 7.87 (s, 1H), 7.62 (dd, J = 11.4, 1.8 Hz, 1H), 7.49 (d, J = 1.8 Hz, 1H), 7.22 - 7.12 (m, 3H), 6.73 (d, J = 7.1 Hz, 1H), 4.11 - 4.00 (m, 1H), 3.54 (s, 4H), 2.03 - 1.93 (m, 1H), 1.14 (d, J = 6.6 Hz, 6H), 0.95 - 0.88 (m, 2H), 0.65 - 0.58 (m, 2H). |
| | [M+H]⁺ = 424.2 |
| **92** | ¹H NMR (400 MHz, *d₆*-DMSO) δ 8.06 (s, 2H), 7.85 (d, J = 8.0 Hz, 1H), 7.82 - 7.75 (m, 2H), 7.73 (t, J = 1.7 Hz, 1H), 7.67 (dd, J = 1.6, 0.8 Hz, 1H), 7.42 - 7.30 (m, 2H), 7.03 (d, J = 8.3 Hz, 1H), 6.98 (dd, J = 7.3, 1.4 Hz, 1H), 6.75 (s, 1H), 6.52 (dd, J = 1.8, 0.8 Hz, 1H), 3.91 - 3.78 (m, 1H), 3.50 (s, 4H), 1.67 - 1.58 (m, 1H), 0.94 (d, J = 6.6 Hz, 6H), 0.80 - 0.70 (m, 2H), 0.42 - 0.32 (m, 2H). |
| | [M+H]⁺ = 472.2 |
| **93** | ¹H NMR (400 MHz, *d₆*-DMSO) δ 9.36 (s, 1H), 8.18 (d, J = 2.1 Hz, 1H), 8.13 (s, 2H), 7.88 (dd, J = 8.4, 2.0 Hz, 1H), 7.80 (s, 1H), 7.14 - 7.04 (m, 2H), 6.77 (d, J = 9.6 Hz, 1H), 6.72 (d, J = 8.4 Hz, 1H), 3.53 (s, 4H), 2.32 (t, J = 7.5 Hz, 2H), 1.50 (dd, J = 12.9, 6.5 Hz, 1H), 1.47 - 1.42 (m, 2H), 1.41 (s, 9H), 0.84 (d, J = 6.4 Hz, 6H). |
| | [M+H]⁺ = 451.1 |
| **94** | ¹H NMR (400 MHz, *d₆*-DMSO) δ 8.59 (d, J = 7.8 Hz, 1H), 8.11 (s, 2H), 7.85 (dd, J = 8.3, 1.9 Hz, 1H), 7.79 (d, J = 1.8 Hz, 1H), 7.35 (s, 1H), 7.30 - 7.21 (m, 1H), 7.10 (dd, J = 8.2, 1.5 Hz, 1H), 7.05 (d, J = 8.3 Hz, 1H), 6.92 (dd, J = 7.4, 1.5 Hz, 1H), 4.27 - 4.04 (m, 1H), 3.52 (s, 4H), 3.30 - 3.22 (m, 2H), 3.14 (d, J = 14.5 Hz, 2H), 2.16 (s, 2H), 2.03 (d, J = 10.3 Hz, 2H), 1.88 (ddd, J = 13.7, 8.2, 5.4 Hz, 1H), 0.99 - 0.90 (m, 2H), 0.65 - 0.56 (m, 2H). |
| | [M+H]⁺ = 529.9 |
| **95** | ¹H NMR (400 MHz, *d₆*-DMSO) δ 8.41 (d, J = 6.5 Hz, 1H), 8.10 (s, 2H), 8.05 (d, J = 1.9 Hz, 1H), 7.80 (dd, J = 8.4, 1.9 Hz, 1H), 7.68 (s, 1H), 7.47 - 7.42 (m, 1H), 7.31 - 7.21 (m, 2H), 7.12 (d, J = 8.4 Hz, 1H), 7.10 - 7.04 (m, 1H), 4.41 (dd, J = 11.7, 7.1 Hz, 1H), 3.89 - 3.78 (m, 2H), 3.70 (td, J = 8.0, 5.9 Hz, 1H), 3.56 (dd, J = 8.8, 4.5 Hz, 1H), 3.52 (s, 4H), 3.30 - 3.22 (m, 1H), 2.18 - 2.08 (m, 1H), 2.02 (s, 2H), 1.96 - 1.85 (m, 1H), 1.79 (d, J = 8.1 Hz, 2H), 1.63 (dd, J = 7.1, 4.6 Hz, 2H), 1.55 - 1.43 (m, 2H). |
| | [M+H]⁺ = 462.2 |
| **96** | ¹H NMR (400 MHz, *d₆*-DMSO) δ 8.20 (d, J = 7.7 Hz, 1H), 8.10 (s, 2H), 8.05 (d, J = 1.9 Hz, 1H), 7.80 (dd, J = 8.3, 1.9 Hz, 1H), 7.69 (s, 1H), 7.44 (s, 1H), 7.26 (d, J = 5.0 Hz, 2H), 7.12 (d, J = 8.4 Hz, 1H), 7.07 (d, J = 2.4 Hz, 1H), 3.97 (dd, J = 11.4, 3.8 Hz, 1H), 3.87 (d, J = 11.7 Hz, 2H), 3.52 (s, 4H), 3.43 - 3.33 (m, 2H), 3.31 - 3.22 (m, 1H), 2.02 (s, 2H), 1.83 - 1.69 (m, 4H), 1.67 - 1.41 (m, 6H). |
| | [M+H]⁺ = 476.0 |
| **97** | ¹H NMR (400 MHz, *d₆*-DMSO) δ 8.13 (s, 2H), 8.07 (dd, J = 6.0, 1.9 Hz, 1H), 7.95 (dd, J = 8.4, 1.9 Hz, 1H), 7.88 (d, J = 26.3 Hz, 1H), 7.28 (td, J = 7.9, 2.4 Hz, 1H), 7.24 - 7.14 (m, 2H), 6.85 (dd, J = 7.3, 1.5 Hz, 1H), 4.94 - 4.83 (m, 1H), 4.09 (td, J = 12.8, 12.1, 5.8 Hz, 1H), 3.82 (q, J = 7.9 Hz, 1H), 3.66 (s, 4H), 3.55 (d, J = 11.9 Hz, 6H), 3.24 - 3.14 (m, 2H), 2.24 (td, J = 11.9, 7.5 Hz, 1H), 1.97 (tt, J = 14.7, 7.7 Hz, 2H), 1.85 - 1.69 (m, 1H). |
| | [M+H]⁺ = 497.9 |
| **98** | ¹H NMR (400 MHz, *d₆*-DMSO) δ 8.16 (d, J = 7.7 Hz, 1H), 8.09 (s, 2H), 7.80 (dd, J = 2.0, 8.4 Hz, 1H), 7.73 (d, J = 2.0 Hz, 1H), 7.44 (s, 1H), 7.20 - 7.14 (m, 1H), 7.12 (t, J = 7.7 Hz, 1H), 7.08 - 7.03 (m, 1H), 6.88 (d, J = 8.4 Hz, 1H), 4.05 (qd, J = 6.7, 13.9 Hz, 1H), 3.51 (s, 4H), 1.97 - 1.88 (m, 1H), 1.15 (d, J = 6.6 Hz, 6H), 0.97 - 0.91 (m, 2H), 0.61 - 0.55 (m, 2H). |
| | [M+H]⁺ = 424.1 |
| **99** | ¹H NMR (400 MHz, *d₆*-DMSO) δ 8.59 (s, 1H), 8.16 (s, 2H), 7.87 - 7.80 (m, 1H), 7.79 (s, 1H), 7.36 (s, 1H), 7.24 (t, J = 7.8 Hz, 1H), 7.12 (d, J = 8.0 Hz, 1H), 7.02 (d, J = 8.4 Hz, 1H), 6.88 (d, J = 7.2 Hz, 1H), 3.54 (s, 4H), 1.87 (ddd, J = 13.7, 8.2, 5.5 Hz, 1H), 1.40 (s, 3H), 1.00 - 0.89 (m, 2H), 0.78 - 0.70 (m, 2H), 0.65 - 0.56 (m, 4H). |
| | [M+H]⁺ = 452.1 |
| **100** | ¹H NMR (400 MHz, *d₆*-DMSO) δ 9.37 (s, 1H), 8.13 (s, 2H), 7.91 (s, 1H), 7.83 (dd, J = 8.4, 2.1 Hz, 1H), 7.71 (d, J = 2.0 Hz, 1H), 7.14 - 7.02 (m, 2H), 6.76 (dd, J = 9.0, 5.9 Hz, 2H), 3.53 (s, 4H), 2.39 - 2.31 (m, 2H), 2.15 (ddd, J = 13.9, 8.6, 5.4 Hz, 1H), 1.50 (dt, J = 13.0, 6.5 Hz, 1H), 1.42 (q, J = 7.1, 6.6 Hz, 2H), 0.94 - 0.87 (m, 2H), 0.84 (d, J = 6.4 Hz, 6H), 0.70 - 0.62 (m, 2H). |
| | [M+H]⁺ = 435.1 |
| **101** | ¹H NMR (400 MHz, *d₆*-DMSO) δ 8.64 (s, 1H), 8.60 (s, 1H), 8.31 (d, J = 2.0 Hz, 1H), 8.17 (s, 2H), 8.08 (dd, J = 8.5, 2.0 Hz, 1H), 7.29 (d, J = 4.5 Hz, 2H), 7.11 (d, J = 8.6 Hz, 1H), 6.99 (t, J = 4.5 Hz, 1H), 3.54 (s, 4H), 2.86 (s, 3H), 1.39 (s, 3H), 0.73 (s, 2H), 0.62 - 0.56 (m, 2H). |
| | [M+H]⁺ = 474.0 |
| **102** | ¹H NMR (400 MHz, *d₆*-DMSO) δ 8.63 (s, 1H), 8.58 (d, J = 2.0 Hz, 1H), 8.29 (s, 1H), 8.20 (s, 2H), 8.17 (d, J = 2.1 Hz, 1H), 7.57 (d, J = 6.6 Hz, 1H), 7.38 (t, J = 7.8 Hz, 1H), 7.18 (d, J = 8.7 Hz, 1H), 7.14 - 7.09 (m, 1H), 3.54 (s, 4H), 3.26 (s, 3H), 1.40 (s, 3H), 0.74 (s, 2H), 0.63 - 0.57 (m, 2H). |
| | [M+H]⁺ = 489.9 |
| **103** | ¹H NMR (400 MHz, *d₆*-DMSO) δ 8.11 (s, 2H), 7.85 (dd, J = 8.4, 1.8 Hz, 1H), 7.79 (d, J = 1.7 Hz, 1H), 7.43 (s, 1H), 7.31 (t, J = 7.9 Hz, 1H), 7.10 (d, J = 6.8 Hz, 1H), 7.06 (dd, J = 5.2, 2.2 Hz, 2H), 4.36 (s, 1H), 3.79 (s, 1H), 3.62 (s, 2H), 3.52 (s, 4H), 3.37 (d, J = 7.6 Hz, 1H), 3.27 (s, 2H), 3.09 (s, 1H), 1.89 (s, 1H), 0.94 (d, J = 8.4 Hz, 2H), 0.61 (s, 2H). |
| | [M+H]⁺ = 515.9 |
| **104** | ¹H NMR (400 MHz, *d₆*-DMSO) δ 8.11 (s, 2H), 7.85 (d, J = 8.6 Hz, 1H), 7.79 (s, 1H), 7.44 - 7.37 (m, 1H), 7.29 (t, J = 7.7 Hz, 1H), 7.12 - 6.73 (m, 3H), 4.38 - 3.65 (m, 3H), 3.52 (s, 4H), 3.40 (s, 2H), 3.29 (s, 1H), 3.11 - 2.99 (m, 1H), 2.34 - 2.24 (m, 1H), 1.88 (s, 1H), 1.05 - 0.57 (m, 10H). |
| | [M+H]⁺ = 510.0 |
| **105** | ¹H NMR (400 MHz, *d₆*-DMSO) δ 8.11 (s, 2H), 7.87 - 7.80 (m, 1H), 7.79 (d, J = 1.9 Hz, 1H), 7.48 - 7.37 (m, 1H), 7.29 (td, J = 7.8, 3.5 Hz, 1H), 7.13 - 6.80 (m, 3H), 4.52 - 3.73 (m, 2H), 3.52 (s, 4H), 3.44 (d, J = 19.8 Hz, 1H), 3.25 - 2.66 (m, 4H), 1.93 - 1.81 (m, 1H), 1.81 - 1.51 (m, 1H), 0.97 - 0.56 (m, 10H). |
| | [M+H]⁺ = 510.0 |
| **106** | ¹H NMR (400 MHz, *d₆*-DMSO) δ 8.13 (s, 2H), 8.00 (d, J = 7.9 Hz, 1H), 7.82 - 7.76 (m, 1H), 7.61 (s, 1H), 7.48 (d, J = 2.0 Hz, 1H), 7.12 (t, J = 7.7 Hz, 1H), 7.08 - 7.02 (m, 2H), 6.53 - 6.47 (m, 1H), 4.10 - 3.98 (m, 1H), 3.52 (s, 4H), 2.14 (s, 3H), 2.00 - 1.90 (m, 1H), 1.13 (d, J = 6.6 Hz, 6H), 0.85 - 0.76 (m, 2H), 0.56 - 0.48 (m, 2H). |
| | [M+H]⁺ = 420.2 |
| **107** | ¹H NMR (400 MHz, *d₆*-DMSO) δ 8.42 (s, 1H), 8.08 (s, 2H), 7.81 - 7.73 (m, 2H), 7.72 (d, J = 1.9 Hz, 1H), 7.50 (d, J = 1.7 Hz, 1H), 7.32 - 7.24 (m, 2H), 7.21 - 7.14 (m, 1H), 7.10 (d, J = 8.4 Hz, 1H), 3.51 (s, 4H), 1.99 - 1.89 (m, 1H), 1.79 - 1.65 (m, 1H), 1.49 (d, J = 7.0 Hz, 2H), 0.99 - 0.87 (m, 8H), 0.76 - 0.69 (m, 2H), 0.61 - 0.53 (m, 4H). |
| | [M+H]⁺ = 460.2 |
| **108** | ¹H NMR (400 MHz, *d₆*-DMSO) δ 9.54 (s, 1H), 8.08 (s, 2H), 7.79 (dd, J = 8.4, 2.0 Hz, 1H), 7.71 (d, J = 1.9 Hz, 1H), 7.62 (s, 1H), 7.53 (s, 1H), 7.18 - 7.11 (m, 3H), 6.82 - 6.76 (m, 1H), 4.36 (dd, J = 8.1, 5.5 Hz, 1H), 4.01 - 3.93 (m, 1H), 3.86 - 3.77 (m, 1H), 3.51 (s, 4H), 2.17 (dq, J = 12.1, 7.8 Hz, 1H), 2.01 - 1.91 (m, 2H), 1.89 - 1.80 (m, 2H), 0.97 - 0.91 (m, 2H), 0.60 - 0.53 (m, 2H). |
| | [M+H]⁺ = 434.3 |
| **109** | ¹H NMR (400 MHz, *d₆*-DMSO) δ 8.48 (t, J = 5.9 Hz, 1H), 8.11 (s, 2H), 7.85 (dd, J = 8.3, 1.9 Hz, 1H), 7.80 (d, J = 1.8 Hz, 1H), 7.34 (s, 1H), 7.25 (t, J = 7.8 Hz, 1H), 7.11 (dd, J = 8.2, 1.4 Hz, 1H), 7.04 (d, J = 8.3 Hz, 1H), 6.90 (dd, J = 7.4, 1.4 Hz, 1H), 3.97 (p, J = 6.3 Hz, 1H), 3.84 - 3.74 (m, 1H), 3.64 (q, J = 7.4 Hz, 1H), 3.52 (s, 4H), 3.37 - 3.32 (m, 1H), 3.29 - 3.22 (m, 1H), 2.00 - 1.76 (m, 4H), 1.70 - 1.60 (m, 1H), 0.99 - 0.91 (m, 2H), 0.64 - 0.57 (m, 2H). |
| | [M+H]⁺ = 482.1 |
| **110** | ¹H NMR (400 MHz, *d₆*-DMSO) δ 9.72 (d, J = 3.5 Hz, 2H), 7.81 (dd, J = 8.4, 2.0 Hz, 1H), 7.78 - 7.70 (m, 2H), 7.50 (s, 1H), 7.20 - 7.12 (m, 3H), 6.80 (dt, J = 7.0, 2.1 Hz, 1H), 3.83 (dd, J = 10.0, 7.2 Hz, 2H), 3.64 - 3.55 (m, 2H), 2.73 (s, 3H), 2.63 - 2.53 (m, 1H), 1.98 (ddd, J = 13.7, 8.4, 5.4 Hz, 1H), 1.09 (d, J = 6.8 Hz, 6H), 1.02 - 0.92 (m, 2H), 0.62 - 0.53 (m, 2H). |
| | [M+H]⁺ = 448.3 |
| **111** | ¹H NMR (400 MHz, *d₆*-DMSO) δ 8.30 (s, 1H), 8.08 (s, 2H), 7.78 (dd, J = 8.3, 1.9 Hz, 1H), 7.76 - 7.69 (m, 2H), 7.48 (d, J = 2.0 Hz, 1H), 7.27 (d, J = 5.3 Hz, 2H), 7.20 - 7.13 (m, 1H), 7.11 (d, J = 8.4 Hz, 1H), 3.51 (s, 4H), 1.98 - 1.88 (m, 1H), 0.98 - 0.85 (m, 11H), 0.85 -0.79 (m, 2H), 0.69 - 0.62 (m, 2H), 0.61 - 0.53 (m, 2H). |
| | [M+H]⁺ = 460.2 |

The following examples are provided as illustrations and in no way limit the scope of this invention.

The following examples illustrate in detail the preparation of some compounds according to the invention. The structures of the products obtained have been confirmed by NMR spectra.

### EXAMPLES

### Example 1: compound (7) in Table I

According to procedure (E), a solution of methyl 2-bromo-3-chlorobenzoate (500 mg, 2.0 mmoles, 1 eq) and potassium cyclopropyltrifluoroborate (445 mg, 3.0 mmoles, 1.5 eq.) in toluene (10 mL) and water (2 mL) was degassed with argon during 5 minutes then tripotassium phosphate (1.08 g, 5.0 mmoles, 2.5 eq.), RuPhos (37.4 mg, 80 µmoles, 0.04 eq.) and palladium(II) acetate (9.1 mg, 40 µmoles, 0.02 eq.) were added. The reaction mixture was heated at 110°C and stirred for 2h30 under inert atmosphere. Upon cooling down to room temperature, it was filtered over a pad of celite and the pad was washed with EtOAc. A saturated aqueous solution of brine was then added to the filtrate and the mixture was extracted with EtOAc. The combined organic layers were dried over MgSO₄, filtered and concentrated under reduced pressure. The resulting residue was purified by column chromatography on silica gel to give methyl 3-chloro-2-cyclopropylbenzoate (201 mg, 47%).
¹H NMR (400 MHz, d6-DMSO) δ 7.59 (dd, J = 8.0, 1.3 Hz, 1H), 7.46 (dd, J = 7.7, 1.3 Hz, 1H), 7.35 (t, J = 7.8 Hz, 1H), 3.86 (s, 3H), 1.94 (tt, J = 8.5, 5.8 Hz, 1H), 1.08 - 0.93 (m, 2H), 0.47 - 0.34 (m, 2H).

According to procedure (B), methyl 3-chloro-2-cyclopropylbenzoate (200 mg, 949 µmoles, 1 eq.) was placed in methanol (2 mL) and a 4M aqueous solution of NaOH (1.2 mL, 4.75 mmoles, 5 eq.) was added. The reaction mixture was heated at 80°C and stirred for 3 hours. It was then concentrated under reduced pressure and, after addition of an aqueous solution of 2M HCl (10 eq.), extracted with dichloromethane. The combined organic phases were dried over magnesium sulphate, filtered and concentrated under reduced pressure to give 3-chloro-2-cyclopropylbenzoic acid (176 mg, 71%).
¹H NMR (400 MHz, d6-DMSO) δ 13.16 (s, 1H), 7.54 (dd, J = 7.9, 1.3 Hz, 1H), 7.43 (dd, J = 7.6, 1.3 Hz, 1H), 7.36 - 7.27 (m, 1H), 1.94 (tt, J = 8.5, 5.7 Hz, 1H), 1.08 - 0.93 (m, 2H), 0.55 - 0.40 (m, 2H).

3-chloro-2-cyclopropylbenzoic acid (170 mg, 865 µmoles, 1 eq.) and 3-methylbutan-1-amine (110 µL, 951 µmoles, 1.1 eq.) were placed in anhydrous *N*,*N-*dimethylformamide (2 mL). HATU (329 mg, 865 µmoles, 1 eq.) and DIPEA (226 µL, 1.30 mmole, 1.5 eq.) were added and the resulting reaction mixture was stirred at room temperature for 16 hours. The reaction was quenched with 1M aqueous hydrochloric acid and extracted with ethyl acetate. The combined organic phases were dried over magnesium sulphate, filtered and concentrated under reduced pressure. The resulting residue was purified by column chromatography on silica gel to give 3-chloro-2-cyclopropyl-N-(3-methylbutyl)benzamide (168 mg, 73%).
¹H NMR (400 MHz, d6-DMSO) δ 8.23 (t, J = 5.4 Hz, 1H), 7.46 (dd, J = 7.9, 1.3 Hz, 1H), 7.27 (t, J = 7.8 Hz, 1H), 7.18 (dd, J = 7.6, 1.2 Hz, 1H), 3.28 - 3.19 (m, 2H), 1.92 (tt, J = 8.6, 5.7 Hz, 1H), 1.65 (tq, J = 13.3, 6.7 Hz, 1H), 1.42 (q, J = 7.0 Hz, 2H), 0.98 - 0.88 (m, 8H), 0.58 - 0.44 (m, 2H).

According to route (A1), a mixture of 3-chloro-2-cyclopropyl-N-(3-methylbutyl)benzamide (99.4 mg, 374 µmoles, 1.1 eq.), methyl 4-amino-3-cyclopropylbenzoate (65 mg, 340 µmoles, 1 eq.), Pd(OAc)₂ (2.3 mg, 10.2 µmoles, 3 mol%), rac-BINAP (4.2 mg, 6.8 µmoles, 2 mol%) and K₂CO₃ (141 mg, 1.02 mmole, 3 eq.) in anhydrous toluene (1 mL) was degassed with N₂ and heated at 130°C for 75 minutes under inert atmosphere. The reaction mixture was cooled down to room temperature, filtered over a pad of celite and the pad was washed with EtOAc. A saturated aqueous solution of brine was then added to the filtrate and the mixture was extracted with EtOAc. The combined organic phases were dried over MgSO₄, filtered and concentrated under reduced pressure. The resulting residue was purified by column chromatography on silica gel to give methyl 3-cyclopropyl-4-({2-cyclopropyl-3-[(3-methylbutyl)carbamoyl]phenyl}amino)benzoate (95 mg, 63%).
¹H NMR (400 MHz, *d₆*-DMSO) δ 8.17 (t, J = 5.6 Hz, 1H), 7.67 (dd, J = 8.5, 2.0 Hz, 1H), 7.63 (d, J = 1.7 Hz, 1H), 7.49 (s, 1H), 7.35 - 7.22 (m, 2H), 6.98 (dd, J = 7.2, 1.4 Hz, 1H), 6.91 (d, J = 8.5 Hz, 1H), 3.78 (s, 3H), 3.29 - 3.21 (m, 2H), 1.98 - 1.78 (m, 2H), 1.66 (dq, J = 13.3, 6.7 Hz, 1H), 1.43 (q, J = 7.0 Hz, 2H), 1.07 - 0.99 (m, 2H), 0.92 (d, J = 6.6 Hz, 6H), 0.83 - 0.75 (m, 2H), 0.70 - 0.61 (m, 2H), 0.42 (q, J = 5.7 Hz, 2H).

According to procedure (B), methyl 3-cyclopropyl-4-({2-cyclopropyl-3-[(3-methylbutyl)carbamoyl]phenyl}amino)benzoate (86.0 mg, 205 µmoles, 1 eq.) was placed in methanol (2 mL) and a 2M aqueous solution of NaOH (1.03 mL, 2.05 mmoles, 10 eq.) was added. The reaction mixture was heated at 80°C and stirred for 3 hours. It was then concentrated under reduced pressure and, after addition of an aqueous solution of 2M HCl (10 eq.), extracted with dichloromethane. The combined organic phases were dried over magnesium sulphate, filtered and concentrated under reduced pressure to give 3-cyclopropyl-4-({2-cyclopropyl-3-[(3-methylbutyl)carbamoyl]phenyl}amino)benzoic acid (73.0 mg, 83%).
¹H NMR (400 MHz, *d₆*-DMSO) δ 12.32 (s, 1H), 8.16 (t, J = 5.6 Hz, 1H), 7.70 - 7.60 (m, 2H), 7.41 (s, 1H), 7.27 (dt, J = 15.3, 7.3 Hz, 2H), 6.99 - 6.91 (m, 2H), 3.29 - 3.22 (m, 2H), 1.98 - 1.78 (m, 2H), 1.67 (dp, J = 13.0, 6.5 Hz, 1H), 1.43 (q, J = 7.0 Hz, 2H), 1.05 - 0.97 (m, 2H), 0.92 (d, J = 6.6 Hz, 6H), 0.86 - 0.78 (m, 2H), 0.70 - 0.61 (m, 2H), 0.43 (q, J = 5.8 Hz, 2H).

According to procedure (C), a reaction mixture of 3-cyclopropyl-4-({2-cyclopropyl-3-[(3-methylbutyl)carbamoyl]phenyl}amino)benzoic acid (66 mg, 154 µmoles, 1.0 eq.) and CDI (30.0 mg, 185 µmoles, 1.2 eq.) in anhydrous DMF (1.0 mL) was stirred at room temperature for 1 hour. The mixture was then added to a solution of imidazolidin-2-imine hydrobromide (53.9 mg, 309 µmoles, 2 eq.) and DIPEA (80.6 µL, 463 µmoles, 3 eq.) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was triturated in acetonitrile to afford 2-cyclopropyl-3-({2-cyclopropyl-4-[(imidazolidin-2-ylidene)carbamoyl]phenyl}amino)-N-(3-methylbutyl)benzamide (7) (20.0 mg, 26%).
¹H NMR (400 MHz, *d₆*-DMSO) δ 8.14 (t, J = 5.5 Hz, 1H), 8.08 (s, 2H), 7.82 (d, J = 8.5 Hz, 2H), 7.26 - 7.15 (m, 3H), 7.03 (d, J = 8.2 Hz, 1H), 6.84 (d, J = 6.2 Hz, 1H), 3.51 (s, 4H), 3.25 (q, J = 6.5 Hz, 2H), 1.96 - 1.78 (m, 2H), 1.67 (dp, J = 13.3, 6.7 Hz, 1H), 1.43 (q, J = 7.0 Hz, 2H), 1.04 - 0.95 (m, 2H), 0.92 (d, J = 6.6 Hz, 6H), 0.87 (d, J = 8.6 Hz, 2H), 0.62 (q, J = 5.4 Hz, 2H), 0.45 (q, J = 5.4 Hz, 2H).
¹³C NMR (151 MHz, *d₆*-DMSO) δ 175.7, 169.6, 165.7, 145.6, 143.1, 141.8, 130.6, 129.6, 129.0, 128.7, 128.2, 127.1, 120.5, 119.0, 114.2, 41.7, 38.4, 37.7, 25.8, 22.9, 11.9, 11.0, 7.3, 6.5
[M+H]⁺ = 474.2

### Example 2: compound (25) in Table I

3-bromo-2-chlorobenzoic acid (500 mg, 2.1 mmoles, 1 eq.) and cyclopropanamine (173 µL, 2.5 mmoles, 1.2 eq.) were placed in anhydrous *N*,*N-*dimethylformamide (5 mL). HATU (1.24 g, 3.1 mmoles, 1.5 eq.) and DIPEA (1.1 mL, 6.2 mmoles, 3 eq.) were added and the resulting reaction mixture was stirred at room temperature for 16 hours. The reaction was quenched with 1M aqueous hydrochloric acid and extracted with ethyl acetate. The combined organic phases were dried over magnesium sulphate, filtered and concentrated under reduced pressure. The resulting residue was purified by column chromatography on silica gel to give 3-bromo-2-chloro-N-cyclopropylbenzamide (375 mg, 60%).
¹H NMR (400 MHz, *d₆*-DMSO) δ 8.54 (d, J = 4.0 Hz, 1H), 7.82 (dd, J = 8.0, 1.6 Hz, 1H), 7.40 (dd, J = 7.6, 1.6 Hz, 1H), 7.32 (t, J = 7.8 Hz, 1H), 2.81 (td, J = 7.3, 4.0 Hz, 1H), 0.70 (td, J = 7.0, 4.8 Hz, 2H), 0.54 - 0.49 (m, 2H).

According to route (A1), a reaction mixture of 3-bromo-2-chloro-N-cyclopropylbenzamide (108 mg, 0.392 mmole, 1.0 eq.), methyl 4-amino-3-cyclopropylbenzoate (75 mg, 0.392 mmole, 1.0 eq.), BrettPhos Pd G3 (26.7 mg, 29.4 µmoles, 7.5 mol%) and Cs₂CO₃ (153 mg, 0.471 mmole, 1.2 eq.) in anhydrous DMF (2 mL) was degassed with N₂ and heated at 80°C for 75 minutes under inert atmosphere. The reaction mixture was cooled down to room temperature, filtered over a pad of celite and the pad was washed with EtOAc. A saturated aqueous solution of brine was then added to the filtrate and the mixture was extracted with EtOAc. The combined organic phases were dried over MgSO₄, filtered and concentrated under reduced pressure. The resulting residue was purified by column chromatography on silica gel to give methyl 4-{[2-chloro-3-(cyclopropylcarbamoyl)phenyl]amino}-3-cyclopropylbenzoate (75 mg, 41%).
¹H NMR (500 MHz, *d₆*-DMSO) δ 8.47 (d, J = 4.4 Hz, 1H), 7.69 (dd, J = 8.5, 2.0 Hz, 1H), 7.63 - 7.58 (m, 2H), 7.36 - 7.29 (m, 2H), 7.08 (s, 1H), 6.89 (d, J = 8.5 Hz, 1H), 3.79 (s, 3H), 2.82 (td, J = 7.3, 3.8 Hz, 1H), 1.88 (s, 1H), 1.01 - 0.96 (m, 2H), 0.69 (dd, J = 7.1, 2.2 Hz, 2H), 0.64 (dd, J = 5.4, 1.7 Hz, 2H), 0.53 (dd, J = 3.9, 2.4 Hz, 2H).

According to procedure (B), methyl 4-{[2-chloro-3-(cyclopropylcarbamoyl)phenyl] amino }-3-cyclopropylbenzoate (75 mg, 0.195 mmole, 1 eq.) was placed in methanol (3 mL) and an aqueous solution of 2M NaOH (0.808 mL, 1.62 mmole, 10 eq.) was added. The reaction mixture was heated at 80°C and stirred for 3 hours. It was then concentrated under reduced pressure and, after addition of an aqueous solution of 2M HCl (10 eq.), extracted with dichloromethane. The combined organic phases were dried over magnesium sulphate, filtered and concentrated under reduced pressure to give 4-{[2-chloro-3-(cyclopropylcarbamoyl)phenyl]amino}-3-cyclopropylbenzoic acid (70 mg, 97%).
¹H NMR (400 MHz, *d₆*-DMSO) δ 12.46 (s, 1H), 8.48 (d, J = 4.4 Hz, 1H), 7.69 (dd, J = 8.4, 2.0 Hz, 1H), 7.60 (d, J = 1.8 Hz, 1H), 7.54 (s, 1H), 7.35 - 7.29 (m, 1H), 7.27 (dd, J = 8.1, 1.8 Hz, 1H), 7.04 (dd, J = 7.2, 1.8 Hz, 1H), 6.93 (d, J = 8.4 Hz, 1H), 2.83 (td, J = 7.3, 4.0 Hz, 1H), 1.93 - 1.84 (m, 1H), 1.01 - 0.95 (m, 2H), 0.69 (dt, J = 7.0, 3.4 Hz, 2H), 0.64 (dd, J = 5.4, 1.8 Hz, 2H), 0.53 (dd, J = 4.0, 2.3 Hz, 2H).

According to procedure (C), a reaction mixture of 4-{[2-chloro-3-(cyclopropylcarbamoyl)phenyl]amino}-3-cyclopropylbenzoic acid (70 mg, 188 µmoles, 1.0 eq.) and CDI (36.5 mg, 225 µmoles, 1.2 eq.) in anhydrous DMF (1.0 mL) was stirred at room temperature for 1 hour. The mixture was then added to a solution of imidazolidin-2-imine hydrobromide (39.4 mg, 225 µmoles, 1.2 eq.) and DIPEA (98 µL, 563 µmoles, 3 eq.) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was purified by column chromatography on silica gel to give 4-{[2-chloro-3-(cyclopropylcarbamoyl)phenyl]amino}-3-cyclopropyl-N-(imidazolidin-2-ylidene)benzamide **(25)** (18.2 mg, 22%).
¹H NMR (400 MHz, *d₆*-DMSO) δ 8.45 (d, J = 4.5 Hz, 1H), 8.11 (s, 2H), 7.85 (dd, J = 8.3, 1.9 Hz, 1H), 7.79 (d, J = 1.8 Hz, 1H), 7.33 (s, 1H), 7.23 (s, 1H), 7.10 (d, J = 1.5 Hz, 1H), 7.04 (d, J = 8.3 Hz, 1H), 6.92 - 6.85 (m, 1H), 3.52 (s, 4H), 2.87 - 2.79 (m, 1H), 1.87 (s, 1H), 0.98 - 0.91 (m, 2H), 0.70 (td, J = 7.1, 4.8 Hz, 2H), 0.60 (dd, J = 5.5, 1.8 Hz, 2H), 0.56 - 0.50 (m, 2H).
¹³C NMR (151 MHz, *d₆*-DMSO) δ 175.5, 168.1, 165.8, 144.0, 140.9, 138.9, 132.9, 132.6, 127.9, 127.8, 120.3, 119.5, 118.8, 118.1, 41.7, 23.1, 11.7, 7.3, 6.1
[M+H]⁺ = 438.0

### Example 3: compound (26) in Table I

2-methylpropanoic acid (461 mg, 5.23 mmoles, 1.2 eq.) and 3-bromoaniline (475 µL, 4.36 mmoles, 1.0 eq.) were placed in anhydrous *N*,*N*-dimethylformamide (10 mL). HATU (2.59 g, 6.54 mmoles, 1.5 eq.) and DIPEA (2.30 mL, 13.1 mmoles, 3.0 eq.) were added and the resulting reaction mixture was stirred at room temperature for 16 hours. The reaction was quenched with 1M aqueous hydrochloric acid and extracted with ethyl acetate. The combined organic phases were dried over magnesium sulphate, filtered and concentrated under reduced pressure. The resulting residue was purified by column chromatography on silica gel to give N-(3-bromophenyl)-2-methylpropanamide (979 mg, 88%).
¹H NMR (400 MHz, *d₆*-DMSO) δ 9.98 (s, 1H), 7.98 (d, J = 1.8 Hz, 1H), 7.51 (d, J = 7.9 Hz, 1H), 7.26 (t, J = 7.9 Hz, 1H), 7.21 (d, J = 8.1 Hz, 1H), 2.58 (p, J = 6.8 Hz, 1H), 1.10 (d, J = 6.8 Hz, 6H).

According to route (A1), a reaction mixture of N-(3-bromophenyl)-2-methylpropanamide (279 mg, 1.15 mmole, 1.1 eq.), methyl 4-amino-3-cyclopropyl-benzoate (200 mg, 1.05 mmole, 1.0 eq.), BrettPhos Pd G3 (47.4 mg, 52.3 µmoles, 5 mol%) and Cs₂CO₃ (409 mg, 1.26 mmole, 1.2 eq.) in anhydrous DMF (3 mL) was degassed with N₂ and heated at 80°C for 75 minutes under inert atmosphere. The reaction mixture was cooled down to room temperature, filtered over a pad of celite and the pad was washed with EtOAc. A saturated aqueous solution of brine was then added to the filtrate and the mixture was extracted with EtOAc. The combined organic phases were dried over MgSO₄, filtered and concentrated under reduced pressure. The resulting residue was purified by column chromatography on silica gel to give methyl 3-cyclopropyl-4-{ [3-(2-methylpropanamido)phenyl]amino}benzoate (368 mg, 99%).
¹H NMR (400 MHz, *d₆*-DMSO) δ 9.77 (s, 1H), 7.86 (s, 1H), 7.66 (dd, J = 8.5, 2.1 Hz, 1H), 7.59 (d, J = 1.9 Hz, 1H), 7.54 (d, J = 2.0 Hz, 1H), 7.25 - 7.17 (m, 2H), 7.14 (d, J = 8.5 Hz, 1H), 6.85 (d, J = 7.3 Hz, 1H), 3.78 (s, 3H), 2.63 - 2.53 (m, 1H), 1.95 (s, 1H), 1.09 (d, J = 6.8 Hz, 6H), 0.98 (dd, J = 8.3, 2.0 Hz, 2H), 0.60 (d, J = 3.6 Hz, 2H).

According to procedure (B), methyl 3-cyclopropyl-4-{[3-(2-methylpropanamido)phenyl]amino}benzoate (368 mg, 1.04 mmole, 1 eq.) was placed in methanol (3 mL) and an aqueous solution of 2M NaOH (2.61 mL, 5.21 mmoles, 5 eq.) was added. The reaction mixture was heated at 80°C and stirred for 3 hours. It was then concentrated under reduced pressure and, after addition of an aqueous solution of 2M HCl (10 eq.), extracted with dichloromethane. The combined organic phases were dried over magnesium sulphate, filtered and concentrated under reduced pressure to give 3-cyclopropyl-4-{[3-(2-methylpropanamido)phenyl]amino}benzoic acid (278 mg, 79%).
¹H NMR (400 MHz, *d₆*-DMSO) δ 12.34 (s, 1H), 9.75 (s, 1H), 7.80 (s, 1H), 7.64 (dd, J = 8.5, 2.0 Hz, 1H), 7.54 (dd, J = 12.8, 1.9 Hz, 2H), 7.24 - 7.10 (m, 3H), 6.84 (dt, J = 7.3, 1.9 Hz, 1H), 2.58 (p, J = 6.8 Hz, 1H), 1.95 (tt, J = 8.4, 5.4 Hz, 1H), 1.09 (d, J = 6.8 Hz, 6H), 0.97 (dd, J = 8.3, 2.0 Hz, 2H), 0.63 - 0.56 (m, 2H).

According to procedure (C), a reaction mixture of 3-cyclopropyl-4-{[3-(2-methylpropanamido)phenyl]amino}benzoic acid (178 mg, 526 µmoles, 1.0 eq.) and CDI (102 mg, 631 µmoles, 1.2 eq.) in anhydrous DMF (1.0 mL) was stirred at room temperature for 1 hour. The mixture was then added to a solution of imidazolidin-2-imine hydrobromide (110 mg, 631 µmoles, 1.2 eq.) and DIPEA (276 µL, 1.58 mmole, 3 eq.) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was purified by column chromatography on silica gel followed by preparative TLC to give 3-cyclopropyl-N-(imidazolidin-2-ylidene)-4-{[3-(2-methylpropanamido)phenyl]amino}benzamide **(26)** (25.7 mg, 12%).
¹H NMR (400 MHz, *d₆*-DMSO) δ 9.71 (s, 1H), 8.21 (s, 2H), 7.82 - 7.73 (m, 1H), 7.69 (s, 2H), 7.51 (s, 1H), 7.13 (dt, J = 13.7, 7.3 Hz, 3H), 6.78 (d, J = 7.7 Hz, 1H), 3.56 (s, 4H), 2.61 - 2.54 (m, 1H), 1.98 (d, J = 14.4 Hz, 1H), 1.08 (d, J = 6.8 Hz, 6H), 0.95 (d, J = 8.4 Hz, 2H), 0.58 (d, J = 4.0 Hz, 2H).
¹³C NMR (151 MHz, *d₆*-DMSO) δ 175.6, 146.1, 144.0, 140.6, 131.1, 129.5, 127.8, 127.5, 116.0, 114.2, 112.2, 109.9, 41.9, 35.3, 19.9, 11.6, 7.6
[M+H]⁺ = 406.0

### Example 4: compound (41) in Table I

According to procedure (E), a solution of methyl 4-amino-3-bromobenzoate (3.00 g, 12.8 mmoles, 1 eq.) and potassium cyclopropyltrifluoroborate (2.84 g, 19.2 mmoles, 1.5 eq.) in toluene (52.5 mL) and water (13.5 mL) was degassed with argon during 5 minutes then tripotassium phosphate (6.88 g, 31.9 mmoles, 2.5 eq.), RuPhos (239 mg, 511 µmoles, 0.04 eq.) and palladium(II) acetate (57.9 mg, 256 µmoles, 0.02 eq.) were added. The reaction mixture was heated at 110°C and stirred for 2h30 under inert atmosphere. Upon cooling down to room temperature, it was filtered over a pad of celite and the pad was washed with EtOAc. A saturated aqueous solution of brine was then added to the filtrate and the mixture was extracted with EtOAc. The combined organic layers were dried over MgSO₄, filtered and concentrated under reduced pressure. The resulting residue was purified by column chromatography on silica gel to give methyl 4-amino-3-cyclopropylbenzoate (2.02 g, 81 %). ¹H NMR (400 MHz, *d₆*-DMSO) δ 7.53 (dd, J = 8.4, 2.0 Hz, 1H), 7.42 (d, J = 1.9 Hz, 1H), 6.63 (d, J = 8.4 Hz, 1H), 5.87 (s, 2H), 3.73 (s, 3H), 1.65 (tt, J = 8.3, 5.4 Hz, 1H), 0.95 - 0.82 (m, 2H), 0.54 - 0.40 (m, 2H).

3-Bromophenol (701 mg, 3.97 mmoles, 1.2 eq.) was placed in *N*,*N-*dimethylformamide (4 mL) with Cs₂CO₃ (1.3 g, 3.97 mmoles, 1.2 eq.). Upon addition of (3-bromopropyl)cyclohexane (715 mg, 3.31 mmoles, 1 eq.), the reaction mixture was stirred at room temperature for 16 hours under an inert atmosphere of argon. To the reaction mixture was added a saturated aqueous solution of NaHCO₃ and it was extracted with ethyl acetate. The combined organic phases were dried over magnesium sulphate, filtered and concentrated under reduced pressure. The resulting residue was purified by column chromatography on silica gel to give 1-bromo-3-(3-cyclohexylpropoxy)benzene (882 mg, 90%).
¹H NMR (500 MHz, *d₆*-DMSO) δ 7.22 (t, J = 8.1 Hz, 1H), 7.14 - 7.08 (m, 2H), 6.93 (dd, J = 8.3, 2.3 Hz, 1H), 3.95 (t, J = 6.5 Hz, 2H), 1.68 (tt, J = 15.1, 9.2 Hz, 7H), 1.32 - 1.06 (m, 6H), 0.92 - 0.82 (m, 2H).

According to procedure (A1), a reaction mixture of 1-bromo-3-(3-cyclohexylpropoxy)benzene (547 mg, 1.84 mmole, 1.1 eq.), methyl 4-amino-3-cyclopropylbenzoate (320 mg, 1.67 mmole, 1 eq.), BrettPhos Pd G3 (31.9 mg, 33.5 µmoles, 2 mol%) and Cs₂CO₃ (818 mg, 2.51 mmoles, 1.5 eq.) in anhydrous DMF (8 mL) was degassed with N₂ and heated at 80°C for 75 minutes under inert atmosphere. The reaction mixture was cooled down to room temperature, filtered over a pad of celite and the pad was washed with EtOAc. A saturated aqueous solution of brine was then added to the filtrate and the mixture was extracted with EtOAc. The combined organic phases were dried over MgSO₄, filtered and concentrated under reduced pressure. The resulting residue was purified by column chromatography on silica gel to give methyl 4-{[3-(3-cyclohexylpropoxy)phenyl]amino}-3-cyclopropylbenzoate (1.35 g, 80%).
¹H NMR (400 MHz, *d₆*-DMSO) δ 7.82 (s, 1H), 7.66 (dd, J = 8.5, 2.0 Hz, 1H), 7.54 (d, J = 2.0 Hz, 1H), 7.24 - 7.14 (m, 2H), 6.76 (d, J = 7.9 Hz, 1H), 6.73 (t, J = 2.1 Hz, 1H), 6.56 (dd, J = 8.1, 2.2 Hz, 1H), 3.92 (t, J = 6.5 Hz, 2H), 3.78 (s, 3H), 1.94 (ddd, J = 13.8, 8.3, 5.4 Hz, 1H), 1.75 - 1.58 (m, 7H), 1.35 - 1.08 (m, 6H), 1.04 - 0.94 (m, 2H), 0.88 (q, J = 10.0, 9.3 Hz, 2H), 0.65 - 0.56 (m, 2H).

According to procedure (B), methyl 4-{[3-(3-cyclohexylpropoxy)phenyl]amino}-3-cyclopropylbenzoate (575 mg, 1.34 mmole, 1 eq.) was placed in methanol (10 mL) and an aqueous solution of 2M NaOH (4.7 mL, 9.4 mmoles, 7 eq.) was added. The reaction mixture was heated at 80°C and stirred for 3 hours. It was then concentrated under reduced pressure and, after addition of an aqueous solution of 2M HCl (7 mL, 14 mmoles, 10.5 eq.), extracted with dichloromethane. The combined organic phases were dried over magnesium sulphate, filtered and concentrated under reduced pressure to give 4-{[3-(3-cyclohexylpropoxy)phenyl]amino}-3-cyclopropylbenzoic acid (540 mg, 97 %).
¹H NMR (400 MHz, *d₆*-DMSO) δ 12.37 (s, 1H), 7.76 (s, 1H), 7.64 (dd, J = 8.5, 2.0 Hz, 1H), 7.52 (d, J = 1.9 Hz, 1H), 7.18 (t, J = 8.6 Hz, 2H), 6.74 (d, J = 7.9 Hz, 1H), 6.71 (d, J = 2.1 Hz, 1H), 6.53 (dd, J = 8.1, 2.1 Hz, 1H), 3.91 (t, J = 6.5 Hz, 2H), 1.94 (ddd, J = 13.6, 8.4, 5.4 Hz, 1H), 1.75 - 1.58 (m, 7H), 1.35 - 1.09 (m, 6H), 0.98 (dd, J = 4.0, 2.0 Hz, 2H), 0.88 (q, J = 10.1, 9.3 Hz, 2H), 0.65 - 0.56 (m, 2H).

According to procedure (C), a reaction mixture of 4-{[3-(3-cyclohexylpropoxy)phenyl]amino}-3-cyclopropylbenzoic acid (150 mg, 362 µmoles, 1 eq.) and CDI (70.5 mg, 435 µmoles, 1.2 eq.) in anhydrous DMF (3.0 mL) was stirred at room temperature for 1 hour. The mixture was then added to a solution of guanidinium carbonate (2:1 salt) (130 mg, 724 µmoles, 2 eq.) and DIPEA (158 µL, 905 µmoles, 2.5 eq) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was purified by column chromatography on silica gel to give 4-{[3-(3-cyclohexylpropoxy)phenyl]amino}-3-cyclopropyl-N-(diaminomethylidene)benzamide (135 mg, 82%).
¹H NMR (400 MHz, *d₆*-DMSO) δ 7.79 (d, J = 8.4 Hz, 1H), 7.70 (s, 1H), 7.57 (s, 1H), 7.12 (dd, J = 11.2, 8.5 Hz, 2H), 6.64 (d, J = 8.0 Hz, 1H), 6.60 (s, 1H), 6.41 (d, J = 8.2 Hz, 1H), 3.89 (t, J = 6.4 Hz, 2H), 2.00 - 1.88 (m, 1H), 1.68 (t, J = 13.7 Hz, 7H), 1.34 - 1.09 (m, 6H), 0.91 (dd, J = 28.4, 9.6 Hz, 4H), 0.58 (d, J = 5.0 Hz, 2H).

To a solution of 4-{[3-(3-cyclohexylpropoxy)phenyl]amino}-3-cyclopropyl-N-(diaminomethylidene)benzamide (69.0 mg, 151 µmoles, 1 eq.) and DIPEA (131 µL, 754 µmoles, 5 eq.) in anhydrous DMF (1.5 mL) was added 2-chloroacetyl chloride (23.7 µL, 226 µmoles, 1.5 eq.). The resulting reaction mixture was stirred at room temperature for 2 hours and then heated at 90°C and stirred for 2 hours. The reaction mixture was then cooled down to room temperature, quenched with a saturated aqueous solution of sodium bicarbonate and extracted with DCM. The combined organic layers were then washed with a saturated aqueous solution of brine, dried over MgSO₄, filtered and concentrated under reduced pressure. The resulting residue was purified by column chromatography on silica gel to give 4-{[3-(3-cyclohexylpropoxy)phenyl]amino}-3-cyclopropyl-N-[(2E)-4-oxoimidazolidin-2-ylidene]benzamide (**41**) (4.0 mg, 5%).
¹H NMR (400 MHz, *d₆*-DMSO) δ 11.24 (s, 1H), 9.35 (s, 1H), 7.81 (d, J = 9.6 Hz, 1H), 7.75 (s, 2H), 7.16 (t, J = 8.2 Hz, 2H), 6.78 - 6.64 (m, 2H), 6.50 (d, J = 8.5 Hz, 1H), 4.06 (s, 2H), 3.91 (t, J = 6.5 Hz, 2H), 1.93 (d, J = 13.8 Hz, 1H), 1.67 (d, J = 14.8 Hz, 7H), 1.30 - 1.16 (m, 6H), 0.98 (d, J = 8.6 Hz, 2H), 0.86 (s, 2H), 0.61 (s, 2H).
[M+H]⁺ = 475.1

### Example 5: compound (45) in Table I

3-bromo-2-chlorobenzoic acid (200 mg, 832 µmoles, 1 eq.) and morpholine (87.0 mg, 999 µmoles, 1.2 eq.) were placed in anhydrous *N*,*N*-dimethylformamide (4 mL). HATU (495 mg, 1.25 mmole, 1.5 eq.) and DIPEA (438 µL, 2.50 mmoles, 3 eq.) were added and the resulting reaction mixture was stirred at room temperature for 16 hours. The reaction was quenched with 1M aqueous hydrochloric acid and extracted with ethyl acetate. The combined organic phases were dried over magnesium sulphate, filtered and concentrated under reduced pressure. The resulting residue was purified by column chromatography on silica gel to give 4-(3-bromo-2-chlorobenzoyl)morpholine (216 mg, 85%).
¹H NMR (400 MHz, *d₆*-DMSO) δ 7.84 (dd, J = 7.8, 1.7 Hz, 1H), 7.46 - 7.33 (m, 2H), 3.66 (qd, J = 8.3, 7.2, 3.8 Hz, 4H), 3.56 - 3.51 (m, 2H), 3.15 - 3.10 (m, 2H).

The experimental set-up was dried with a heat gun under a nitrogen stream, kept under inert atmosphere and then cooled down to room temperature. Methyl 3-acetyl-4-aminobenzoate (1.10 g, 5.41 mmoles, 1 eq.) and anhydrous THF (40 mL) were then added and the reaction mixture was cooled down to -10°C. A 3M methylmagnesium bromide solution in diethyl ether (3.79 mL, 11.4 mmoles, 2.1 eq.) was added dropwise and the reaction mixture was stirred at -10°C during 30 minutes, then additional 3M methylmagnesium bromide solution in diethyl ether (1.80 mL, 5.43 mmoles, 1 eq.) was added and the reaction mixture was further stirred at -10°C during 30 minutes. The reaction mixture was quenched with a saturated aqueous solution of ammonium chloride at -10°C then warmed up to room temperature and extracted with DCM. The combined organic phases were dried over magnesium sulphate, filtered and concentrated under reduced pressure. The resulting residue was purified by column chromatography on silica gel to give methyl 4-amino-3-(2-hydroxypropan-2-yl)benzoate (986 mg, 87%).
¹H NMR (400 MHz, *d₆*-DMSO) δ 7.64 (s, 1H), 7.55 (d, J = 8.4 Hz, 1H), 6.64 (d, J = 8.4 Hz, 1H), 6.22 (s, 2H), 5.37 (s, 1H), 3.74 (s, 3H), 1.51 (s, 6H).

According to route (A1), a mixture of 4-(3-bromo-2-chlorobenzoyl)morpholine (218 mg, 717 µmoles, 1.5 eq.), methyl 4-amino-3-(2-hydroxypropan-2-yl)benzoate (100 mg, 478 µmoles, 1 eq.), Pd(OAc)₂ (3.2 mg, 14.3 µmoles, 3 mol%), rac-BINAP (6.0 mg, 9.6 µmoles, 2 mol%) and K₂CO₃ (198 mg, 1.43 mmole, 3 eq.) in anhydrous toluene (3 mL) was degassed with N₂ and heated at 110°C for 75 minutes under inert atmosphere. The reaction mixture was cooled down to room temperature, filtered over a pad of celite and the pad was washed with EtOAc. A saturated aqueous solution of brine was then added to the filtrate and the mixture was extracted with EtOAc. The combined organic phases were dried over MgSO₄, filtered and concentrated under reduced pressure. The resulting residue was purified by column chromatography on silica gel to give methyl 4-{[2-chloro-3-(morpholine-4-carbonyl)phenyl]amino}-3-(2-hydroxypropan-2-yl)benzoate (278 mg, 99%).
¹H NMR (400 MHz, *d₆*-DMSO) δ 9.53 (s, 1H), 7.86 (d, J = 2.0 Hz, 1H), 7.79 (dd, J = 8.5, 2.0 Hz, 1H), 7.47 (dd, J = 8.2, 1.4 Hz, 1H), 7.33 (dd, J = 8.3, 4.5 Hz, 2H), 6.95 (dd, J = 7.4, 1.4 Hz, 1H), 6.18 (s, 1H), 3.82 (s, 3H), 3.67 (s, 4H), 3.58 - 3.54 (m, 2H), 3.22 - 3.18 (m, 2H), 1.58 (d, J = 5.8 Hz, 6H).

According to procedure (B), methyl 4-{[2-chloro-3-(morpholine-4-carbonyl)phenyl]amino}-3-(2-hydroxypropan-2-yl)benzoate (278 mg, 642 µmoles, 1 eq.) was placed in methanol (5 mL) and a 2M aqueous solution of NaOH (1.61 mL, 3.21 mmoles, 5 eq.) was added. The reaction mixture was heated at 80°C and stirred for 3 hours. It was then concentrated under reduced pressure and, after addition of an aqueous solution of 2M HCl (10 eq.), extracted with dichloromethane. The combined organic phases were dried over magnesium sulphate, filtered and concentrated under reduced pressure to give 4-{[2-chloro-3-(morpholine-4-carbonyl)phenyl]amino}-3-(2-hydroxypropan-2-yl)benzoic acid (240 mg, 55%).
¹H NMR (400 MHz, *d₆*-DMSO) δ 12.56 (s, 1H), 9.48 (s, 1H), 7.86 (d, J = 1.9 Hz, 1H), 7.77 (dd, J = 8.5, 1.9 Hz, 1H), 7.46 (d, J = 8.2 Hz, 1H), 7.33 (d, J = 1.9 Hz, 2H), 6.92 (dd, J = 7.4, 1.3 Hz, 1H), 6.14 (s, 1H), 3.67 (s, 4H), 3.58 - 3.54 (m, 2H), 3.22 - 3.18 (m, 2H), 1.58 (d, J = 6.5 Hz, 6H).

According to procedure (C), a reaction mixture of 4-{[2-chloro-3-(morpholine-4-carbonyl)phenyl]amino}-3-(2-hydroxypropan-2-yl)benzoic acid (80 mg, 191 µmoles, 1.0 eq.) and CDI (37.2 mg, 229 µmoles, 1.2 eq.) in anhydrous DMF (1.0 mL) was stirred at room temperature for 1 hour. The mixture was then added to a solution of imidazolidin-2-imine hydrobromide (63.4 mg, 382 µmoles, 2 eq.) and DIPEA (100 µL, 573 µmoles, 3 eq.) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was purified by column chromatography on silica gel to give 4-{[2-chloro-3-(morpholine-4-carbonyl)phenyl]amino}-3-(2-hydroxypropan-2-yl)-N-(imidazolidin-2-ylidene)benzamide **(45)** (9.9 mg, 11%).
¹H NMR (400 MHz, *d₆*-DMSO) δ 9.33 (s, 1H), 8.13 (s, 2H), 8.05 (s, 1H), 7.92 (d, J = 8.2 Hz, 1H), 7.42 (d, J = 7.4 Hz, 1H), 7.29 (dd, J = 8.1, 3.9 Hz, 2H), 6.85 (d, J = 7.7 Hz, 1H), 6.04 (s, 1H), 3.67 (s, 4H), 3.59 - 3.50 (m, 6H), 3.21 (d, J = 5.1 Hz, 2H), 1.56 (d, J = 10.1 Hz, 6H).
¹³C NMR (151 MHz, *d₆*-DMSO) δ 166.3, 140.3, 137.3, 135.5, 128.8, 128.6, 127.1, 118.9, 118.2, 117.9, 116.9, 72.8, 66.6, 66.4, 47.1, 41.9, 41.7, 29.9
[M+H]⁺ = 486.2

### Example 6: compound (46) in Table I

According to procedure (C), a reaction mixture of 4-{[3-(3-cyclohexylpropoxy)phenyl]amino}-3-cyclopropylbenzoic acid (100 mg, 241 µmoles, 1 eq.) and CDI (47.0 mg, 290 µmoles, 1.2 eq.) in anhydrous DMF (1.0 mL) was stirred at room temperature for 1 hour. The mixture was then added to a solution of imidazolidin-2-imine hydrobromide (84.4 mg, 483 µmoles, 2 eq.) and DIPEA (126 µL, 724 µmoles, 3 eq.) in anhydrous DMF (1.0 mL) and the resulting mixture was heated at 75°C and stirred for 16 hours. The reaction mixture was then cooled down to room temperature, quenched with a saturated aqueous solution of sodium bicarbonate and extracted with EtOAc. The combined organic layers were then washed with a saturated aqueous solution of brine, dried over MgSO₄, filtered and concentrated under reduced pressure. The resulting residue was purified by column chromatography on silica gel to give 4-{[3-(3-cyclohexylpropoxy)phenyl]amino}-3-cyclopropyl-N-(imidazolidin-2-ylidene)benzamide (27.0 mg, 23%).
¹H NMR (400 MHz, *d₆*-DMSO) δ 8.08 (s, 2H), 7.79 (dd, J = 8.4, 1.9 Hz, 1H), 7.70 (d, J = 1.8 Hz, 1H), 7.58 (s, 1H), 7.18 - 7.07 (m, 2H), 6.65 (d, J = 8.0 Hz, 1H), 6.61 (t, J = 2.1 Hz, 1H), 6.43 (dd, J = 8.1, 2.2 Hz, 1H), 3.89 (t, J = 6.5 Hz, 2H), 3.51 (s, 4H), 1.94 (ddd, J = 13.7, 8.4, 5.5 Hz, 1H), 1.74 - 1.58 (m, 7H), 1.21 (ddt, J = 36.1, 21.2, 10.0 Hz, 6H), 0.98 - 0.83 (m, 4H), 0.61 - 0.52 (m, 2H).
[M+H]⁺ = 431.3

To a solution of 4-{[3-(3-cyclohexylpropoxy)phenyl]amino}-3-cyclopropyl-N-(imidazolidin-2-ylidene)benzamide (84.0 mg, 109 µmoles, 1 eq.) and DIPEA (95 µL, 547 µmoles, 5 eq.) in DCM (2.0 mL) was added acetyl chloride (16 µL, 219 µmoles, 2.0 eq.). The resulting reaction mixture was stirred at room temperature for 20 minutes and then quenched with a saturated aqueous solution of brine and extracted with DCM. The combined organic layers were then dried over MgSO₄, filtered and concentrated under reduced pressure. The resulting residue was purified by column chromatography on silica gel to give N-[(2E)-1-acetylimidazolidin-2-ylidene]-4-{[3-(3-cyclohexylpropoxy)phenyl]amino}-3-cyclopropylbenzamide **(46)** (12.0 mg, 20%).
¹H NMR (400 MHz, *d₆*-DMSO) δ 9.70 (s, 1H), 7.81 (d, J = 8.4 Hz, 1H), 7.73 (d, J = 10.6 Hz, 2H), 7.16 (dd, J = 14.6, 8.2 Hz, 2H), 6.71 (d, J = 8.1 Hz, 1H), 6.67 (s, 1H), 6.48 (d, J = 8.4 Hz, 1H), 3.91 (t, J = 6.5 Hz, 2H), 3.87 - 3.78 (m, 2H), 3.59 (t, J = 8.5 Hz, 2H), 2.72 (s, 3H), 1.96 (t, J = 5.3 Hz, 1H), 1.68 (t, J = 14.8 Hz, 7H), 1.33 - 1.09 (m, 6H), 0.98 (q, J = 5.1, 4.5 Hz, 2H), 0.88 (q, J = 10.2, 9.2 Hz, 2H), 0.58 (q, J = 5.1 Hz, 2H).
¹³C NMR (151 MHz, *d₆*-DMSO) δ 176.3, 170.0, 160.0, 158.4, 146.5, 144.8, 131.1, 130.2, 129.6, 128.1, 127.8, 115.8, 111.5, 107.6, 105.5, 68.1, 42.5, 37.2, 33.7, 33.3, 26.6, 26.5, 26.3, 25.9, 11.4, 7.7
[M+H]⁺ = 503.1

### Pharmacological data

### Example 7: RSV virus

The compounds of the invention have been the subject of pharmacological tests which have demonstrated their relevance as active substances in therapy and in particular for preventing, inhibiting or treating RSV virus infection.

### MATERIAL AND METHODS

### Protocol for screening antiviral compounds for RSV inhibition and cytotoxicity using Viral ToxGlo assay

HEp-2 cells were maintained in Eagle's minimum essential medium (EMEM) with Earle's BSS adjusted to contain 2mM L-glutamine, 10% fetal bovine serum, 100 U/ml penicillin and 100µg/ml streptomycin. For the purposes of the screening assay they were grown to 90% confluency, trypsinized and recovered. The trypsin was neutralised with cell culture media and cells were centrifuged at 150 x g for 5 minutes before discarding the supernatant and resuspending cell pellet in assay media (EMEM with Earle's BSS adjusted to contain 2mM L-glutamine, 2% fetal bovine serum and 100 U/ml penicillin and 100µg/ml streptomycin). The cells were seeded into white clear-bottomed cell culture plates at a density of 1.5x10⁴ cells/well in 50µl and 4x10³ cells/well in 25µl for 96 well plates and 384 well plates respectively. For the media/background control column assay media only was added. Cell plates were placed in a humid chamber and incubated overnight at 37°C/5% CO₂. After overnight incubation cells were checked for confluency and healthy appearance.

Test articles were made up at 10x test concentration in a maximum DMSO concentration of 10% (final assay concentration maximal 1% DMSO) and added to the cell plates in volumes of 10µl for 96 well plates and 5µl for 384 well plates. For cell control and virus control wells the test article solvent only was added. Virus or assay media for cytotoxicity test wells and media/cell control wells was added immediately after test articles at an MOI of 0.5, 40 or 20µl for 96 and 384 well plates respectively. Virus suspension was prepared by thawing RSV A2 frozen stocks and diluting to the required concentration of plaque forming units in assay media on ice.

Cell plates were further incubated inside a humid chamber for 72h p.i at 37°C/5%CO₂. After the incubation period cells were observed under the microscope to check for characteristic cytopathic effect in virus control wells and healthy cells in the cell control wells. After plates were adjusted to room temperature 20/40µl Viral ToxGlo (Promega) was added to each well of the 384/96 well cell plates. Plates were incubated at room temperature, protected from light on a plate rocker for 20 minutes before measuring the luminescence on a spectrophotometer (Biotek Synergy HTX).

RSV inhibition was calculated as percentage of cytopathic effect inhibition relative to the virus control and cytotoxicity as percentage of cell survival relative to cell control wells. This allowed EC₅₀ values to be calculated for each test article where a virus inhibition or cytotoxic dose response was identified. EC₅₀ values ranging between 0.001 µM and 2.5 µM were found, and more particularly for compounds 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 69, 70, 71, 72, 73, 74, 91, 92, 94, 95, 96, 97, 98, 99, 100, 102, 103, 104, 105, 106, 107, 109, 110 and 111.

**Table III**

| **Ex** | **EC₅₀ (nM)** |
|---|---|
| **1** | 1.6 |
| **2** | 1.9 |
| **3** | 2.2 |
| **4** | 5.7 |
| **5** | 6.1 |
| **6** | 6.5 |
| **7** | 7.0 |
| **8** | 10.3 |
| **9** | 10.8 |
| **10** | 11.5 |
| **11** | 11.8 |
| **12** | 12.2 |
| **13** | 13.6 |
| **14** | 14.4 |
| **15** | 14.9 |
| **16** | 15.6 |
| **17** | 19.8 |
| **18** | 21.2 |
| **19** | 22.8 |
| **20** | 23.5 |
| **21** | 25.8 |
| **22** | 26.0 |
| **23** | 30.3 |
| **24** | 31.7 |
| **25** | 32.2 |
| **26** | 34.4 |
| **27** | 41.4 |
| **28** | 42.1 |
| **29** | 42.3 |
| **30** | 42.7 |
| **31** | 47.7 |
| **32** | 49.2 |
| **33** | 50.2 |
| **34** | 50.4 |
| **35** | 51.6 |
| **36** | 55.4 |
| **37** | 55.5 |
| **38** | 63.7 |
| **39** | 66.2 |
| **40** | 69.2 |
| **41** | 77.2 |
| **42** | 78.4 |
| **43** | 79.8 |
| **44** | 90.4 |
| **45** | 91.5 |
| **46** | 103.9 |
| **47** | 113.4 |
| **48** | 117.1 |
| **49** | 118.1 |
| **50** | 135.7 |
| **51** | 140.4 |
| **52** | 141.8 |
| **53** | 152.8 |
| **54** | 165.9 |
| **55** | 168.3 |
| **56** | 174.0 |
| **57** | 199.7 |
| **58** | 206.6 |
| **59** | 237.1 |
| **60** | 241.1 |
| **61** | 515.9 |
| **62** | 640.9 |
| **63** | 726.9 |
| **64** | 950.4 |
| **69** | 1134 |
| **70** | 1148 |
| **71** | 1170 |
| **72** | 1227 |
| **73** | 1288 |
| **74** | 1307 |
| **91** | 2.0 |
| **92** | 276.7 |
| **94** | 240.0 |
| **95** | 95.9 |
| **96** | 839.4 |
| **97** | 51.0 |
| **98** | 228.4 |
| **99** | 17.8 |
| **100** | 458.3 |
| **102** | 819.4 |
| **103** | 19.4 |
| **104** | 12.4 |
| **105** | 3.2 |
| **106** | 5.7 |
| **107** | 14.0 |
| **109** | 45.0 |
| **110** | 323.8 |
| **111** | 30.4 |

### Conclusion

Based on the previous results, it can be concluded that the compounds of formula (I) are suitable chemical compounds for treating and/or preventing RNA virus infections caused by RNA viruses of group V, more particularly, pneumovirus infections, and most particularly RSV virus infections.

The present invention further relates to a pharmaceutical composition comprising at least one new compound as defined above or any of its pharmaceutically acceptable salts, or at least any of compounds (1) to (111) as defined above or any of its pharmaceutically acceptable salts and also at least one pharmaceutically acceptable excipient.

Pharmaceutical compositions of the invention can contain one or more compound(s) of the invention in any form described herein.

Still a further object of the present invention consists of the use of at least one compound of formula (I), as defined above, and compounds (1) to (111) as defined above, or one of their pharmaceutically acceptable salts according to the present invention for preparing a drug to prevent or treat, in a subject, a RNA virus infection caused by a RNA virus from group V according to the Baltimore classification, and for example a RSV infection.

Therefore, the present invention relates to one compound of formula (I), as defined above, and compounds (1) to (111) or one of their acceptable salts as an agent for inhibiting, preventing or treating a RNA virus infection, and most preferably a RNA virus infection from group V, and for example a RSV infection.

According to a particular embodiment, the treatment is continuous or noncontinuous.

A "continuous treatment" means a long-term treatment which can be implemented with various administration frequencies, such as once every day, every three days, once a week, or once every two weeks or once every month.

According to one embodiment, the compound of formula (I), or anyone of its pharmaceutically acceptable salts, is administered at a dose varying from 0.1 to 1000 mg, in particular varying from 0.1 to 10 mg, or for example varying from 10 to 200 mg, or for example varying from 200 to 1000 mg.

Another object of the invention relates to a therapeutic method for treating and/or preventing a subject from a RNA virus infection, and most preferably a RNA virus infection caused by a virus belonging to group V of the Baltimore classification comprising the administration of a therapeutically effective quantity of a compound of formula (I), compounds (1) to (111), as defined above, or one of their acceptable salts.

In a specific embodiment, the invention provides a use of a compound of formula (I) according to the invention or a pharmaceutically acceptable salt thereof or a pharmaceutically active derivative thereof or a method according to the invention wherein the compound of formula (I) is to be administered in combination with a co-agent useful in the treatment of said RNA virus infection, and most preferably said RNA virus infection from group V, and for example RSV infection.

The compounds can be administered through any mode of administration such as, for example, intramuscular, intravenous, intranasal or oral route, etc.

Compounds of the present invention may, in appropriate cases, be administered as prodrugs, such as esters, of compounds with which the invention is concerned. "Prodrug" means a compound which is convertible *in vivo* by metabolic means (*e.g.* by hydrolysis, reduction or oxidation) to a compound of the present invention. For example, an ester prodrug of a compound of the present invention may be convertible by hydrolysis *in vivo* to the parent molecule. Suitable esters of compounds of the present invention are for example acetates, citrates, lactates, tartrates, malonates, oxalates, salicylates, propionates, succinates, fumarates, maleates, methylene-bis-β-hydroxynaphthoates, gentisates, isethionates, di-p-toluoyltartrates, methanesulphonates, ethanesulphonates, benzenesulphonates, p-toluenesulphonates, cyclohexylsulfamates and quinates. Examples of ester prodrugs are those described by F. J. Leinweber, Drug Metab. Res., 1987, 18, 379. As used herein, references to the compounds of the present invention are meant to also include any prodrug or metabolite forms.

The inventive composition can further include one or more additives such as diluents, excipients, stabilizers and preservatives. Such additives are well known to those skilled in the art and are described notably in *"*Ullmann's Encyclopedia of Industrial Chemistry, 6th Ed." (various editors, 1989-1998, Marcel Dekker) and in *"*Pharmaceutical Dosage Forms and Drug Delivery Systems" (ANSEL et al., 1994, WILLIAMS & WILKINS).

The aforementioned excipients are selected according to the dosage form and the desired mode of administration.

According to another embodiment, pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated.

Compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance.

In a particular embodiment, a compound of formula (I) according to the invention is administered orally.

Oral route of administration is in particular preferred in the prophylaxis or treatment aspect of the invention.

## Claims

1. A compound of formula (I): 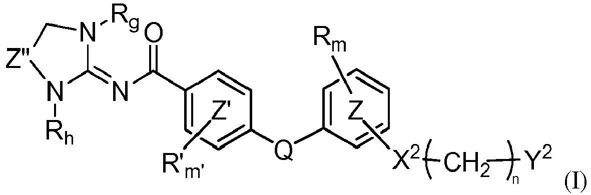 wherein: 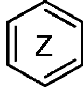
ring and 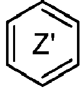 ring independently mean a phenylene or a pyridylene group,
Z" represents a -CH₂- group or a -CO- group,
R_{g} and Rₕ independently represent a hydrogen atom, a (C₁-C₄)alkyl group, a (C₃-C₆)cycloalkyl group or a -COCH₃ group,
Q is NH or O,
X² represents
a -CO-NRₖ- group, wherein Rₖ represents a hydrogen atom or a methyl group, a -NR'ₖ-CO- group, wherein R'ₖ represents a hydrogen atom or a methyl group,
a -O- group,
a -CO- group,
a -SO₂-group,
a -CS-NH- group,
a -CH₂-NH-,
a 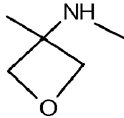 group, or
a divalent 5-membered heteroaromatic ring comprising 1, 2, 3 or 4 heteroatoms, such as a triazole, an imidazole, a tetrazole or an oxadiazole,
n is 0, 1, 2 or 3,
m and m' are independently 0, 1 or 2,
Y² represents
a hydrogen atom,
a halogen atom,
a hydroxyl group,
a morpholinyl group, optionally substituted by a (C₁-C₄)alkyl group,
a piperidinyl group, optionally interrupted by a SO₂ group,
a (C₁-C₄)alkenyl group,
a -PO(ORₐ)(OR_{b}) group,
or
a -CR¹R²R³ group, wherein R¹, R² and R³ independently represent a hydrogen atom, a fluorine atom or a (C₁-C₄)alkyl group, being understood that no more than one of R¹, R² and R³ is a hydrogen atom, or R¹ and R² form together with the carbon atom bearing them a (C₃-C₈)cycloalkyl group, said (C₃-C₈)cycloalkyl group being optionally substituted by one or two (C₁-C₄)alkyl group, halogen atom, trifluoromethyl group, cyano group or (C₁-C₄)alkoxy group and said (C₃-C₈)cycloalkyl group being optionally interrupted on said R¹ and/or R² by one or two oxygen atom(s) or by a -SO₂-group,
R and R' independently represent
a (C₁-C₄)alkyl group, optionally substituted by a hydroxyl group, and optionally interrupted by a -SO₂- group or a -SO- group,
a (C₁-C₄)alkenyl group,
a (C₃-C₆)cycloalkyl group,
a tetrahydrofuranyl group,
a tetrahydropyranyl group,
a trifluoromethyl group,
a furanyl group,
a halogen atom,
or
a (C₁-C₅)alkoxy group,
Rₐ and R_{b} independently represent a hydrogen atom or a (C₁-C₄)alkyl group,
or any of its pharmaceutically acceptable salt.

2. A compound of formula (I) according to claim 1, wherein 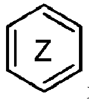 ring and 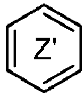 ring both represent a phenylene group or 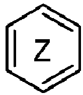 ring represents a pyridylene group and 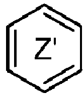 ring represents a phenylene group, or any of its pharmaceutically acceptable salt.

3. A compound of formula (I) according to claim 1 or 2, wherein Q is a NH group or any of its pharmaceutically acceptable salt.

4. A compound of formula (I) according to anyone of claims 1 to 3, wherein R_{g} and Rh represent a hydrogen atom or any of its pharmaceutically acceptable salt.

5. A compound of formula (I) according to anyone of claims 1 to 4, wherein X
² represents
a -CO-NH- group,
a -NH-CO- group,
a -O- group,
a -CO- group,
or
a divalent 5-membered heteroaromatic ring comprising at least 2 nitrogen atoms, such as a triazole, an imidazole or a tetrazole,
or any of its pharmaceutically acceptable salt.

6. A compound of formula (I) according to anyone of claims 1 to 5, wherein Y
² represents
a hydrogen atom,
a halogen atom,
a morpholinyl group, optionally substituted by a (C₁-C₄)alkyl group,
a (C₁-C₄)alkenyl group,
a -PO(ORₐ)(OR_{b}) group,
or
a -CR¹R²R³ group, wherein R¹, R² and R³ independently represent a hydrogen atom, a fluorine atom or a (C₁-C₄)alkyl group, being understood that no more than one of R¹, R² and R³ is a hydrogen atom, or R¹ and R² form together with the carbon atom bearing them a (C₃-C₈)cycloalkyl group, said (C₃-C₈)cycloalkyl group being optionally substituted by one or two (C₁-C₄)alkyl group, halogen atom, trifluoromethyl group or cyano group and said (C₃-C₈)cycloalkyl group being optionally interrupted on said R¹ and/or R² by one or two oxygen atom(s) or by a -SO₂- group,
with Rₐ and R_{b} being as defined in claim 1,
or any of its pharmaceutically acceptable salt.

7. A compound of formula (I) according to anyone of claims 1 to 6, wherein R
and R' independently represent
a (C₁-C₄)alkyl group, optionally substituted by a hydroxyl group, and optionally interrupted by a -SO₂- group or a -SO- group,
a (C₃-C₆)cycloalkyl group,
a tetrahydrofuranyl group,
a tetrahydropyranyl group,
a trifluoromethyl group,
a furanyl group,
a chlorine or fluorine atom,
or
a (C₁-C₅)alkoxy group,
or any of its pharmaceutically acceptable salt.

8. A compound of formula (I) according to claim 1 selected from
| (I) | |
|---|---|
| 1 | 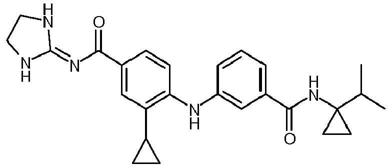 |
| 2 | 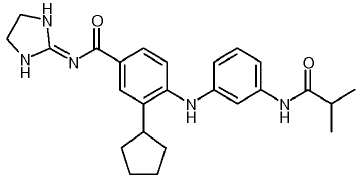 |
| 3 | 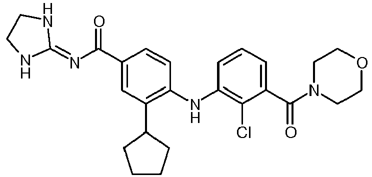 |
| 4 | 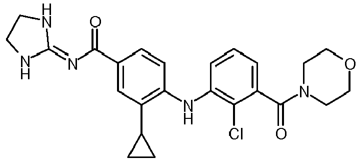 |
| 5 | 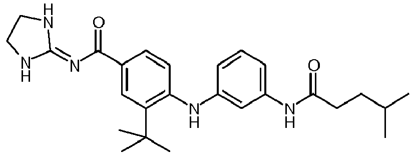 |
| 6 | 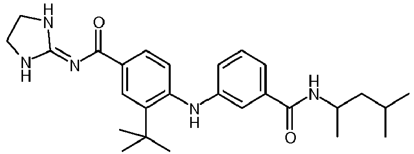 |
| 7 | 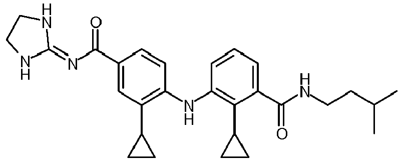 |
| 8 | 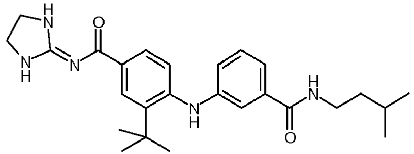 |
| 9 | 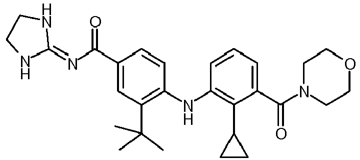 |
| 10 | 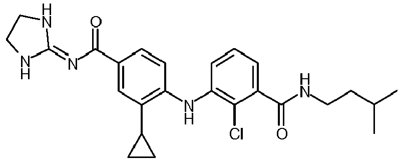 |
| 11 | 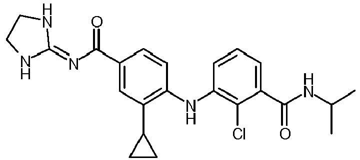 |
| 12 | 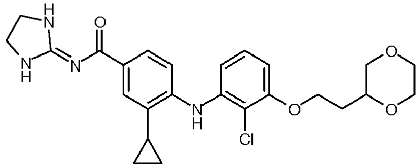 |
| 13 | 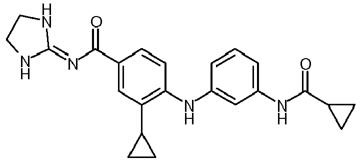 |
| 14 | 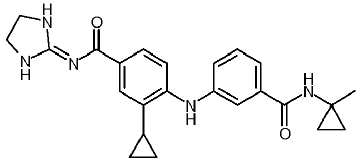 |
| 15 | 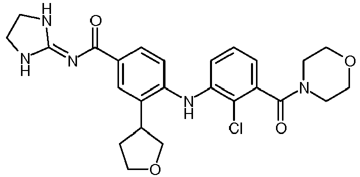 |
| 16 | 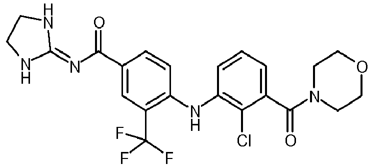 |
| 17 | 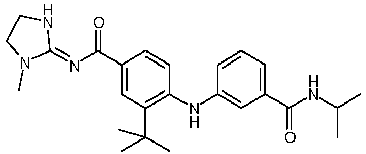 |
| 18 | 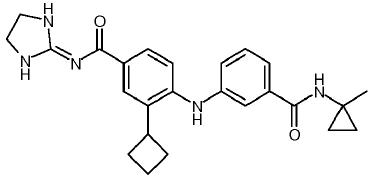 |
| 19 | 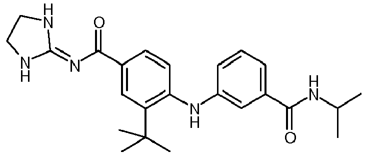 |
| 20 | 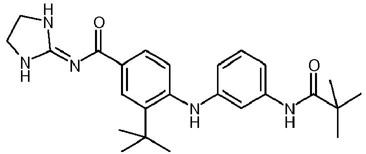 |
| 21 | 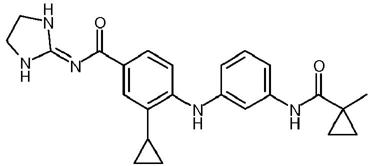 |
| 22 | 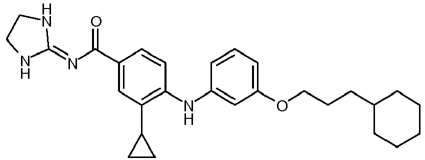 |
| 23 | 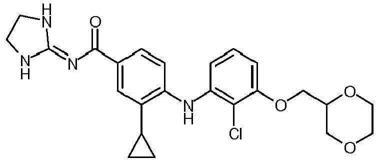 |
| 24 | 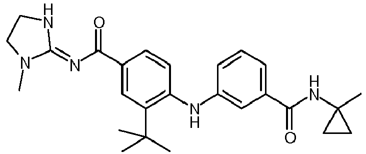 |
| 25 | 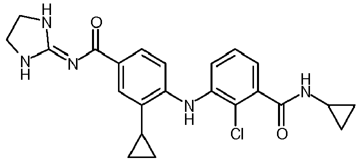 |
| 26 | 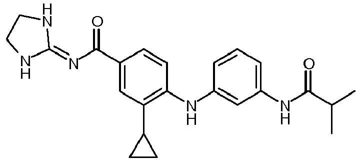 |
| 27 | 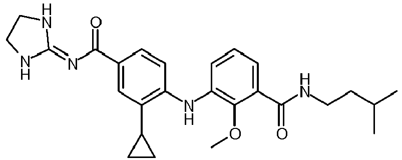 |
| 28 | 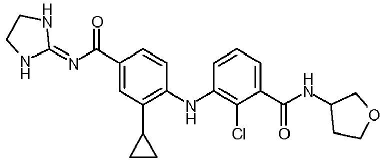 |
| 29 | 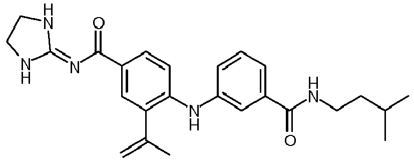 |
| 30 | 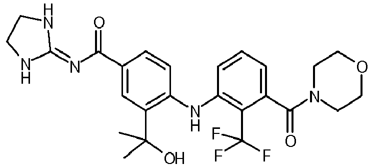 |
| 31 | 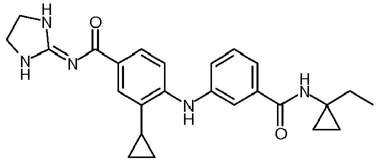 |
| 32 | 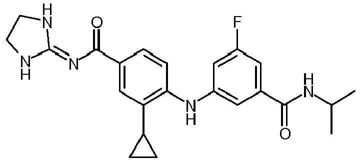 |
| 33 | 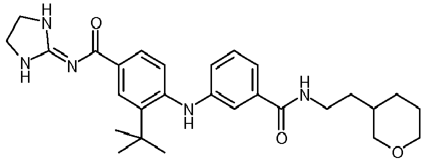 |
| 34 | 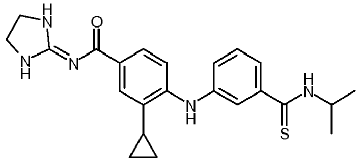 |
| 35 | 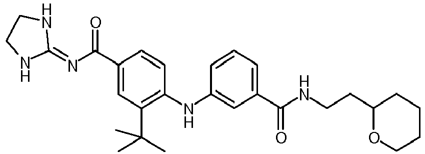 |
| 36 | 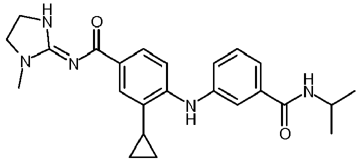 |
| 37 | 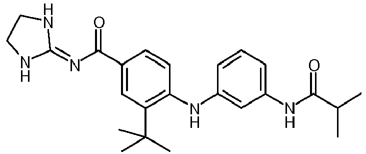 |
| 38 | 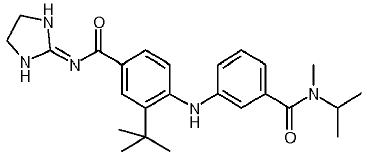 |
| 39 | 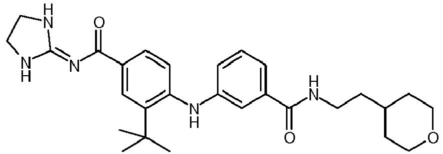 |
| 40 | 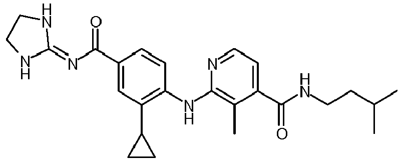 |
| 41 | 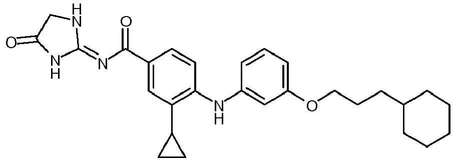 |
| 42 | 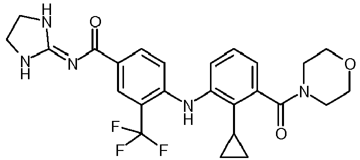 |
| 43 | 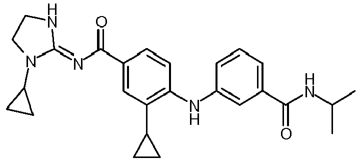 |
| 44 | 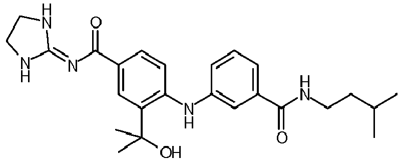 |
| 45 | 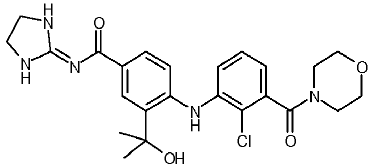 |
| 46 | 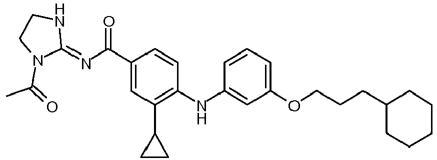 |
| 47 | 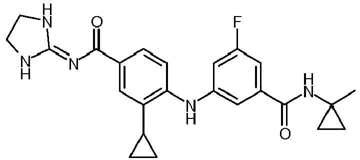 |
| 48 | 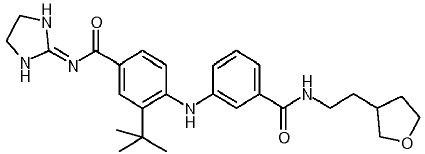 |
| 49 | 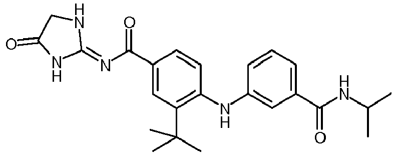 |
| 50 | 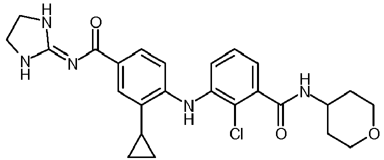 |
| 51 | 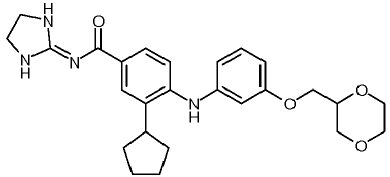 |
| 52 | 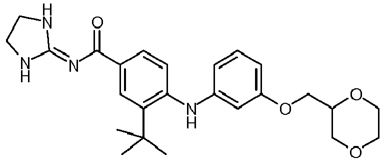 |
| 53 | 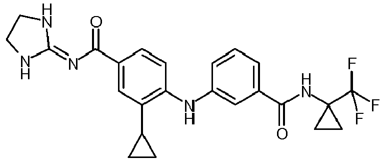 |
| 54 | 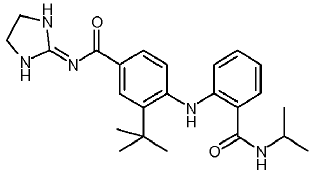 |
| 55 | 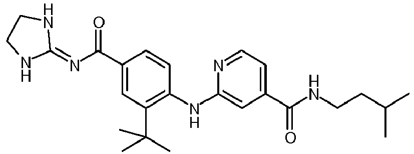 |
| 56 | 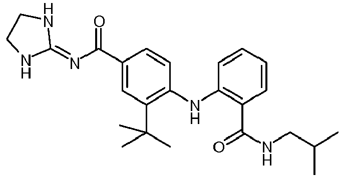 |
| 57 | 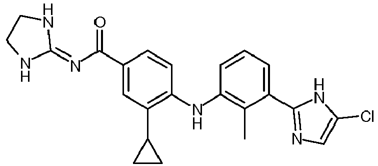 |
| 58 | 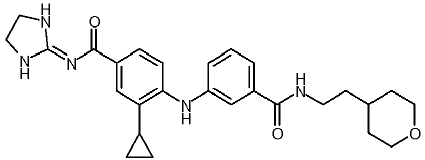 |
| 59 | 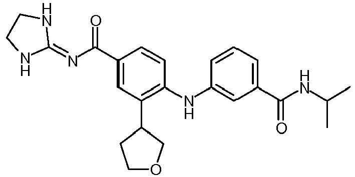 |
| 60 | 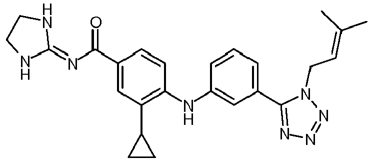 |
| 61 | 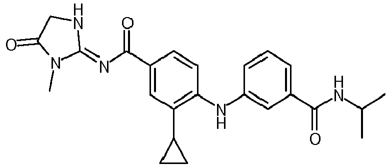 |
| 62 | 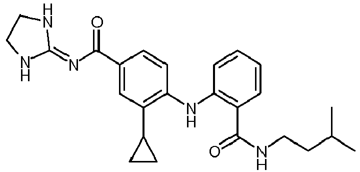 |
| 63 | 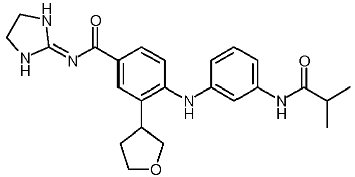 |
| 64 | 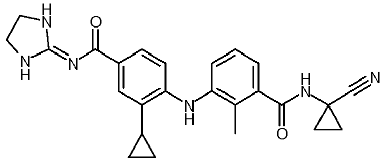 |
| 65 | 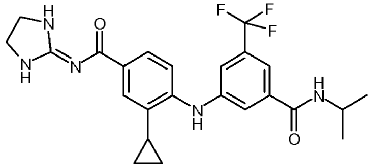 |
| 66 | 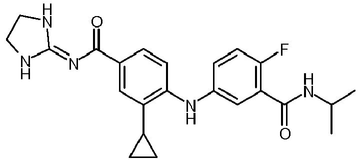 |
| 67 | 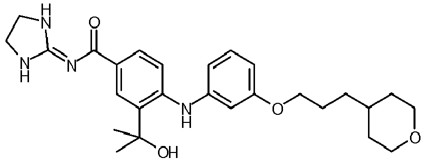 |
| 68 | 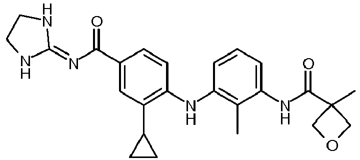 |
| 69 | 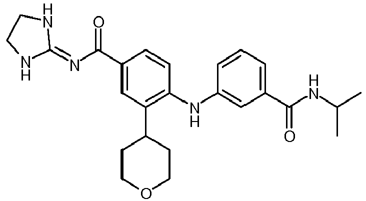 |
| 70 | 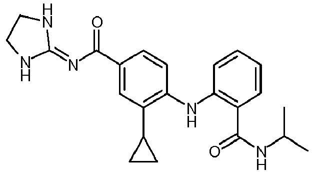 |
| 71 | 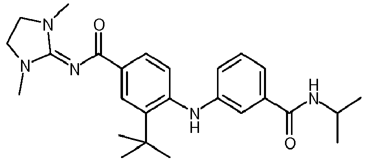 |
| 72 | 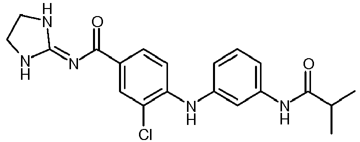 |
| 73 | 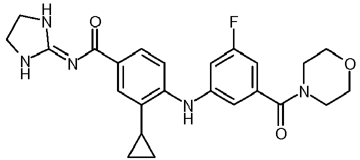 |
| 74 | 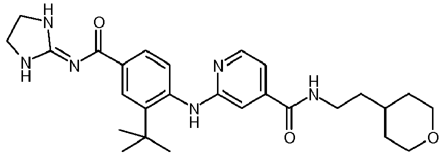 |
| 75 | 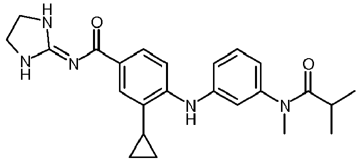 |
| 76 | 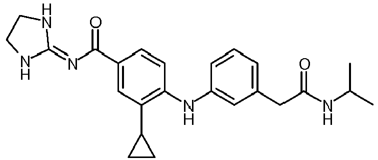 |
| 77 | 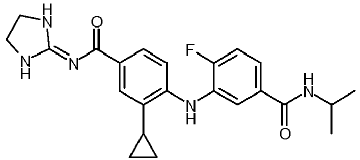 |
| 78 | 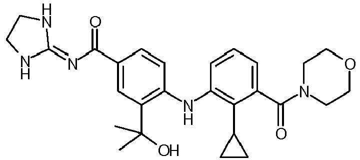 |
| 79 | 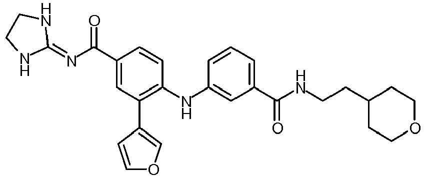 |
| 80 | 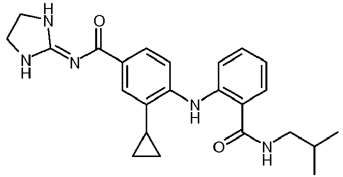 |
| 81 | 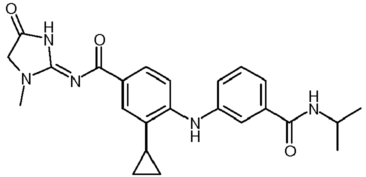 |
| 82 | 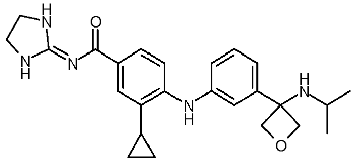 |
| 83 | 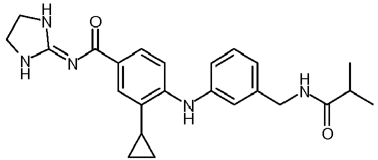 |
| 84 | 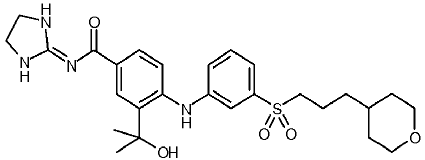 |
| 85 | 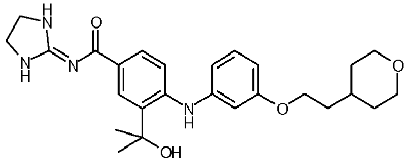 |
| 86 | 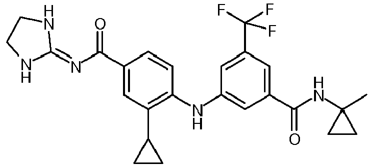 |
| 87 | 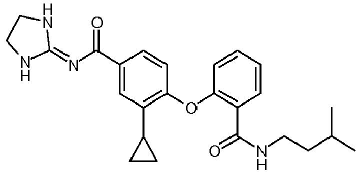 |
| 88 | 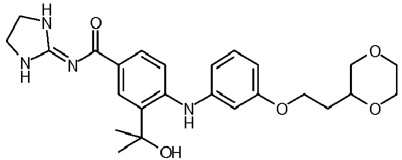 |
| 89 | 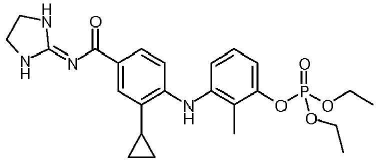 |
| 90 | 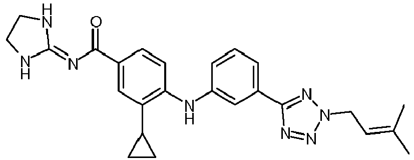 |
| 91 | 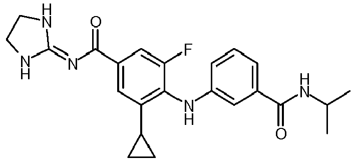 |
| 92 | 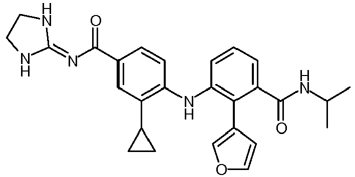 |
| 93 | 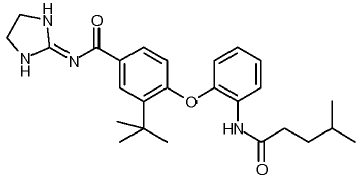 |
| 94 | 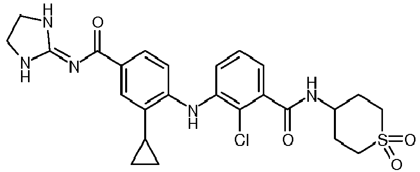 |
| 95 | 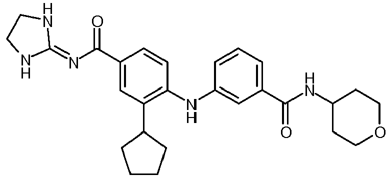 |
| 96 | 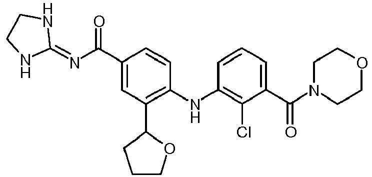 |
| 97 | 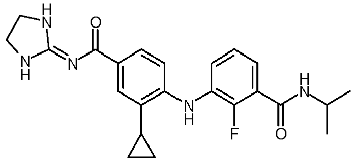 |
| 98 | 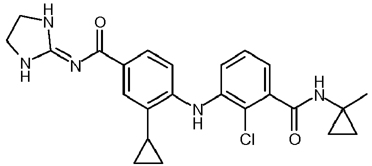 |
| 99 | 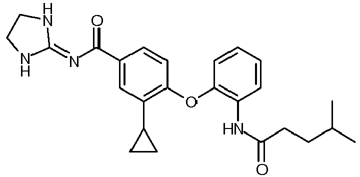 |
| 100 | 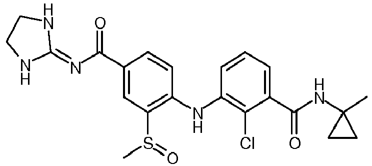 |
| 101 | 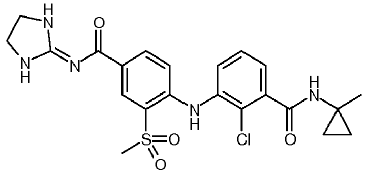 |
| 102 | 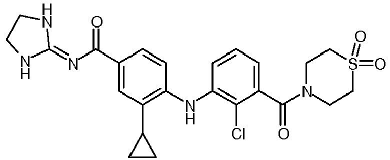 |
| 103 | 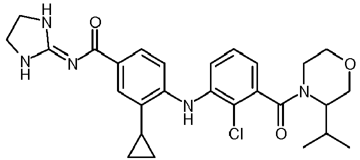 |
| 104 | 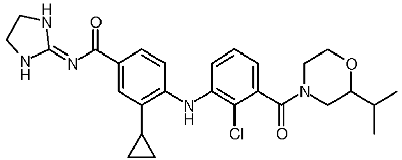 |
| 105 | 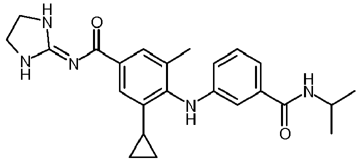 |
| 106 | 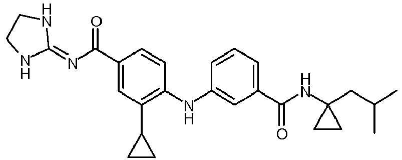 |
| 107 | 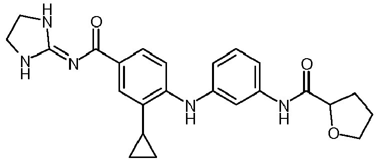 |
| 108 | 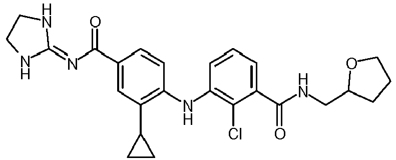 |
| 109 | 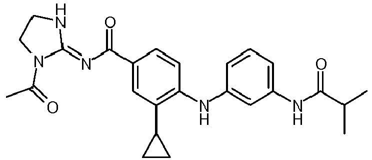 |
| 110 | 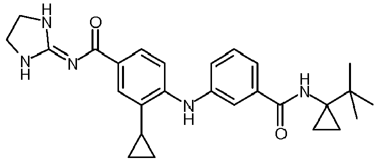 |
| 111 | 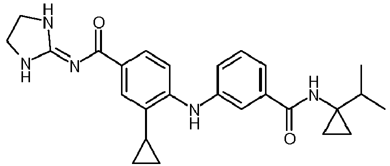 |
or any of its pharmaceutically acceptable salt.

9. A compound of formula (I) as defined in anyone of claims 1 to 7 or any of its pharmaceutically acceptable salts, and any of compounds (1) to (111) as defined in claim 8 or any of its pharmaceutically acceptable salts, for use as a medicament.

10. A compound of formula (I) as defined in anyone of claims 1 to 7 or any of its pharmaceutically acceptable salts, and any of compounds (1) to (111) as defined in claim 8 or any of its pharmaceutically acceptable salts for use in the treatment and/or prevention of a RNA virus infection caused by a RNA virus belonging to group V of the Baltimore classification, and in particular a RSV viral infection or a virus-related condition.

11. A pharmaceutical composition comprising at least one of formula (I) as defined in anyone of claims 1 to 7 or any of its pharmaceutically acceptable salts, and any of compounds (1) to (111) as defined in claim 8 or any of its pharmaceutically acceptable salts and also at least one pharmaceutically acceptable excipient.

12. Synthesis process for manufacturing new compounds of formula (I) as defined in anyone of claims 1 to 7, wherein Q is NH, comprising at least a step of coupling a compound of formula (II)
(i) a step of coupling a compound of formula (II) 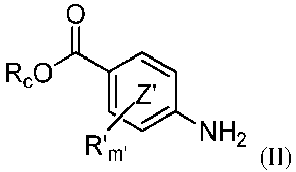 with a compound of formula (III) 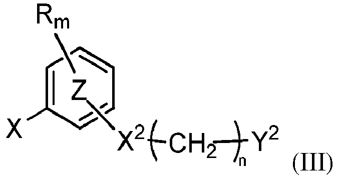 wherein R, R', m, m', n, 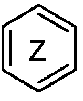 ring, 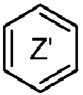 ring, X², Y² are as defined in anyone of claims 1 to 7, R_{c} is an alkyl group, such as a ethyl or methyl group and X is a chlorine atom, an iodine atom or a bromine atom, in presence of an inorganic base and a diphosphine and in the presence of an organometallic catalyst, to obtain a compound of formula (IV) 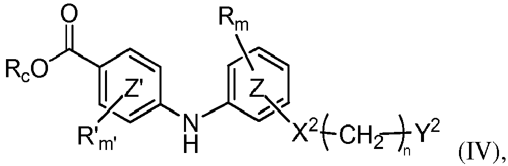 wherein Rc means an alkyl group, such as an ethyl group or a methyl group, preferentially a methyl group and R, R', m, m', n, 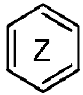 ring, 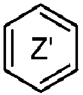 ring, X², Y² are as defined in anyone of claims 1 to 7, followed by a hydrolysis leading to a compound of formula 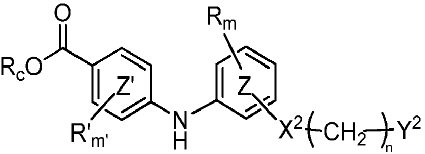 (V) wherein Rc is a hydrogen atom and R, R', m, m', n, 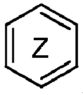 ring, 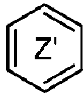 ring, X², Y² are as defined in anyone of claims 1 to 7, and
(ii) a step of reacting a compound of formula (V) with a compound or formula 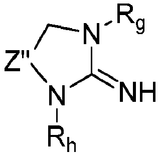 wherein Rg and Rh are as defined in claim 1, in presence of an organic base such as N,N-diisopropylethylamine and in presence of a coupling agent such as 1,1'-carbonyldiimidazole to obtain a compound for formula (I) wherein Q is NH, as defined in anyone of claims 1 to 7.

13. Synthesis process for manufacturing new compounds of formula (I) as defined in anyone of claims 1 to 7, wherein Q is O, comprising at least a step of coupling a compound of formula (II') 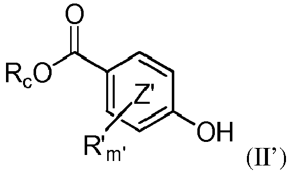 with a compound of formula (III) 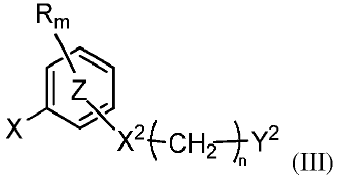 wherein R, R', R_{c}, Rₕ, R_{g}, m, m', n, 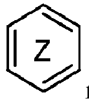 ring, 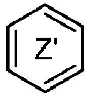 ring, X², Y² are as defined in anyone of claims 1 to 7 and X is a fluorine atom, in presence of an inorganic base, to obtain a compound of formula (I), wherein Q is O, as defined in anyone of claims 1 to 7.
